# FASCICULE DE BREVET EUROPEEN

(11) **EP 0 915 823 B1**
(45) Date de publication et mention de la délivrance du brevet: **18.04.2001**
(21) Numéro de dépôt: 97947075.4
(22) Date de dépôt: 17.11.1997
(51) Int. Cl.: C07C 59/84, A61K 31/19, A61K 31/215, A61K 7/00, C07C 33/38, C07C 47/238, C07C 69/618, C07C 233/11, C07C 239/18, C07C 251/48, C07C 323/62, C07D 295/185, C07D 339/08

(54) **COMPOSES BIAROMATIQUES, COMPOSITIONS PHARMACEUTIQUES ET COSMETIQUES LES CONTENANT ET UTILISATIONS**
BISAROMATISCHE VERBINDUNGEN, SIE ENTHALTENDE PHARMAZEUTISCHE UND KOSMETISCHE ZUSAMMENSETZUNGEN UND VERWENDUNGEN
BI-AROMATIC COMPOUNDS, PHARMACEUTICAL AND COSMETIC COMPOSITIONS CONTAINING SAME AND USES

(30) Priorité: 19.11.1996 FR 9614098
(43) Date de publication de la demande: 19.05.1999
(73) Titulaire: CENTRE INTERNATIONAL DE RECHERCHES DERMATOLOGIQUES GALDERMA - CIRD GALDERMA, F-06560 Valbonne (FR)
(72) Inventeur: BERNARDON, Jean-Michel, F-06650 Le Rouret (FR); DIAZ, Philippe, Le Jardin de Saint-Antoine, F-06200 Nice (FR)
(74) Mandataire: Tezier Herman, Béatrice
(86) Numéro de dépôt international: FR9702063
(87) Numéro de publication internationale: WO9822423

(56) Documents cités:
- EP-A- 0 260 162
- FR-A- 2 278 331
- FR-A- 2 371 195
- GB-A- 2 228 734
- KUO-LONG YU: "Application of the Heck reaction in the synthesis of truncated naphthoic acid retinoids" BIOORGANIC & MEDICINAL CHEMISTRY LETTERS, vol. 6, no. 23, 1996, pages 2859-2864, XP002061556

## Description

L'invention concerne, à titre de produits industriels nouveaux et utiles, des composés biaromatiques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les documents EP-A-0 260 162 (D1) et GB-A-2 228 734 (D2) décrivent des composés présentant une activité antiproliférative. Ces composés ont une structure bicyclique dans laquelle l'un des cycles porte une liaison insaturée située en position para par rapport à l'autre cycle.

La présente invention concerne des composés peuvent être représentés par la formule générale (I) suivante : 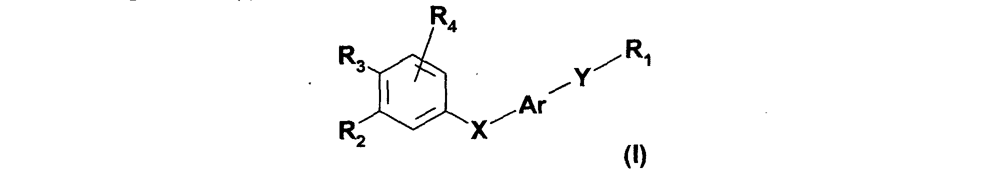 dans laquelle:
- R₁ représente
   (i) le radical -CH₃,
   (ii) le radical -CH₂-O-R₅,
   (iii) le radical -O-R₅,
   (iv) le radical -CO-R₆,
   R₅ et R₆ ayant les significations données ci-après,
- Y représente un radical choisi parmi les radicaux de formules (a) et (b) suivantes: 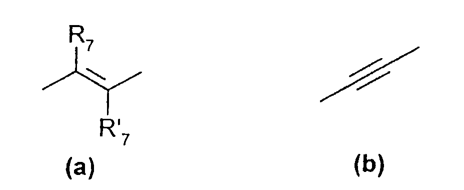 R₇ et R'₇ ayant les significations données ci-après,
- Ar représente un radical choisi parmi les radicaux de formules (c) à (f) suivantes: 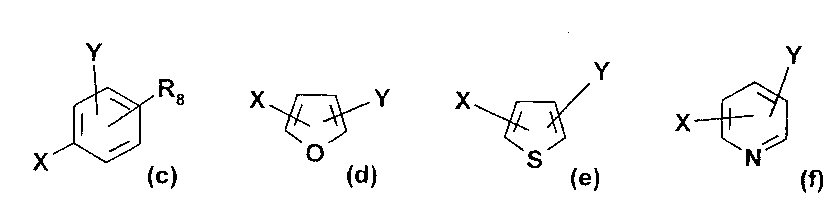 dans lesquelles le radical Y est en position ortho ou méta par rapport au radical X, X et Y de ces formules correspondants à X et Y représentés dans la formule (I),
   R₈ ayant la signification donnée ci-après,
- X représente un atome d'oxygène, de soufre, un radical -SO-, -SO₂-,N(R₉)- ou un radical choisi parmi les radicaux de formules (g) à (r) suivantes: 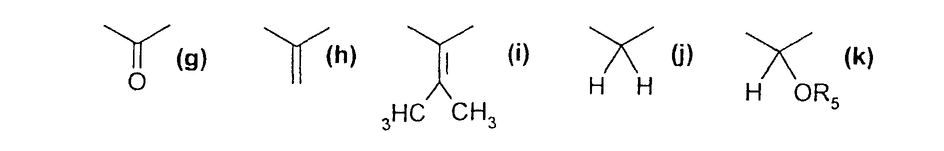 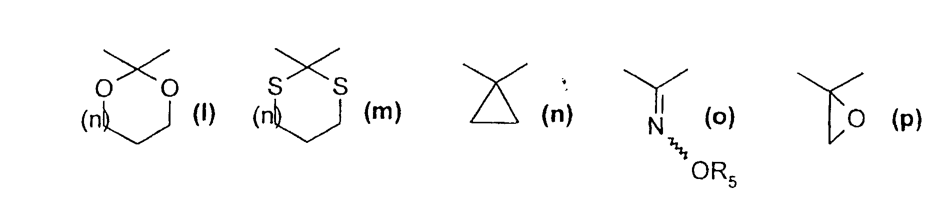 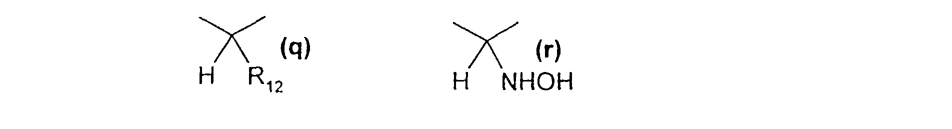 R₅, R₉, R₁₂ et n ayant les significations données ci-après,
- R₂ et R₃, identiques ou différents, sont choisis dans le groupe constitué par :
   (i) un atome d'hydrogène,
   (ii) un radical alkyle présentant au moins 3 atomes de carbone, parmi lesquels le carbone attaché au radical phényl de la formule (I) est substitué par au moins deux atomes de carbone,
   (iii) un radical alkyle linéaire ou ramifié,
   (iv) un radical-OR₅,
   (v) un radical -SR₅,
   (vi) un radical polyéther,
   R₅ ayant la signification donnée ci-après,
   étant entendu que R₂ et R₃ pris ensemble peuvent former avec le cycle aromatique adjacent un cycle à 5 ou 6 chaînons éventuellement substitué par des groupes méthyle et/ou éventuellement interrompu par un atome d'oxygène ou de soufre,
   étant entendu que, lorsque R₂ et R₃ ne forment pas un cycle, au moins un des radicaux R₂ et R₃ a une signification (ii) mentionnée ci-dessus,
- R₄ et R₈, identiques ou différents, représentent un atome d'hydrogène, un atome d'halogène, un radical alkyle linéaire ou ramifié, ou un radical -OR₅, un radical polyéther,
- R₅ représente un atome d'hydrogène, un radical alkyle ayant de 1 à 6 atomes de carbone ou un radical -COR₁₀,
   R₁₀ ayant la signification donnée ci-après,
- R₆ représente :
   (a) un atome d'hydrogène
   (b) un radical alkyle ayant de 1 à 6 atomes de carbone
   (c) un radical de formule: 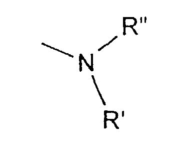 R' et R" ayant les significations données ci-après,
   (d) un radical -OR₁₁
      R₁₁ ayant la signification donnée ci-après,
   - R₇, R'₇ et R₉, identiques ou différents, représentent un atome d'hydrogène ou un radical alkyle ayant de 1 à 6 atomes de carbone,
   - n est un nombre entier égal à 0 ou 1,
   - R₁₀ représente un radical alkyle ayant de 1 à 6 atomes de carbone,
   - R₁₁ représente un atome d'hydrogène, un radical alkyle linéaire ou ramifié, un radical alkényl, un radical mono ou polyhydroxyalkyle, un radical aryle ou aralkyle, éventuellement substitué (s) ou un reste de sucre ou un reste d'amino acide ou de peptide,
   -R₁₂ représente un radical alkyle ayant de 1 à 6 atomes de carbone,
   - R' et R", identiques ou différents, représentent un atome d'hydrogène, un radical alkyl inférieur, un radical mono ou polyhydroxyalkyle, un radical aryle éventuellement substitué ou un reste d'amino acide, de peptide ou de sucre ou encore pris ensemble forment un hétérocycle,
   et les isomères optiques et géométriques desdits composés de formule (I) ainsi que leurs sels .

Lorsque les composés selon l'invention se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique. Lorsque les composés selon l'invention se présentent sous forme de sels par addition d'une base, il s'agit préférentiellement de sels d'un métal alcalin ou alcalino-terreux ou encore de zinc ou d'une amine organique.

Selon la présente invention, on entend par radical alkyle linéaire ou ramifié un radical linéaire ou ramifié, éventuellement substitué par un ou plusieurs atomes d'halogène, ayant de 1 à 20, de préférence de 1 à 12, atomes de carbone, avantageusement les radicaux méthyle, éthyle, isopropyle, butyle, tertiobutyle, hexyle, nonyle et dodécyle. Lorsqu'il est inférieur, le radical alkyle comprend de 1 à 6 atomes de carbone. On préfère un radical méthyle, éthyle, isopropyle, tertiobutyle et hexyle.

Parmi les radicaux alkyle linéaire ayant de 1 à 20 atomes de carbone, on peut citer notamment les radicaux méthyle, éthyle, propyle, 2-éthyl-hexyle, octyle, dodécyle, hexadécyle et octadécyl.

Parmi les radicaux alkyle ramifié ayant de 1 à 20 atomes de carbone, de préférence de 3 à 20, on peut citer notamment les radicaux 2-méthylbutyle, 2-méthylpentyle, 1-méthylhexyle, 3-méthylheptyle.

Parmi les radicaux alkyle présentant au moins 3 atomes de carbone, parmi lesquels le carbone attaché au radical phényl de la formule (I) est substitué par au moins deux atomes de carbone, on peut citer les radicaux isopropyle, tertiobutyle, 1,1-diméthylhexyle et 1,1-diméthyldécyle. De préférence, ces radicaux présentent au maximum 20 atomes de carbone, encore plus préférentiellement au maximum 12 atomes de carbone. De manière avantageuse, le radical (ii) est le radical tertiobutyle.

Par radical alkényle, on entend un radical ayant de 2 à 20 atomes de carbone linéaire ou ramifié comportant une ou plusieurs doubles liaisons.

Parmi les radicaux alkényle, on préfère un radical contenant de 2 à 5 atomes de carbone et présentant une ou plusieurs insaturations éthyléniques, tel que plus particulièrement le radical allyle.

Par radical monohydroxyalkyle ou polyhydroxyalkyle, on doit entendre un radical contenant de 1 à 6 atomes de carbone et de 1 à 5 groupes hydroxyles.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle éventuellement substitués, on préfère un radical phényle éventuellement substitué par au moins un atome d'halogène, un radical hydroxyle, un radical alkyle, une fonction nitro, un groupe méthoxy ou une fonction amine éventuellement substituée.

Parmi les radicaux aralkyle, éventuellement substitués, on préfère le radical benzyle ou phénéthyle éventuellement substitué par au moins un atome d'halogène, un radical hydroxyle, une fonction nitro ou un groupe méthoxy.

Par reste de sucre, on entend un reste dérivant notamment de glucose, de galactose ou de mannose, ou bien encore de l'acide glucuronique.

Par reste d'aminoacide, on entend notamment un reste dérivant de l'un des acides aminés tels que la lysine, la glycine ou l'acide aspartique, et par reste de peptide on entend plus particulièrement un reste de dipeptide ou de tripeptide résultant de la combinaison d'acides aminés.

Par hétérocycle, on entend de préférence un radical pipéridino, morpholino, pyrrolidino ou pipérazino, éventuellement substitué en position 4 par un radical alkyle en C₁-C₆ ou polyhydroxyalkyle tels que définis ci-dessus.

Parmi les radicaux polyéther, on préfère un radical contenant de 2 à 6 atomes de carbone, notamment les radicaux méthoxyméthoxy, méthoxyéthoxy, méthoxyéthoxyméthoxy, méthoxyméthoxyéthyl, méthoxyméthoxypropyl et méthoxyhexyloxy.

Lorsque les radicaux R₄ et R₈ représentent un atome d'halogène, celui-ci est de préférence un atome de fluor, de brome ou de chlore.

Parmi les composés de formule (I) ci-dessus rentrant dans le cadre de la présente invention, on peut notamment citer les suivants :
Acide 3-[3-(5,5,8,8-tetramethyl-5,6,7,8-tetrahydro-2-naphthylcarbonyl)phenyl] acrylique.
Acide 3-[3-(3,5,5,8,8-pentamethyl-5,6,7,8-tetrahydro-2-naphthylcarbonyl) phenyl]acrylique.
Acide 3-[3-(3,5,5,8,8-pentamethyl-5,6,7,8-tetrahydro-2-naphthylthio)phenyl] acrylique.
Acide 3-[3-(5,5,8,8-tetramethyl-5,6,7,8-tetrahydro-2-naphthylthio)phenyl] acrylique.
3{2-[1-(3,5,5,8,8-pentaméthyl-5,6,7,8-tétrahydro-2-naphthyl)vinyl] phenyl} acrylate d'éthyle.
Acide 3{2-[1 -(3,5,5,8,8-pentaméthyl-5,6,7,8-tétrahydro-2-naphthyl) vinyl]phenyl} acrylique.
3{3-[1-(3,5,5,8,8-pentaméthyl-5,6,7,8-tétrahydro-2-naphthyl)vinyl ]phenyl} acrylate d'éthyle.
Acide 3{3-[1-(3,5,5,8,8-pentaméthyl-5,6,7,8-tétrahydro-2-naphthyl)vinyl] phenyl} acrylique.
3{2-[1-(5,5,8,8-tétraméthyl-5,6,7,8-tétrahydro-2-naphthyl)vinyl]phenyl} acrylate d'éthyle.
Acide 3{2-[1-(5,5,8,8-tétraméthyl-5,6,7,8-tétrahydro-2-naphthyl)vinyl] phenyl} acrylique.
3{3-[1-(5,5,8,8-tétraméthyl-5,6,7,8-tétrahydro-2-naphthyl)vinyl]phenyl} acrylate d'éthyle.
Acide 3{3-[1-(5,5,8,8-tétraméthyl-5,6,7,8-tétrahydro-2-naphthyl) vinyl]phenyl} acrylique.
3{3-[1-(5,5,8,8-tétraméthyl-5,6,7,8-tétrahydro-2-naphtyl)éthyl]phenyl} acrylate d'éthyle.
Acide 3{3-[1-(5,5,8,8-tétraméthyl-5,6,7,8-tétrahydro-2-naphtyl) éthyl] phenyl} acrylique.
3{2-[1-(5,5,8,8-tétraméthyl-5,6,7,8-tétrahydro-2-naphtyl)éthyl]phenyl} acrylate d'éthyle.
Acide 3{2-[1-(5,5,8,8-tétraméthyl-5,6,7,8-tétrahydro-2-naphtyl) éthyl] phenyl} acrylique.
3{2-[1-(3,5,5,8,8-pentaméthyl-5,6,7,8-tétrahydro-2-naphtyl)éthyl]phenyl} acrylate d'éthyle.
Acide 3{2-[1-(3,5,5,8,8-pentaméthyl-5,6,7,8-tétrahydro-2-naphtyl)éthyl] phenyl} acrylique.
3{3-[1-(3,5,5,8,8-pentaméihyl-5,6,7,8-tétrahydro-2-naphiyl)éthyl]phenyl} acrylate d'éthyie.
Acide 3{3-[1-(3,5,5,8,8-pentaméthyl-5,6,7,8-tétrahydro-2-naphtyl) éthyl] phenyl} acrylique.
3{2-[1-(5,5,8,8-tétraméthyl-5,6,7,8-tétrahydro-2-naphtyl)cyclopropyl] phenyl} acrylate d'éthyle.
Acide 3{2-[1-(5,5,8,8-tétraméthyl-5,6,7,8-tétrahydro-2-naohtyl) cyclopropyl]phenyl} acrylique.
3{3-[1-(5,5,8,8-tétraméthyl-5,6,7,8-tétrahydro-2-naphtyl)cyclopropyl] phenyl} acrylate d'éthyie.
Acide 3{3-[1-(5,5,8,8-tétraméthyl-5,6,7,8-tétrahydro-2-naphtyl) cyclopropyl]phenyl} acrylique.
3-{3-[Hydroxyimino-(3,5,5,8,8-pentaméthyl-5,6,7,8-tetrahydro-naphthalen-2-yl)-methyl]-phenyl}-acrylate d'éthyle.
Acide 3-(3-[Hydroxyinnino-(3,5,5,8,8-peniaméthyl-5,6,7,8-tetrahyaro-naphthalen-2-yl)-methyl]-phenyl}-acrylique.
3-{3-[Hydroxyimino-(5,5,8,8-tetramethyl-5,6,7,8-tetrahydro-naphthalen-2-yl)-methyl]-phenyl}-acrylate d'éthyle.
Acide 3-{3-[Hydroxyimino-(5,5,8,8-tétramethyl-5,6,7,8-tetrahydro-naphthalen-2-yl)-methyl]-phenyl}-acrylique.
3-{3-[2-(3,5,5,8,8-pentamethyl-5,6,7,8-tetrahydro-naphthalen-2-yl)-[1,3] dithian-2-yl]-phenyl}-acrylate d'éthyle.
Acide 3-{3-[2-(3,5,5,8,8-pentamethyl-5,6,7,8-tetrahydro-naphthalen-2-yl)-[1,3] dithian-2-yl]-phenyl}-acrylique
Acide 3-(3-[Hydroxylamine-(3,5,5,8,8-pentamethyl-5,6,7,8-tetrahydro-naphthalen-2-yl)-methyl]-phenyl}-acrylique.
3-{3-[Hydroxylamine-(5,5,8,8-tetramethyl-5,6,7,8-tetrahydro-naphthalen-2-yl)-methyl]-phenyl}-acrylate d'éthyle.
Acide 3-{2-[Hydroxylamine-(3,5,5,8,8-pentamethyl-5,6,7,8-tetrahydro-naphthalen-2-yl)-methyl]-phenyl}-acrylique.
3-{2-[Hydroxylamine-(5,5,8,8-tetramethyl-5,6,7,8-tetrahydro-naphthalen-2-yl)-methyl]-phenyl}-acrylate d'éthyle.
Acide {3-[1-(5,5,8,8-Tetramethyl-5,6,7,8-tetrahydro-naphthalen-2-yl)-cyclopropyl]-phenyl}-propynoique.
Acide {3-[1-(3,5,5,8,8-Pentamethyl-5,6,7,8-tetrahydro-naphthalen-2-yl)-cyclopropyl]-phenyl}-propynoique
Acide {2-(1-(5,5,8,8-Tetramethyl-5,6,7,8-tetrahydro-naphthalen-2-yl)-cyclopropyl]-phenyl}-propynoique
Acide {2-[1-(3,5,5,8,8-Pentamethyl-5,6,7,8-tetrahydro-naphthalen-2-yl)-cyclopropyl]-phenyl}-propynoique
Acide 3{2-[1-(3,5,5,8,8-Pentaméthyl-5,6,7,8-tétrahydro-2-naphtyl) cyclopropyl]phenyl} acrylique.
Acide 3{3-[1-(3,5,5,8,8-Pentaméthyl-5,6,7,8-tétrahydro-2-naphtyl) cyclopropyl]phenyl} acrylique.
Acide 3-{4-Hydroxy-3-[1-(5,5,8,8-tetramethyl-5,6,7,8-tetrahydro-naphthalen-2-yl)-cyclopropyl]-phenyl}-acrylique
Acide 3-{3-Hydroxy-2-[1-(5,5,8,8-tetramethyl-5,6,7,8-tetrahydro-naphthalen-2-yl)-cyclopropyl]-phenyl}-acrylique
Acide 3-{3-[1-(5,5,8,8-tetramethyl-5,6,7,8-tetrahydro-naphthalen-2-yl)-cyclopropyl]-4-méthoxyphenyl}-acrylique
Acide 3-{2-[1-(5,5,8,8-tetramethyl-5,6,7,8-tetrahydro-naphthalen-2-yl)-cyclopropyl]-3-méthoxyphenyl)-acrylique
Acide 3-{4-Hydroxy-3-[1-(3,5,5,8,8-pentamethyl-5,6,7,8-tetrahydro-naphthalen-2-yl)-cyclopropyl]-phenyl}-acrylique
Acide 3-{3-Hydroxy-2-[1-(3,5,5,8,8-pentamethyl-5,6,7,8-tetrahydro-naphthalen-2-yl)-cyclopropyl]-phenyl}-acrylique
Acide 3-{3-[1-(3,5,5,8,8-pentamethyl-5,6,7,8-tetrahydro-naphthalen-2-yl)-cyclopropyl]-4-méthoxyphenyl}-acrylique
Acide 3-{2-[1-(3,5,5,8,8-pentamethyl-5,6,7,8-tetrahydro-naphthalen-2-yl)-cyclopropyl]-3-méthoxyphenyl}-acrylique
3-{3-[1-(5,5,8,8-Tetramethyl-5,6,7,8-tetrahydro-naphthalen-2-yl)-cyclopropyl]-phenyl}-acrylamide
3-{2-[1-(5,5,8,8-Tetramethyl-5,6,7,8-tetrahydro-naphthalen-2-yl)-cyclopropyl]-phenyl}-acrylamide
3-{3-[1-(3,5,5,8,8-pentamethyl-5,6,7,8-tetrahydro-naphthalen-2-yl)-cyclopropyl]-phenyl}-acrylamide
3-{2-[1-(5,5,8,8-pentamethyl-5,6,7,8-tetrahydro-naphthalen-2-yl)-cyclopropyl]-phenyl}-acrylamide
*N*-Ethyl-3-{3-[1-(5,5,8,8-tetramethyl-5,6,7,8-tetrahydro-naphthalen-2-yl)-cyclopropyl]-phenyl}-acrylamide
*N*-Ethyl-3-{2-[1-(5,5,8,8-tetramethyl-5,6,7,8-tetrahydro-naphthalen-2-yl)-cyclopropyl]-phenyl}-acrylamide
*N*-Ethyl-3-{3-[1-(3,5,5,8,8-pentamethyl-5,6,7,8-tetrahydro-naphthalen-2-yl)-cyclopropyl]-phenyl}-acrylamide
*N*-Ethyl-3-{2-[1-(3,5,5,8,8-pentamethyl-5,6,7, 8-tetrahydro-naphthalen-2-yl)-cyclopropyl]-phenyl}-acrylamide
1-Morpholin-4-yl-3-{3-[1-(5,5,8,8-tetramethyl-5,6,7,8-tetrahydro-naphthalen-2-yl)-cyclopropyl]-phenyl}-propenone
1-Morpholin-4-yl-3-{2-[1-(5,5,8,8-tetramethyl-5,6,7,8-tetrahydro-naphthalen-2-yl)-cyclopropyl]-phenyl}-propenone
1-Morpholin-4-yl-3-{3-[1-(3,5,5,8,8-pentamethyl-5,6,7,8-tetrahydro-naphthalen-2-yl)-cyclopropyl]-phenyl}-propenone
1-Morpholin-4-yl-3-{2-[1-(3,5,5,8,8-pentamethyl-5,6,7,8-tetrahydro-naphthalen-2-yl)-cyclopropyl]-phenyl}-propenone
*N*-(4-Hydroxy-phenyl)-3-{3-[1-(5,5,8,8-tetramethyl-5,6,7,8-tetrahydro-naphthalen-2-yl)-cyclopropyl]-phenyl}-acrylamide
*N*-(4-Hydroxy-phenyl)-3-{2-[1-(5,5,8,8-tetramethyl-5,6,7,8-tetrahydro-naphthalen-2-yl)-cyclopropyl]-phenyl}-acrylamide
*N*-(4-Hydroxy-phenyl)-3-{3-[1-(3,5,5,8,8-pentamethyl-5,6,7,8-tetrahydro-naphthalen-2-yl)-cyclopropyl]-phenyl}-acrylamide
*N*-(4-Hydroxy-phenyl)-3-{2-[1-(3,5,5,8,8-pentamethyl-5,8,7,8-tetrahydro-naphthalen-2-yl)-cyclopropyl]-phenyl}-acrylamide
3-{3-[1-(5,5,8,8-Tetramethyl-5,6,7,8-tetrahydro-naphthalen-2-yl)-ethyl]-phenyl}-propenal
3-{2-[1-(5,5,8,8-Tetramethyl-5,6,7,8-tetrahydro-naphthalen-2-yl)-ethyl]-phenyl}-propenal
3-{3-[1-(3,5,5,8,8-Pentamethyl-5,6,7,8-tetrahydro-naphthalen-2-yl)-ethyl]-phenyl)-propenal
3-{2-[1-(3,5,5,8,8-Pentamethyl-5,6,7,8-tetrahydro-naphthalen-2-yl)-ethyl]-phenyl}-propenal
3-{3-[1-(5,5,8,8-Tetramethyl-5,6,7,8-tetrahydro-naphthalen-2-yl)-ethyl]-phenyl}-prop-2-en-1-ol
3-{2-[1-(5,5,8,8-Tetramethyl-5,6,7,8-tetrahydro-naphthalen-2-yl)-ethyl]-phenyl}-prop-2-en-1-ol
3-{3-[1-(3,5,5,8,8-Pentamethyl-5,6,7,8-tetrahydro-naphthalen-2-yl)-ethyl]-phenyl)-prop-2-en-1-ol
3-{2-[1-(3,5,5,8,8-Pentamethyl-5,6,7,8-tetrahydro-naphthalen-2-yl)-ethyl]-phenyl}-prop-2-en-1-ol

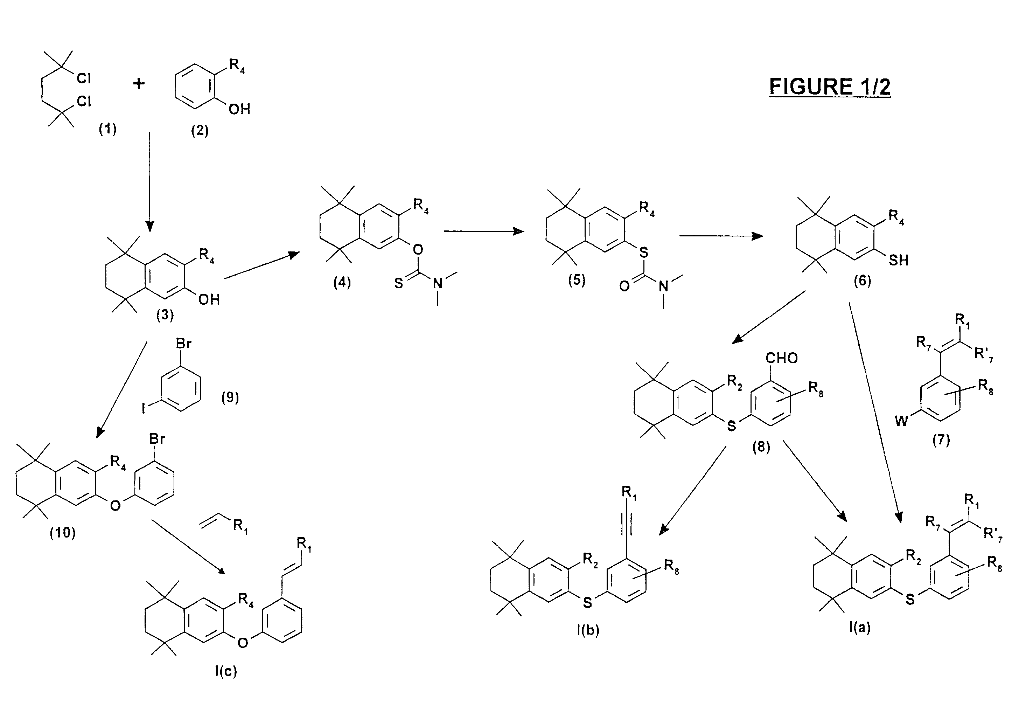

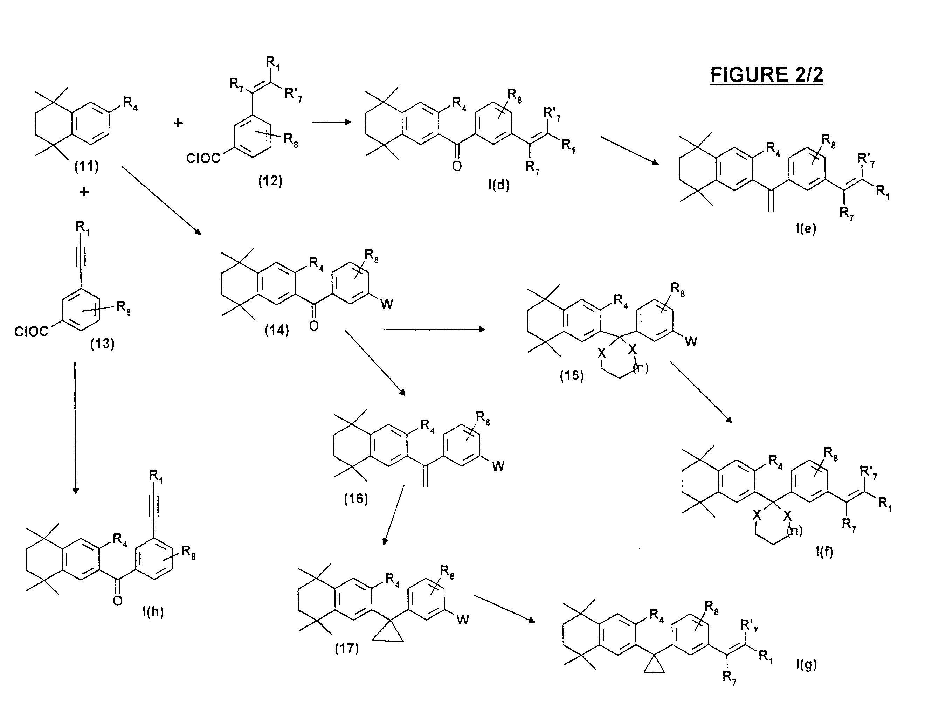

Selon la présente invention, les composés de formule (I) plus particulièrement préférés sont ceux pour lesquels l'une au moins, et de préférence toutes, les conditions ci-dessous sont respectées :
R₁ représente le radical -CO-R₆
Ar représente les radicaux de formule (c) ou (f)
X représente les radicaux de formule (g), (h), (n) ou (m)
R₂ et R₃ pris ensemble forment avec le cycle aromatique adjacent un cycle à 5 ou 6 chaînons éventuellement substitué par des groupes méthyle et/ou éventuellement interrompu par un atome d'oxygène ou de soufre.
La présente invention a également pour objet les procédés de préparation des composés de formule (I), en particulier selon les schémas réactionnels donnés aux figures 1 et 2.

Les composés de formule l(a) peuvent être obtenus (Fig. 1) à partir du sel de sodium ou de potassium des dérivés thiol (6) par couplage avec des dérivés halogénés (7), de préférence un dérivé bromé ou iodé en présence d'un catalyseur tel que certains complexes de métaux de transition dans un solvant alcoolique tel l'alcool éthylique ou butylique. Comme catalyseur on peut en particulier mentionner ceux dérivés du nickel ou du palladium par exemple les complexes de Ni II avec diverses phosphines et le tétrakis(triphénylphosphine)palladium(0).
Les dérivés thiols (6) pouvant être obtenus à partir des dérivés phénoliques (3) via les dérivés dialkylthiocarbamates (4) et (5) selon les conditions générales décrites par M. Newman et H. Karnes dans J. Org. Chem 1966 31 3980-4.
Les dérivés phénoliques (3) pouvant être obtenus par une réaction de type Friedel et Crafts à partir d'un phénol (2) et d'un dérivé dihalogéné (1) en présence d'un acide de Lewis par exemple le chlorure d'aluminium.

Les dérivés de formule I(a) lorsque R₁ est une fonction acide peuvent être aussi obtenus à partir des dérivés (8) par une réaction de type Horner-Emmons avec le triéthylphosphonoacétate en présence d'une base telle l'hydrure de sodium puis saponification de la fonction ester avec la soude ou la potasse dans un solvant alcoolique.

Les composés de formule l(b) peuvent être obtenus (Fig. 1) à partir des dérivés benzaldéhydes (8) par transformation de la fonction aldéhyde en fonction acétylène par exemple en utilisant la réaction de Corey-Fuchs puis lithiation et réaction avec par exemple le chloroformiate d'éthyle, le CO₂. Les dérivés (8) peuvent être obtenus à partir du sel de sodium ou de potassium des dérivés thiol (6) par couplage avec des dérivés benzaldéhydes de préférence un dérivé bromé ou iodé en présence d'un catalyseur tel que certains complexes de métaux de transition dans un solvant alcoolique tel l'alcool éthylique ou butylique.

Les composés de formule l(c) peuvent être obtenus (Fig. 1) par une réaction de type Heck entre des dérivés halogénés (10) et des esters de l'acide acrylique en présence de triéthylamine ou de carbonate de potassium et d'acétate de palladium et de triphénylphosphine. Les dérivés (10) pouvant être obtenus par couplage du sel de sodium des dérivés phénoliques (3) avec des dérivé halogénés (9), de préférence un dérivé iodé, en présence d'un complexe de bromure de cuivre et de diméthyl sulfure dans un solvant tel la pyridine.

Les dérivés de formule I(d) et I(h) peuvent être obtenus (Fig. 2) à partir des dérivés (11) par une réaction de type Friedel et Crafts avec respectivement les chlorures d'acide (12) et (13) dans un solvant tel le dichlorométhane en présence de chlorure d'aluminium.

Les dérivés de formule I(e) peuvent être (Fig. 2) obtenus à partir des dérivés l(d) par une réaction de type Wittig en utilisant le bromure de méthyltriphenyl phosphonium en présence d'une base telle le tert-butylate de potassium ou l'hexamethyldisilazide de potassium.

Les dérivés de formule l(f) et l(g) peuvent être obtenus (Fig. 2) par une réaction de type Heck entre respectivement les dérivés halogénés (15) et (16) et des esters de l'acide acrylique. Les dérivés (15) pouvant être obtenus par acétalisation ou thioacétalisation des dérivés cétoniques (14) par exemple en utilisant l'éthanedithiol ou le propanedithiol dans le dichlorométhane en présence d'un catalyseur comme le trifluorure de bore éthérate, ou en utilisant l'ethylèneglycol ou le propylèneglycol dans un solvant aromatique tel le toluène en présence d'acide para-toluènesulfonique.

Les dérivés (14) pouvant être obtenus par une réaction de type Friedel et Crafts entre les dérivés (11) et des chlorures d'acide halogénés plus particulièrement des chlorures d'acide iodés.

Les dérivés (17) pouvant être obtenus à partir des dérivés cétoniques (14) tout d'abord par une réaction de Wittig en utilisant le bromure de méthyltriphenyl phosphonium en présence d'une base telle le tert-butylate de potassium ou l'hexamethyldisilazide de potassium, puis par cyclopropanation soit en utilisant le chloroiodométhane et le diéthylzinc ou le diiodomethane et le zinc.

Lorsque R₁ représente le radical -COOH, les composés sont préferentiellement préparés en protégeant R₁ par un groupe protecteur de type allylique, benzylique ou tert-butylique.

Le passage à la forme libre peut être effectué :
- dans le cas d'un groupe protecteur allylique, au moyen d'un catalyseur tel certains complexes de métaux de transition en présence d'une amine secondaire.
- dans le cas d'un groupe protecteur benzylique, par débenzylation en présence d'hydrogène, au moyen d'un catalyseur tel le palladium sur charbon.
- dans le cas d'un groupe protecteur tert-butylique au moyen d'iodure de triméthylsilane.

Lorsque R₁ représente une fonction alcool les composés peuvent être obtenus à partir des dérivés aldéhydiques correspondants par action d'un hydrure alcalin, tel le borohydrure de sodium, dans un solvant alcoolique (par exemple le méthanol), ou par couplage du dérivé halogéné correspondant avec un dérivé de l'alcool 3-(tributylétain)allylique.

Lorsque R₁ représente une fonction aldéhyde les composés peuvent être obtenus à partir des dérivés alcools par oxydation en présence d'oxyde de manganèse, de pyridinium dichromate ou du réactif de Swern.

Lorsque R₁ représente une fonction amide les composés peuvent être obtenus à partir des dérivés carboxyliques correspondants par réaction avec des amines aliphatiques, aromatiques, hétérocycliques soit par l'intermédiaire d'un chlorure d'acide ou en présence de dicyclohexylcarbodiimide ou de carbonyidiimidazole.

Les produits de formule générale (I) peuvent servir de produits de départ pour la fabrication d'autres composés de formule générale (I). Ces dérivés seront obtenus selon les méthodes classiques de synthèse employées en chimie, telles que celles décrites dans "Advanced Organic Chemistry" de J. March; John Willey and Sons, 1985.

Par exemple, on peut procéder aux modifications fonctionnelles du groupe R₁ comme indiqué ci-dessous :
acide carboxylique → ester
ester → acide carboxylique
acide → chlorure d' acide
chlorure d' acide → amide
acide → amide
acide → alcool
alcool → aldéhyde
amide → amine
thiol → thioéther
thioéther → sulfoxyde
thioéther → sulfone
acide sulfonique → ester sulfonique
acide sulfonique → sulfonamide
acide sulfinique → ester sulfinique
Ces composés se lient aux récepteurs RXRs, certains possédant une activité agoniste, d'autres une activité antagoniste.

Les propriétés de binding et de transactivation comme agoniste aux récepteurs RXRs sont déterminées par des méthodes connues dans l'art, comme par exemple : MARTIN. B et all, Skin Pharmacol., 1992, **5,** 57-65 ; CAVEY. M. T. et al, Anal. Biochem., 1990, **186**, 19-23 ; LEVIN et al, Nature 1992, **355,** 359-61 ; ALLENBY et al, Proc. Natl. Acad. Sci., 1993, **90,** 30-4 ; ALLENBY et al, J. Biol. Chem., 1994, **269,** 16689-95.

L'activité agoniste RXRs est aussi déterminée par le test tel que décrit dans la demande de brevet français n° 95-07301 déposée le 19 juin 1995 par la demanderesse. Ce test comprend les étapes suivantes : (i) on applique topiquement sur une partie de la peau d'un mammifère une quantité suffisante d'un composé qui est un ligand actif d'au moins un récepteur de la superfamille des récepteurs nucléaires stéroïdiens/thyroïdiens autre qu'un ligand spécifique des récepteurs RXRs et pouvant s'hétérodimériser avec les RXRs tel qu'une molécule agoniste des RARs, (ii) on administre par voie systémique ou topique sur cette même partie de la peau du mammifère avant, pendant ou après l'étape (i) une molécule susceptible de présenter une activité agoniste des RXRs, (iii) on évalue la réponse sur la partie de la peau ainsi traitée du mammifère. Ainsi la réponse à une application topique sur l'oreille d'un mammifère d'une molécule agoniste des RARs qui correspond à une augmentation de l'épaisseur de cette oreille peut être augmentée par l'administration par voie systémique ou topique d'une molécule agoniste des récepteurs RXRs.

L'activité antagoniste RXRα est évaluée dans le test de transactivation par détermination de la dose (IC₅₀) qui inhibe de 50% l'activité transactivatrice d'un agoniste sélectif RXRα: l'acide 6-(3,5,5,8,8-pentaméthyl-5,6,7,8-tétrahydro-2-naphthylthio)nicotinique (CD 2809) selon le protocole suivant :
Les cellules Hela sont co-transfectées avec un vecteur d'expression codant pour RXRα (p565-RXRα) et un plasmide rapporteur contenant l'élément de réponse 1/2 CRBP II cloné en amont du promoteur hétérologue de la thymidine kinase et du gène rapporteur de la chloramphènicolm-acétyl-transfèrase (CAT). Dix-huit heures après co-transfection les cellules sont traitées avec une concentration fixe du CD 2809 et des concentrations croissantes de la molécule à évaluer. Après vingt-quatre heures de traitement le dosage de l'activité CAT est effectué par ELISA. La concentration fixe de CD2809 utilisée est 5 10⁻⁸M et correspond à son EC₅₀.

La présente invention a également pour objet à titre de médicament les composés de formule (I) telle que définie ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique, ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation, pour prévenir ou pour réparer les vergetures, ou encore pour favoriser la cicatrisation,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose ou l'hypertension ainsi que le diabète non-insulino dépendant,
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphényl-imidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

### Acide3-[3-(5,5,8,8-tetramethyl-5,6,7,8-tetrahydro-2-naphthylcarbonyl) phenyl] acrylique.

(a) Chlorure de 3-iodobenzoyle.
   Dans un ballon, on introduit une solution de 15 g (0,06 mole) d' acide 3-iodobenzoïque dans 100 ml de dichlorométhane anhydre et ajoute 13 ml (0,063 mole) de dicyclohexylamine et agite pendant une heure. On ajoute ensuite 4,6 ml (0,063 mole) de chlorure de thionyle et agite une heure. On évapore à sec, reprend par l'éther éthylique anhydre, filtre le sel de dicyclohexylamine et évapore le filtrat. On recueille 17 g (100%) du chlorure d'acide brut qui sera utilisé tel quel pour la suite de la synthèse.
(b) 3-iodophenyl-(5,5,8,8-tetramethyl-5,6,7,8-tetrahydro-2-naphthyl)methanone.
   Dans un tricol et sous courant d'azote, on introduit 10,3 g (55 mmoles) de 5,5,8,8-tetramethyl-5,6,7,8-tetrahydro-2-naphthalene 8,1 g (60,5 mmoles) de chlorure d'aluminium et 100 ml de dichlorométhane. A 0°C, on introduit goutte à goutte une solution de 16,1 g (65 mmoles) de chlorure de 3-iodobenzoyle préparé précédemment dans 50 ml de dichlorométhane et laisse remonter à température ambiante. On verse le milieu réactionnel dans l'eau, extrait avec du dichlorométhane, décante la phase organique, sèche sur sulfate de magnésium, évapore. Le résidu obtenu est purifié par chromatographie sur colonne de silice élué avec un mélange de dichlorométhane et d'heptane (50-50). Après évaporation des solvants, on recueille 12,5 g (54,6%) du dérivé cétonique attendu de point de fusion 130-1°C.
(c) 3-[3-(5,5,8,8-tetramethyl-5,6,7,8-tetrahydro-2-naphthylcarbonyl)phenyl]acrylate de méthyle.
   Dans un tricol et sous courant d'azote, on introduit une solution de 2 g (4,8 mmoles) de 3-iodophenyl-(5,5,8,8-tetramethyl-5,6,7,8-tetrahydro-2-naphthyl) methanone dans 30 ml de DMF et ajoute successivement 1,7 g de carbonate de potassium, 1,4 g de chlorure de tétrabutylammonium et 600 µl (6,2 mmoles) d'acrylate de méthyle. On dégaze le milieu réactionnel en faisant barboter dans la solution un courant d'argon et introduit 22 mg (0,1 mmole) d'acétate de palladium. On chauffe à 55°C pendant six heures, verse le milieu réactionnel dans l'eau, extrait avec de l'acétate d'éthyle, décante la phase organique, lave à l'eau, sèche sur sulfate de magnésium, évapore. Le résidu obtenu est purifié par chromatographie sur colonne de silice élué avec un mélange d'acétate d'éthyle et d'heptane (90-10). Après évaporation des solvants, on recueille 1,39 g (77%) de l'ester méthylique attendu de point de fusion 103-4°C.
(d) acide 3-[3-(5,5,8,8-tetramethyl-5,6,7,8-tetrahydro-2-naphthylcarbonyl)phenyl] acrylique.
   Dans un ballon, on introduit une solution de 1,8 g (4,8 mmoles) de l'ester méthylique précédent dans 20 ml de THF et ajoute 20 ml d'une solution de soude 2N. On agite à température ambiante pendant six heures, évapore à sec le milieu réactionnel. On reprend le résidu dans l'eau, acidifie à pH 1 avec de l'acide chlorhydrique, extrait avec de l'éther éthylique, décante la phase organique, lave à l'eau, sèche sur sulfate de magnésium, évapore. Le résidu est trituré dans l'heptane, filtré, séché. on recueille 1,5 g (86%) d'acide 3-[3-(5,5,8,8-tetramethyl-5,6,7,8-tetrahydro-2-naphthylcarbonyl)phenyl]acrylique de point de fusion 160-5°C.

### EXEMPLE 2

### Acide 3-[3-(3,5,5,8,8-pentamethyl-5,6,7,8-tetrahydro-2-naphthylcarbonyl) phenyl]acrylique.

(a) 3-iodophenyl-(3,5,5,8,8-pentamethyl-5,6,7,8-tetrahydro-2-naphthyl)methanone.
   De manière analogue à l'exemple 1(b) par réaction de 11,8 g (58 mmoles) de 3,5,5,8,8-pentamethyl-5,6,7,8-tetrahydro-2-naphthalène avec 18,7 g (68 mmoles) de chlorure de 3-iodobenzoyle, on obtient 6,5 g (82%) du dérivé cétonique attendu sous forme d'une huile jaunâtre.
(b) 3-[3-(3,5,5,8,8-pentamethyl-5,6,7,8-tetrahydro-2-naphthylcarbonyl)phenyl] acrylate de methyle.
   De manière analogue à l'exemple 1(c) par réaction de 6,54 g (15 mmoles) de 3-iodophenyl-(3,5,5,8,8-pentamethyl-5,6,7,8-tetrahydro-2-naphthyl)methanone avec 1,8 ml (19,7 mmoles) d'acrylate de méthyle, on obtient 6,5 g (100%) de l'ester méthylique attendu sous forme d'une huile orangée.
(c) acide 3-[3-(3,5,5,8,8-pentamethyl-5,6,7,8-tetrahydro-2-naphthylcarbonyl) phenyl]acrylique.
   De manière analogue à l'exemple 1d) à partir de 6,5 g (15 mmoles) de l'ester méthylique précédent, on obtient 2,1 g (38%) d'acide 3-[3-(3,5,5,8,8-pentamethyl-5,6,7,8-tetrahydro-2-naphthylcarbonyl)phenyl]acrylique de point de fusion 176-7°C.

### EXEMPLE 3

### Acide 3-[3-(5,5,8,8-tetramethyl-5,6,7,8-tetrahydro-2-naphthylthio)phenyl] acrylique.

Dans un tricol et sous courant d'azote, on introduit 1,26 g (5,7 mmoles) de 5,6,7,8-tétrahydro-5,5,8,8-tétraméthyl-2-naphtylthiol 1,3 g (5,7 mmoles) d'acide 3-bromocinnamique et 100 ml de d'alcool tert-butylique. On introduit par petites quantités 2,3 g (20 mmoles) de tert-butylate de potassium puis 200 mg de tétrakis(triphénylphosphine)palladium(0) puis chauffe à reflux pendant huit heures. On verse le milieu réactionnel dans l'eau, ajuste à pH 5 avec de l'acide chlorhydrique 1N, extrait avec de l'acétate d'éthyle, sèche sur sulfate de magnésium, évapore. Le résidu obtenu est purifié par chromatographie sur colonne de silice élué avec un mélange d'acétate d'éthyle et d'heptane (50-50). Après évaporation des solvants, on recueille 610 mg (29%) d'acide 3-[3-(5,5,8,8-tetramethyl-5,6,7,8-tetrahydro-2-naphthylthio)phenyl] acrylique de point de fusion 193-4°C.

### EXEMPLE 4

### Acide 3-[3-(3,5,5,8,8-pentamethyl-5,6,7,8-tetrahydro-2-naphthylthio)phenyl] acrylique.

(a) 3,5,5,8,8-pentaméthyl-5,6,7,8-tétrahydro-2-naohtol.
   Dans un tricol on introduit 50,8 g (0,27 mole) de 2,5-dichloro-2,5-diméthyl hexane 30 g (0,27 mole) de 2-méthyiphénol et 500 ml de dichlorométhane. A 0°C on ajoute par petites quantités 14,8 g (0,11 mole) de chlorure d'aluminium et agite à température ambiante pendant douze heures. On verse le milieu réactionnel dans l'eau glacée, extrait avec le dichlorométhane, décante la phase organique, lave à l'eau bicarbonatée, sèche sur sulfate de magnésium, évapore. Le résidu obtenu est trituré dans l'hexane, filtré, on recueille après séchage 54,4 g (90%) de phénol attendu de point de fusion 125-6°C.
(b) O-3,5,5,8,8-pentaméthyl-5,6,7,8-tétrahydro-2-naphtyldimethylthiocarbamate,
   Dans un ballon et sous courant d'azote, on introduit 4,1 g (0,138 mole) d'hydrure de sodium (80% dans l'huile) et 200 ml de DMF. On refroidit à 0°C et ajoute goutte à goutte une solution de 25,2 g (0,115 mole) de 3,5,5,8,8-pentaméthyl-5,6,7,8-tétrahydro-2-naphtol dans 100 ml de DMF et agite jusqu'à cessation du dégagement gazeux, on ajoute ensuite une solution de 18,55 g (0,15 mole) de chlorure de dimethylthiocarbamoyle dans 200 ml de DMF et agite pendant huit heures à température ambiante. On verse le milieu réactionnel dans l'eau, extrait avec de l'acétate d'éthyle, décante la phase organique, lave à l'eau, sèche sur sulfate de magnésium, évapore. Le solide obtenu est purifié par chromatographie sur colonne de silice élue avec un mélange d'acétate d'éthyle et d'hexane (30-70). Après évaporation des solvants, on recueille 20 g (68%) du produit attendu de point de fusion 110-1°C.
(c) S-3,5,5,8,8-pentaméthyl-5,6,7,8-tétrahydro-2-naphtyldimethyrthiocarbamate.
   Dans un ballon et sous courant d'azote, on introduit 20,1 g (65,8 mmoles) du produit précédent et chauffe à 240°C pendant six heures. On extrait le milieu réactionnel avec du dichloromethane, lave à l'eau, décante la phase organique, sèche sur sulfate de magnésium, évapore. On recueille 18,1 g (90%) du produit attendu de point de fusion 138-9°C.
(d) 3,5,5,8,8-pentaméthyl-5,6,7,8-tétrahydro-2-naphtylthiol.
   Dans un ballon, on introduit 23 g (75 mmoles) du produit précédent et 300 ml d'alcool methylique. On ajoute 30 g (750mmoles) d'hydroxyde de sodium et chauffe à reflux pendant trois heures. On évapore le milieu réactionnel, reprend par l'eau, acidifie avec de l'acide chlorhydrique concentré, filtre. Le solide obtenu est lavé à l'eau, séché, on recueille 18 g (99%) de 3,5,5,8,8-pentaméthyl-5,6,7,8-tétrahydro-2-naphtylthiol de point de fusion 97-8°C.
(e) acide 3-[3-(3,5,5,8,8-pentaméthyl-5,6,7,8-tetrahydro-2-naphthylthio)phenyl] acrylique
   Dans un tricol et sous courant d'azote, on introduit 5 g (21,4 mmoles) de 5,6,7,8-tétrahydro3-,5,5,8,8 pentaméthyl-2-naphtylthiol 5g (21,4 mmoles) d'acide 3-bromocinnamique et 100 ml de d'alcool tert-butylique. On introduit par petites quantités 8,4 g (74,8 mmoles) de tert-butylate de potassium puis 650 mg de tétrakis(triphénylphosphine)palladium(0) puis chauffe à reflux pendant huit heures. On verse le milieu réactionnel dans l'eau, ajuste à pH 5 avec de l'acide chlorhydrique 1N, extrait avec de l'acétate d'éthyle, sèche sur sulfate de magnésium, évapore. Le résidu obtenu est purifié par chromatographie sur colonne de silice élué avec un mélange d'acétate d'éthyie'et d'heptane (70-30). Après évaporation des solvants, on recueille 5,6 g (69%) d'acide 3-[3-(3,5,5,8,8-pentamethyl-5,6,7,8-tetrahydro-2-naphthylthio)phenyl] acrylique de point de fusion 198-9°C.

### EXEMPLE 5

### 3{2-[1-(3,5,5,8,8-pentaméthyl-5,6,7,8-tétrahydro-2-naphthyl)vinyl] phenyl} acrylate d'éthyle.

a) 6-[1-(2-iodophényl)-vinyl]-1,1,4,4,7-pentaméthyl-1,2,3,4-tétrahydro-naphtalène.
   Du tert-butylate de potassium (7,26 g 64,8 mmol) est additionné à une solution de 2-iodophényl-(3,5,5,8,8-pentaméthyl-5,6,7,8-tétrahydro-2-naphthyi) méthanone (20g, 46,3 mmol) et de bromure de méthyltriphénylphoshonium (21,5g, 60,2 mmol) dans le THF (100 ml). Le mélange est agité 20h à température ambiante. La solution est extraite par de d'acétate d'éthyle. Après décantation la phase organique est lavée deux fois par 40 ml d'eau, séchée sur sulfate de magnésium anhydre et concentré à l'évaporateur rotatif sous vide à 40°C. Le produit est purifié parchromatographie flash sur colonne de silice.
   Solide blanc. Masse: 17,5 g, Rendement: 88%.
   RMN d ppm:
   1H/CDCl3: 1.23 (s, 6H); 1.27 (s, 6H); 1.66 (s,4H); 2.30 (s, 3H); 5.48 à 5.53 (dd, 2H); 6.88 à 7.33 (m, 5H), 7.89 (d, 1H).
   13C/CDCl3: 14.1, CH3/ 31.9, 4^{*}CH3/ 33.8, 2^{*}Cq/ 35.2, 2^{*}CH2/ 98.0, Cq(C-l)/ 120.3, Cq (C=C)/127.8, CH/ 128.4, CH/ 128.5, CH/ 128.8, CH/ 130.4, CH/ 132.8, Cq/ 137.8, Cq/ 140.1, CH/ 142.0, Cq/ 144.2, Cq/ 146.8, Cq/ 151.4, Cq.
(b) *2-[1-(3,5,5,8,8-pentaméthyl-5,6,7,8-tétrahydro-2-naphthyl)vinyl] phenyl carboxylate de méthyle.*
   Une solution de 6-[1-(2-iodophényl)-vinyl]-1,1,4,4,7-pentaméthyl-1,2,3,4-tétrahydro-naphtalène (17,2 g, 40 mmol), de diacétate de palladium (1,02 g, 4,5 mmol), de tributylamine (21,9 ml, 92 mmol) dans le méthanol (500 ml) est chauffée 3h à 100°C sous pression de monoxyde de carbone (3 bars). Après concentration à l'évaporateur sous vide à 40°C, l'huile obtenue est diluée dans l'acétate d'éthyle et lavée trois fois à l'eau. Le produit est purifié par chromatographie flash sur colonne de silice.
   Solide brun. Masse: 9,9 g. Rendement: 69%. TF: 53°C.
   1H/CDCl3: 1.22 (s, 6H); 1.26 (s, 6H); 1.65 (s, 4H); 2.06 (s, 3H); 3.49 (s, 3H); 5.38 à 5.48 (dd, 2H); 7.03 (s, 1H); 7.06 (s, 1H); 7.29 à 7.46 (m, 4H); 7.53 à 7.56 (m, 1H).
   13C/CDCl3: 20.6, CH3/ 31.8, 4^{*}CH3/ 33.8, 2^{*}Cq/ 35.2, 2^{*}CH2/ 51.9, CH3/ 118.0, Cq/ 127.2, CH/ 128.4, CH/ 128.6, CH/ 128.9, CH/ 130.2, CH/ 130.7, CH/ 131.7, Cq/ 132.9, Cq/ 138.0, Cq/ 141.7, Cq/ 142.5, Cq/ 144.1, Cq/ 149.2, Cq/ 169.5, Cq.
(c) *2-[1-(3,5,5,8,8-pentaméthyl-5,6,7,8-tétrahydro-2-naphthyl)vinyl] phenyl carbaldéhyde.*
   Une solution 1M d'hydrure de diisobutylaluminium dans le toluène (17,3 ml, 17,3 mmol) est additionnée à 0°C, goutte à goutte à une solution de 2-[1-(3,5,5,8,8-pentaméthyl-5,6,7,8-tétrahydro-2-naphthyl)vinyl] phenyl carboxylate de méthyle (2,5 g, 6,9 mmol) dans le toluène (50ml). La solution est agitée 1h à 0°C, puis traitée par une solution de tartrate double de sodium et potassium filtrée et reprise dans un mélange d'éther éthylique et d'eau. La phase organique est lavée à l'eau, séchée sur sulfate de magnésium et concentrée à l'évaporateur rotatif sous vide à 40°C.
   L'huile obtenue est agitée à TA 4h en présence de dichromate de pyridinium (5g, 13,3 mmol) dans le CH₂Cl₂ (50 ml), puis la solution est filtrée sur silice et concentrée à l'évaporateur rotatif sous vide à 40°C. Le produit est purifié par chromatographie flash sur colonne de silice.
   Solide blanc. Masse: 1,2 g, Rendement: 52%. F= 55°C
   1H/CDCl3: 1.26 (s, 6H); 1.28 (s, 6H); 1.68 (s, 4H); 2.18 (s, 3H); 5.30 (d, 1H); 5.71 (d, 1H); 7.05 (s, 1H); 7.16 (s, 1H); 7.22 (dd, 1H); 7.39 (dt, 1H); 7.49 (dt, 1H); 7.96 (dd, 1H); 10.32 (s, 1H).
   13C/CDCl3: 20.5, CH3/ 31.7, 4^{*}CH3/ 33.8, 2^{*}Cq/ 34.5, 2^{*}CH2/ 122.8, CH2/ 127.5, CH/ 127.7, CH/ 128.1, CH/ 128.7, CH/ 129.8, CH/ 132.4, Cq/ 133.1, CH/ 134.1, Cq/138.4, Cq/ 142.5, Cq/ 144.8, Cq/ 146.3, Cq/ 146.4, Cq/ 192.1, Cq
(d) *3{2-[1-(3,5,5,8,8-pentaméthyl-5,6,7,8-tétrahydro-2-naphthyl)vinyl] phenyl} acrylate d'éthyle.*
   De l'hydrure de sodium à 75% dans l'huile (140 mg, 4,4 mmol), est additionné à un mélange de 2-[1-(3,5,5,8,8-pentaméthyl-5,6,7,8-tétrahydro-2-naphthyl)vinyl] phenyl carbaldéhyde (1,2 g, 3,6 mmol) et de triéthylphosphonoacétate (1,1 ml, 5,5 mmol) dans le THF (20ml). Le mélange est agité 2h à température ambiante, extrait à l'acétate d'éthyle et lavé à l'eau. Après séchage la phase organique est concentrée à l'évaporateur rotatif sous vide à 40°C, le produit est purifié par chromatographie flash sur colonne de silice.
   Masse: 1,28 g, Rendement: 88%.
   RMN (250 MHz):
   1H/CDCl3: 1.13 à 1.33 (m, 15H); 1.67( s, 4H); 1.97 (s, 3H); 4.21 (q, 2H); 5.27 (d, 1H); 5.60 (s, 1H); 6.3 (d, h); 7.01 (s, 1H); 7.1 à 7.21 (m, 2H);
   7.23 à 7.31 (m, 2H); 7.60 (m, 1H); 8.05 (d, 1H).
   13C/CDCl3: 14.3, CH3/ 20.4, CH3/ 31.8, 4^{*}CH3/ 33.9, 2^{*}Cq/ 35.2, 2^{*}CH2/ 60.3, CH2/ 118.8, CH/ 121.4, CH2/ 127.1, CH/ 127.4, CH/ 128.1, CH/ 128.4, CH/ 129.4, CH/ 129.6, CH/ 132.5, Cq/ 132.9, Cq/ 139.0, Cq/ 142.3, Cq/ 143.2, Cq/ 144.2, CH/ 144.3, Cq/ 148.1, Cq/ 166.8, Cq.

### EXEMPLE 6

### Acide 3{2-[1-(3,5,5,8, 8-pentaméthyl-5,6,7,8-tétrahydro-2-naphthyl) vinyl]phenyl}acrylique.

Une solution du produit de l'exemple 5 (1,28 g, 3,2 mmol) et d'hydroxyde de sodium (1,3 g) dans le THF (50ml) est chauffée 6h à reflux, acidifiée à pH 1 (HCI concentré), extraite à l'acétate d'éthyle et lavée à l'eau. Après séchage la phase organique est concentré à l'évaporateur rotatif sous vide à 40°C, le produit est lavé à l'heptane.
Masse: 880 mg, Rendement: 74%. Fp: 200°C.
1H/CDCl3: 1.26 (s, 12H); 1.67 (s, 4H); 1.93 (s, 3H); 5.29 (d, 1H); 5.61 (d, 1H); 6.28 (d, 1H); 7.01 (s, 1H); 7.18 (s, 1H); 7.20 (m, 1H); 7.25 à 7.35 (m, 2H); 7.61 (m, 1H); 8.12 (d, 1H).
13C/CDCl: 20.4; CH3/ 31.8; 4^{*}CH3/ 33.9; 2^{*}Cq/ 35.2; 2^{*}CH2/ 117.6; CH/ 121.4; CH2/ 127.2; CH/ 127.5; CH/ 128.1; CH/ 128.5; CH/ 129.7; CH/ 129.9; CH/ 132.5; Cq/ 139.0; Cq/ 142.4; Cq/ 143.4; Cq/ 144.5; Cq/ 146.6; Cq/ 148.3; CH/ 171.9; Cq.

### EXEMPLE 7

### 3{3-[1-(3,5,5,8,8-pentaméthyl-5,6,7,8-tétrahydro-2-naphthyl)vinyl]phenyl} acrylate d'éthyle.

(a) 6-[1-(3-iodophényl)-vinyl]-1,1,4,4,7-pentaméthyl-1,2,3,4-tétrahydro-naphtalène
   Du tert-butylate de potassium (7,26 g, 64,8) est additionné à une solution de 3-iodophényl-(3,5,5,8,8-pentaméthyl-5,6,7,8-tétrahydro-2-naphthyl)méthanone (20g, 46,3 mmol) et de bromure de méthyltriphénylphoshonium (21,5g, 60,2 mmol) dans le THF (100 ml). Le mélange est agité 20h à température ambiante. La solution est extraite par de l'acétate d'éthyle. Après décantation la phase organique est lavée deux fois par 40 ml d'eau, séchée sur sulfate de magnésium anhydre et concentrée à l'évaporateur rotatif sous vide à 40°C. Le produit est purifié par chromatographie flash sur colonne de silice.
   Solide blanc. Masse: 19,8 g, Rendement: 99%. Fp: 60°C.
   RMN d ppm:
   1H/CDCl3: 1.26 (s, 6H); 1.29 (s, 6H); 1.69 (s, 4H); 1.96 (s, 3H); 5.22 (d, 1H); 5.68 (s, 1H); 6.98 à 7.29 (m, 6H); 7.58 (dt, 1H); 7.70 (t, 1H).
   13C/CDCl3: 20.0, CH3/ 31.9, 4^{*}CH3/ 33.9, 2^{*}Cq/ 35.2, 2^{*}CH2/ 94.5, Cq/ 116.0, CH/ 128.2, 2^{*}CH/ 129.9, CH/ 132.7, Cq/ 135.3, CH/ 136.3, CH/ 137.8, Cq/ 142.2, Cq/ 143.4, Cq/ 144.3, Cq/ 148.6, Cq.
(b) 3-[1-(3,5,5,8,8-pentaméthyl-5,6,7,8-tétrahydro-2-naphthyl)vinyl] phenyl carboxylate de méthyle
   Une solution de 6-[1-(3-iodophényl)-vinyl]-1,1,4,4,7-pentaméthyl-1,2,3,4-tétrahydro-naphtalène (10,8 g, 25 mmol), de diacétate de palladium (650 mg, 2,9 mmol), de tributylamine (13,8 ml, 58 mmol) dans le méthanol (300 ml) est chauffée 3h à 100°C sous pression de monoxyde de carbone (3 bars). Après concentration à l'évaporateur sous vide à 40°C, l'huile obtenue est diluée dans l'acétate d'éthyle et lavée trois fois à l'eau. Le produit est purifié par chromatographie flash sur colonne de silice.
   Solide brun. Masse: 7,7 g. Rendement: 86%. TF: 75°C.
   1H/CDCl3: 1.28 (s, 6H); 1.30 (s, 6H); 1.70 (s, 4H); 1.95 (s, 3H); 3.90 (s, 3H) 5.28 (d, 1H); 5.77 (s, 1H); 7.07 (s, 1H); 714 (s, 1H); 7.32 à 7.37 (m,2H); 7.90 à 7.95 (s, 1H); 8.10 (s,1H).
   13C/CDCl3: 19.8, CH/ 31.7, 4^{*}CH3/ 33.7, 2^{*}Cq/ 35.0, 2^{*}CH2/ 51.9, CH3/ 115.8, CH2/ 127.3, CH/ 127.9, 2^{*}CH/ 128.1, CH/ 128.3, CH/ 130.0, Cq/ 131.2, CH/ 132.5, Cq/137.9, Cq/ 141.4, Cq/ 142.1, Cq/ 144.1, Cq/ 148.9, Cq/ 167.0, Cq
(c) *3-[1-(3,5,5,8,8-pentaméthyl-5,6,7,8-tétrahydro-2-naphthyl)vinyl] phenyl carbaldéhyde.*
   Une solution 1M d'hydrure de diisobutylaluminium dans le toluène (12,5 ml, 12,5 mmol) est additionnée à 0°C, goutte à goutte à une solution de 3-[1-(3,5,5,8,8-pentaméthyl-5,6,7,8-tétrahydro-2-naphthyl)vinyl] phenyl carboxylate de méthyle (1,8 g, 5 mmol) dans le toluène (30ml). La solution est agitée 1h à 0°C, puis traitée par une solution de tartrate double de sodium et potassium filtrée et reprise dans un mélange d'éther éthylique et d'eau. La phase organique est lavée à l'eau, séchée sur sulfate de magnésium et concentrée à l'évaporateur rotatif sous vide à 40°C.
   L'huile obtenue est agitée à TA 4h en présence de dichromate de pyridinium (3,6 g, 9,6 mmol) dans le CH₂Cl₂ (50 ml), puis la solution est filtrée sur silice et concentrée à l'évaporateur rotatif sous vide à 40°C. Le produit est purifié par chromatographie flash sur colonne de silice.
   Solide blanc. Masse: 1,6 g, Rendement: 87%. F= 62°C
   RMN:
   1H/CDCl3: 1.28 (s, 6H); 1.31 (s, 6H); 1.70 (s, 4H); 1.95 (s, 3H); 5.31 (d, 1H); 579 (d, 1H); 7.08 (s, 1H); 7.14 (s, 1H); 7.41 à 7.53 (m, 2H); 7.78 (dt, 1H); 7.84 (t, 1H); 10.00 (s, 1H).
   13C/CDCl3: 20.3, CH3/ 32.2, 4^{*}CH3/ 34.2, 2^{*}Cq/ 35.5, 2^{*}CH2/ 116.7, CH2/ 125.9, CH/ 128.1, CH/ 128.36, CH/ 128.44, CH/ 128.8, CH/ 132.9, Cq/ 138.1, CH/ 142.5, Cq/ 142.7, Cq/ 149.1, Cq/ 192.8, Cq.
(d) *3-{3-[1-(3,5,5,8,8-pentaméthyl-5,6,7,8-tétrahydro-2-naphthyl)vinyl] phenyl} acrylate d'éthyle.*
   De l'hydrure de sodium à 75% dans l'huile (183 mg, 5,7 mmol), est additionné à un mélange de 3-[1-(3,5,5,8,8-pentaméthyl-5,6,7,8-tétrahydro-2-naphthyl)vinyl] phenyl carbaldéhyde (1,57 g, 4,7 mmol) et de triéthylphosphonoacétate (1,44 ml, 7,25 mmol) dans le THF (30ml). Le mélange est agité 1h à température ambiante, extrait à l'acétate d'éthyle et lavé à l'eau. Après séchage la phase organique est concentrée à l'évaporateur rotatif sous vide à 40°C, le produit est purifié par chromatographie flash sur colonne de silice.
   Masse: 1,5 g, Rendement: 81%.
   RMN ¹H (250 MHz):
   1H/CDCl3: 1.28 à 1.31 (m, 15H); 1.70 (s, 4H); 1.95 (s, 3H); 4.25 (q, 2H); 5.25 (d, 1H); 5.73 (d, 1H); 6.41 (d, 1H); 7.07 (s, 1H); 7.13 (s, 1H); 7.25 à 7.45 (m, 4H); 7.64 (d, 1H).
   13C/CDCl3: 14.3, CH3/ 19.9, CH3/ 31.8, 4^{*}CH3/ 33.8, Cq/ 35.2, CH2/ 60.5, CH2/ 115.5, CH2/ 118.2, CH/ 126.56, CH/ 125.61, CH/ 128.0, 2^{*}CH/ 128.6, CH/ 128.8, CHI 132.6, Cq/ 134.4, Cq/ 138.1, Cq/ 141.8, Cq/ 142.2, Cq/ 144.2, Cq/ 144.7; CH/ 149.2, Cq/ 167.0, Cq.

### EXEMPLE 8

### Acide 3{3-[1-(3,5,5,8,8-pentaméthyl-5,6,7,8-tétrahydro-2-naphthyl)vinyl] phenyl}acrylique.

Une solution du produit de l'exemple 7 (1,5 g, 3,7 mmol) et d'hydroxyde de sodium (1,5 g) dans le THF (50ml) est chauffée 8h à reflux, acidifiée à pH 1 (HCI concentré), extraite à l'acétate d'éthyle et lavée à l'eau. Après séchage la phase organique est concentrée à l'évaporateur rotatif sous vide à 40°C, le produit est lavé à l'heptane.
Masse: 1,1 g, Rendement: 77%. Fp: 195°C.
1H/CDCl3: 1.28 (s, 6H); 1.3 (s, 6H); 1.70 (s, 4H); 1.96 (s, 3H); 5.27 (d, 1H); 5.74 (d, 1H); 6.41 (d; 1H); 7.08 (s, 1H); 7.14 (s, 1H); 7.31 à 7.49 (m, 4H); 7.75 (d, 1H) 13C/CDCl3: 20.3; CH3/ 32.2; 4^{*}CH3/ 34.2; Cq/ 34.3; Cq/ 35.5; 2^{*}CH2/ 116.1; CH2/ 117.6; CH/ 127.25; CH/ 127.3; CH/ 128.4; 2^{*}CH/ 129.3; CH/ 129.5; CH/ 133.0; Cq/ 134.4; Cq/ 138.4; Cq/ 142.3; Cq/ 142.6; Cq/ 144.6; Cq/ 147.6: CH/ 149.5; Cq/172.5; Cq.

### EXEMPLE 9

### 3{2-[1-(5,5,8,8-tétraméthyl-5,6,7,8-tétrahydro-2-naphthyl)vinyl]phenyl} acrylate d'éthyle.

(a) 6-[1-(2-iodophényl)-vinyl]-1,1,4,4-tétraméthyl-1,2,3,4-tétrahydro-naphtalène
   Du tert-butylate de potassium (4,03 g, 36 mmol) est additionné à une solution de 2-iodophényl-(5,5,8,8-tétraméthyl-5,6,7,8-tétrahydro-2-naphthyl)méthanone (10,5 g, 25,1 mmol) et de bromure de méthyltriphénylphoshonium (12 g, 33,6 mmol) dans le THF (50 ml). Le mélange est agité 4h à température. La solution est extraite par de l'acétate d'éthyle. Après décantation la phase organique est lavée deux fois par 40 ml d'eau, séchée sur sulfate de magnésium anhydre et concentrée à l'évaporateur rotatif sous vide à 40°C. Le produit est purifié par chromatographie flash sur colonne de silice.
   Solide blanc. Masse: 9,54 g, Rendement: 91%.
(b) 2-[1-(5,5,8,8-tétraméthyl-5,6,7,8-tétrahydro-2-naphthyl)vinyl] phenyl carboxylate de méthyle
   Une solution de 6-[1-(2-iodophényl)-vinyl]-1,1,4,4-tétraméthyl-1,2,3,4-tétrahydro-naphtalène (9,5 g, 22 mmol), de diacétate de palladium (560 mg, 2,5 mmol), de tributylamine (12 ml, 50 mmol) dans le méthanol (500 ml) est chauffée 3h à 100°C sous pression de monoxyde de carbone (3 bars). Après concentration à l'évaporateur sous vide à 40°C, l'huile obtenue est diluée dans l'acétate d'éthyle et lavée trois fois à l'eau. Le produit est purifié par chromatographie flash sur colonne de silice.
   Huile jaune. Masse: 6 g. Rendement: 79%.
(c) *2-[1-(5,5,8,8-tétraméthyl-5,6,7,8-tétrahydro-2-naphthyl)vinyl]phenyl carbaldéhyde.*
   Une solution 1M d'hydrure de diisobutylaluminium dans le toluène (6,5 ml, 6,5 mmol) est additionnée à 0°C, goutte à goutte à une solution de 2-[1-(5,5,8,8-tétraméthyl-5,6,7,8-tétrahydro-2-naphthyl)vinyl] phenyl carboxylate de méthyle (1 g, 2,8 mmol) dans le toluène (30ml). La solution est agitée 1h à 0°C, puis traitée par une solution de tartrate double de sodium et potassium filtrée et reprise dans un mélange d'éther éthylique et d'eau. La phase organique est lavée à l'eau, séchée sur sulfate de magnésium et concentrée à l'évaporateur rotatif sous vide à 40°C.
   L'huile obtenue est agitée à TA 4h en présence de dichromate de pyridinium (2 g, 5,3 mmol) dans le CH₂Cl₂ (50 ml), puis la solution est filtrée sur silice et concentrée à l'évaporateur rotatif sous vide à 40°C. Le produit est purifié par chromatographie flash sur colonne de silice.
   Huile incolore. Masse: 580 mg, Rendement: 64%.
   RMN ¹H (CDCl3, 250 Mhz):
   1H/CDCl3: 1.20 (s, 6H); 1.27 (s, 6H); 1.67 (s, 4H); 5.21 (s, 1H); 5.95 (s, 1H); 7.00 à 7.60 (m, 6H); 8.00 (db, 1H)
(d) *3-{2-[1-(5,5,8,8-tétraméthyl-5,6,7,8-tétrahydro-2-naphthyl)vinyl]phenyl} acrylate d'éthyle.*
   De l'hydrure de sodium à 80% dans l'huile (91 mg, 3 mmol), est additionné à un mélange de 2-[1-(5,5,8,8-tétraméthyl-5,6,7,8-tétrahydro-2-naphthyl)vinyl] phenyl carbaldéhyde (800 mg, 2,5 mmol) et de triéthylphosphonoacétate (600 ul, 3 mmol) dans le THF (20ml). Le mélange est agité 1h à température ambiante, extrait à l'acétate d'éthyle et lavé à l'eau. Après séchage la phase organique est concentrée à l'évaporateur rotatif sous vide à 40°C, le produit est purifié par chromatographie flash sur colonne de silice.
   Masse: 800 mg, Rendement: 82%.
   RMN 1H (CDCl₃, 250 Mhz):
   1H/CDCl3: 1.20 à 1.28 (m, 15H); 1.66 (s, 4H); 4.16 (q, 1H); 5.12 (s, 1H); 5.86 (s, 1H); 6.32 (d, 1H); 6.99 (db, 1H); 7.19 à 7.37 (5H); 7.66 (m, 1H); 7.77 (d, 1H).
   13C/CDCl3: 14.3, CH3/ 31.8, 4^{*}CH3/ 34.1, Cq/ 34.2, Cq/ 35.0, CH2/ 35.1, CH2/ 60.2, CH2/ 116.2, CH2/ 118.9, CH/ 124.2, CH/ 125.2, CH/ 126.4, CH/ 126.5, CH/ 127.7, CH/ 129.5, CH/ 130.6, CH/ 133.4, Cq/ 137.7, CH/ 143.0, Cq/ 143.6, Cq/ 144.7, Cq/ 147.4, Cq/ 166.8, Cq

### EXEMPLE 10

### Acide 3{2-[1-(5,5,8,8-tétraméthyl-5,6,7,8-tétrahydro-2-naphthyl)vinyl] phenyl} acrylique.

Une solution du produit de l'exemple 9 (800 mg, 2,1 mmol) et d'hydroxyde de sodium (800 mg) dans le THF (30ml) est chauffée 15h à reflux, acidifiée à pH 1 (HCI concentré), extraite à l'acétate d'éthyle et lavée à l'eau. Après séchage la phase organique est concentré à l'évaporateur rotatif sous vide à 40°C, le produit est lavé à l'heptane.
Masse: 475 mg, Rendement: 64%. Fp: 135°C.
1H/CDCl3: 1.20 (s, 6H); 1.25 (s, 6H); 1.65 (s, 3H); 5.11 (d, 1H); 5.86 (d, 1H); 6.30 (d, 1H); 6.96 (dd, 1H); 7.19 à 7.39 (m, 5H); 7.67 (m, 1H); 7.86 (d, 1H).
13C/CDCl3: 31.7, 4^{*}CH3/ 34.1, Cq/ 34.2, Cq/ 35.1, 2^{*}CH2/ 117.5, 3^{*}CH/ 124.3, CH/ 125.2, CH/ 126.5, CH/ 126.6, CH/ 127.8, CH/ 130.0, CH/ 130.6, CH/ 132.4, Cq/ 137.6, Cq/ 143.2, Cq/ 144.8, Cq/ 146.0, CH/ 149.5, Cq/ 172.2, Cq

### EXEMPLE 11

### 3{3-[1-(5,5,8,8-tétraméthyl-5,6,7,8-tétrahydro-2-naphthyl)vinyl]phenyl} acrylate d'éthyle.

(a) 3-(5,5,8,8-tétraméthyl-5,6,7,8-tétrahydro-2-naphthylcarbonyl) benzoate de méthyle
   Une solution de 3-iodophényl-(5,5,8,8-tétraméthyl-5,6,7,8-tétrahydro-2-naphthyl)méthanone (10,5 g, 25,1 mmol), de diacétate de palladium (564 mg, 2,5 mmol), de tributylamine (12 ml, 50,5 mmol) dans le méthanol (500 ml) est chauffée 3h à 100°C sous pression de monoxyde de carbone (3 bars). Après concentration à l'évaporateur sous vide à 40°C, l'huile obtenue est diluée dans l'acétate d'éthyle et lavée trois fois à l'eau. Le produit est purifié par chromatographie flash sur colonne de silice.
   Solide blanc. Masse: 6,6 g. Rendement: 75%.Fp: 135°C.
(b) 3-(1-(5,5,8,8-tétraméthyl-5,6,7,8-tétrahydro-2-naphthyl)vinyl] phenyl carboxylate de méthyle
   Du tert-butylate de potassium (3 g, 26,5 mmol) est additionné à une solution de 3-(5,5,8,8-tétraméthyl-5,6,7,8-tétrahydro-2-naphthylcarbonyl) benzoate de méthyle (6,6 g, 18,9 mmol) et de bromure de méthyltriphénylphoshonium (9,4 g, 26,3 mmol) dans le THF (60 ml). Le mélange est agité 1h à température ambiante. La solution est extraite par de l'acétate d'éthyle. Après décantation la phase organique est lavée deux fois par 40 ml d'eau, séchée sur sulfate de magnésium anhydre et concentré à l'évaporateur rotatif sous vide à 40°C. Le produit est purifié par chromatographie flash sur colonne de silice.
   Solide blanc. Rendement: 87%. Fp: 64°C.
(c) *3-[1-(5,5,8,8-tétraméthyl-5,6,7,8-tétrahydro-2-naphthyl)vinyl] phenyl carbaldéhyde.*
   Une solution 1M d'hydrure de diisobutylaluminium dans le toluène (7,3 ml, 7,3 mmol) est additionnée à 0°C, goutte à goutte à une solution de 3-[1-(5,5,8,8-tétraméthyl-5,6,7,8-tétrahydro-2-naphthyl)vinyl] phenyl carboxylate de méthyle (1,1 g, 3,16 mmol) dans le toluène (30ml). La solution est agitée 1h à 0°C, puis traitée par une solution de tartrate double de sodium et potassium filtrée et reprise dans un mélange d'éther éthylique et d'eau. La phase organique est lavée à l'eau, séchée sur sulfate de magnésium et concentrée à l'évaporateur rotatif sous vide à 40°C.
   L'huile obtenue est agitée à TA 4h en présence de dichromate de pyridinium (2,2 g, 5,8 mmol) dans le CH₂Cl₂ (50 ml), puis la solution est filtrée sur silice et concentrée à l'évaporateur rotatif sous vide à 40°C. Le produit est purifié par chromatographie flash sur colonne de silice.
   Huile jaune. Masse: 800 mg, Rendement: 80%.
   RMN 1H/CDCl3: 1.24 (s, 6H); 1.30 (s, 6H); 1.70 (s, 4H); 5.50 (d, 1H); 7.05 (dd, 1H); 7.08 à 7.29 (m, 2H); 7.50 (t, 1H); 7.64 (dt, 1H); 7.83 à 7.89 (m, 2H),
(d) *3-{3-[1-(5,5,8,8-tétraméthyl-5,6,7,8-tétrahydro-2-naphthyl)vinyl]phenyl} acrylate d'éthyle.*
   De l'hydrure de sodium à 80% dans l'huile (183 mg, 2,3 mmol), est additionné à un mélange de 3-[1-(5,5,8,8-tétraméthyl-5,6,7,8-tétrahydro-2-naphthyl)vinyl] phenyl carbaldéhyde (580 mg, 1,8 mmol) et de triéthylphosphonoacétate (435 ul, 2,2 mmol) dans le THF (20ml). Le mélange est agité 2h à température ambiante, extrait à l'acétate d'éthyle et lavé à l'eau. Après séchage la phase organique est concentrée à l'évaporateur rotatif sous vide à 40°C, le produit est purifié par chromatographie flash sur colonne de silice.
   Masse: 690 mg, Rendement: 98%.
   RMN (250 MHz):
   1H/CDCl3: 1.22 à 1.36 (m, 15H); 1.70 (s, 4H); 4.26 (q, 2H); 5.42 (dd, 2H); 6.42 (d, 1H); 7.06 (dd, 1H); 7.24 à 7.52 (m, 6H); 7.68 (d, 1H).
   13C/CDCl3: 14.3, CH3/ 31.8, 4^{*}CH3/ 34.2, Cq/ 34.3, Cq/ 35.07, CH2/ 35.15, CH2/ 114.0, CH2/ 118.4, CH/ 125.4, CH/ 126.3, CH/ 126.4, CH/ 127.2, CH/ 128.0, CH/ 128.6, CH/ 130.3, CH/ 134.4, Cq/ 137.8, Cq/ 142.5, Cq/ 144.6, CH/ 144.8, Cq/ 149.5, Cq/ 167.0, Cq.

### EXEMPLE 12

### Acide 3{3-[1-(5,5,8,8-tétraméthyl-5,6,7,8-tétrahydro-2-naphthyl)vinyl]phenyl} acrylique.

Une solution du produit de l'exemple 11 (690 mg, 1,8 mmol) et d'hydroxyde de sodium (285 mg) dans le THF (20ml) est chauffée 8h à reflux, acidifiée à pH 1 (HCI concentré), extraite à l'acétate d'éthyle et lavée à l'eau. Après séchage la phase organique est concentrée à l'évaporateur rotatif sous vide à 40°C, le produit est lavé à l'heptane.
Masse: 500 mg, Rendement: 78%. Fp: 140°C.
1H/CDCl3: 1.25 (s, 6H); 1.30 (s, 6H); 1.70 (s, 3H); 5.46 (d, 2H); 6.43 (d, 1H); 7.07 (dd, 1H); 7:28 à 7.54 (m, 6H); 7,77 (d, 1H).
13C/CDCl3: 31.8, 4^{*}CH3/ 34.2, Cq/ 34.3, Cq/ 35.0, CH2/ 35.1, CH2/ 114.1, CH/ 117.4, CH/ 125.3, CH/ 126.2, CH/ 126.4, CH/ 127.4 CH/ 128.3, CH/ 128.7, CH/ 130.8, CH/ 133.9, CH/ 137.7, CH/ 142.5, Cq/ 144.7, Ca/ 144.8, Cq/ 147.1, Cq/ 149.4, Cq/ 172.2, Cq.

### EXEMPLE 13

### 3{3-[1-(5,5,8,8-tétraméthyl-5,6,7,8-tétrahydro-2-naphtyl}éthyl]phenyl} acrylate d'éthyle.

(a) 3-[1-(5,5,8,8-tétraméthyl-5,6,7,8-tétrahydro-2-naphtyl)éthyl]benzoate de méthyle.
   Une solution de 3-[1-(5,5,8,8-tétraméthyl-5,5,7,8-tétrahyaro-2-naphthyl) vinyl] phenyl carboxylate de méthyle (1,2 g, 3,45 mmol) dans l'acétate d'éthyle, en présence de palladium sur charbon (0,3g) sous une pression de 6 bars d'hydrogène est agitée à température ambiante 4 h. Le mélange est filtré sur célite puis concentré à l'évaporateur rotatif sous vide à 40°C.
   Huile incolore. Masse: 1,2 g, Rendement: 100%.
(b) 3-[1-(5,5,8,8-tétraméthyl-5,6,7,8-tétrahydro-2-naphtyl)éthyl]phényle carbaldéhyde.
   Le 3-[1-(5,5,8,8-tétraméthyl-5,6,7,8-tétrahydro-2-naphtyl)éthyl]benzoate de méthyle est soumis à une réduction par l'hydrure de diisobutylaluminium pour donner l'alcool correspondánt qui est oxydé en aldéhyde par action de dichromate de pyridinium selon le procédé décrit dans l'exemple 5 (c).
   Huile jaune. Masse 1,1g. Rendement: 100%.
   1H/CDCl3: 1.25 (sb, 12H); 1.66 (sb, 7H); 4.17 (q, 1H); 6.93 (d, 1H); 7.14 à 7.48 (m, 4H), 7.69 (db, 1H); 7.78 (s, 1H).
(c) 3-{3-[1-(5,5,8,8,-tétraméthyl-5,6,7,8-tétrahydro-2-naphtyl)éthyl]phényle} acrylate d'éthyle.
   Le 3-{3-[1-(5,5,8,8-tétraméthyl-5,6,7,8-tétrahydro-2-naphtyl)éthyl]phényle}acrylate d'éthyle est formé par action de triéthylphosphonoacétate sur le 3-[1-(5,5,8,8-Tétraméthyl-5,6,7,8-tétrahydro-2naphtyl)ethyl]phényle carbaldéhyde selon le procédé décrit dans l'exemple 5(d).
   Huile incolore. Masse: 1,25 g, Rendement: 93%.
   1H/CDCl3: 1.25 à 1.36 (m, 15H); 1.61 à 1.66 (m, 7H); 4.09 (q, 1H); 4.26 (q, 2H); 6.40 (d, 1H); 6.92 (dd, 1H); 7.14 à 7.38 (m, 7H); 7.65 (d, 1H).
   13C/CDCl3: 14.4; CH3/ 31.96; 4^{*}CH3/ 34.0; Cq/ 34.3; Cq/ 35.3; CH2/ 44.5; CH3/ 60.5; CH2/ 118.1; CH/ 124.8; CH/ 125.6; 2^{*}CH/ 126.6; CH/ 127.6; CH/ 128.9; CH/ 129.8; CH/ 135; Cq/ 140; Cq/ 142; Cq/ 144; Cq/ 145.0; CH/ 147; Cq/ 166; Cq.

### EXEMPLE 14

### Acide 3{3-[1-(5,5,8,8-tétraméthyl-5,6,7,8-tétrahydro-2-naphtyl)éthyl] phenyl} acrylique.

Une solution du produit de l'exemple 13 (1,25 g, 3,2 mmol) et d'hydroxyde de sodium (1,3 g) dans le THF (30ml) est chauffée 3 h à reflux acidifiée à pH 1 (HCI concentré), extraite à l'acétate d'éthyle et lavée à l'eau. Après séchage la phase organique est concentrée à l'évaporateur rotatif sous vide à 40°C, le produit est lavé à l'heptane.
Solide blanc. Masse: 750 mg, Rendement: 65%. Fp: 145°C.
1H/CDCl3: 1.25 (sb, 12H); 1.61 à 1,66 (m, 7H); 4.11 (q, 1H); 6.42 (d, 1H); 6.93 (dd, 1H); 7.15 à 7.41 (m, 6H); 7.76 (d, 1H).
13C/CDCl3: 21.9, CH3/ 31.9, 4^{*}CH3/ 34.0, Cq/ 34.3, Cq/ 35.2, 2^{*}CH2/ 44.5, CH/ 116.8, CH/ 124.7, CH/ 125.5, CH/ 125.9, CH/ 126.5, CH/ 127.9, CH/ 128.9, CH/ 130.3, CH/ 134.0. Cq/ 142.3, Cq/ 142.7, Cq / 144.8, Cq/ 147.4, CH/ 147.6, Cq/ 171.3, Cq.

### EXEMPLE 15

### 3{2-[1-(5,5,8,8-tétraméthyl-5,6,7,8-tétrahydro-2-naphtyl)éthyl]phenyl} acrylate d'éthyle.

(a) 2-[1-(5,5,8,8-tétraméthyl-5,6,7,8-tétrahydro-2-naphtyl)éthyl]benzoate de méthyle.
   Une solution de 2-[1-(5,5,8,8-tétraméthyl-5,6,7,8-tétrahydro-2-naphthyl)vinyl] phenyl carboxylate de méthyle (1,1 g, 3,48 mmol) dans l'acétate d'éthyle, en présence de palladium sur charbon (275 mg) sous une pression de 6 bars d'hydrogène est agitée à température ambiante 4 h. Le mélange est filtré sur célite puis concentré à l'évaporateur rotatif sous vide à 40°C.
   Huile incolore. Masse: 1,1 g, Rendement: 100%.
(b) 2-[1-(5,5,8,8-tétraméthyl-5,6,7,8-tétrahydro-2-naphtyl)éthyl]phényle carbaldéhyde.
   Le 2-[1-(5,5,8,8-tétraméthyl-5,6,7,8-tétrahydro-2-naphtyl)éthyl]benzoate de méthyle est soumis à une réduction par l'hydrure de diisobutylaluminium pour donner l'alcool correspondant qui est oxydé en aldéhyde par action de dichromate de pyridinium selon le procédé décrit dans l'exemple 5 (c).
   Huile incolore. Masse 1,1g. Rendement: 100%.
   1H/CDCl₃: 1.20 à 1.30 (m, 12H); 1.58 à 1.74 (m, 7H); 5.13 (q, 1H); 6.91 (dd, 1H); 7.13 à 7.56 (6H); 7.82 (dd, 1H); 10.35 (s, 1H).
(c) 3-{2-[1-[5,5,8,8-tétraméthyl-5,6,7,8-tétrahydro-2-naphtyl)éthyl]phényle} acrylate d'éthyle.
   Le 3-{2-[1-(5,5,8,8-tétraméthyl-5,6,7,8-tétrahydro-2-naphtyl)éthyl]phényle}acrylate d'éthyle est formé par action de triéthylphosphonoacétate sur le 3-[1-(5,5,8,8-tétraméthyl-5,6,7,8-tétrahydro-2-naphtyl)éthyl]phényle carbaldéhyde selon le procédé décrit dans l'exemple-5 (e).
   Huile incolore Masse: 450 mg, Rendement: 33%.
   1H/CDCl3: 1.21à 1.35 (m, 15H); 1.56 à 1.65 (m, 7H); 4.25 (q, 2H); 4.48 (q, 1H); 6.26 (d, 1H); 6.90 (dd, 1H); 7.14 à 7.37 (m, 5H) ; 7.49 (d, 1H); 8.14 (d, 1H)
   13C/CDCl3: 14.3; CH3/ 22.0; CH3/ 31.8; 4^{*}CH3/ 33.9; Cq/ 34.2; Cq/ 35.1; CH2/ 35.2; CH2/ 40.2; CH/ 60.4; CH2/ 120.2; CH/ 124.8, CH/ 125.7; CH/ 126.3; CH/ 126.5; CH/ 126.8; CH/ 127.4; CH/ 130.0; CH/ 133.3; Cq/ 142.0; Cq/ 142.4; Cq/ 142.8; CH/ 144.7; Cq/ 145.8; Cq/ 166.9; Cq.

### EXEMPLE 16

### Acide 3{2-[1-(5,5,8,8-tétraméthyl-5,6,7,8-tétrahydro-2-naphtyl) éthyl]phenyl} acrylique.

Une solution du produit de l'exemple 15 (450 mg, 1,1 mmol) et d'hydroxyde de sodium (450 mg) dans le THF (20ml) est chauffée 15 h à reflux, acidifiée à pH 1 (HCI concentré), extraite à l'acétate d'éthyle et lavée à l'eau. Après séchage la phase organique est concentrée à l'évaporateur rotatif sous vide à 40°C, le produit est lavé à l'heptane.
Solide blanc. Masse: 325 mg, Rendement: 79%. Fp: 150°C.
1H/CDCl3: 1.23 à 1.26 (m, 12H); 1.61 à 1,65 (m, 7H); 4.47 (q, 1H); 6.26 (d, 1H); 6.88 (dd, 1H); 7.16 à 7.41 (m, 5H); 7.53 (d, 1H); 8.25 (d, 1H) 13C/CDCl3: 22.0, CH3/ 31,8, 4^{*}CH3/ 33.9, Cq/ 34.2, Cq/ 35.2, 2^{*}CH2/ 40.5, CH/ 100,5, CH/ 118.8, CH/ 124.7, CH/ 125.7, CH/ 126.4, CH/ 126.5, CH/ 127.0, CH/ 127.5, CH/ 130.4, CH/ 132.9, Cq/ 142.0, Cq/ 142.5, Cq/ 145.2, 2^{*}Cq/ 146.0, CH/ 171.8, Cq.

### EXEMPLE 17

### 3{2-[1-(3,5,5,8,8-pentaméthyl-5,6,7,8-tétrahydro-2-naphtyl)éthyl]phenyl} acrylate d'éthyle.

(a) 2-[1-(3,5,5,8,8-pentaméthyl-5,5,7,8-tétrahydro-2-naphtyl)éthyl] benzoate de méthyle.
   Une solution de 2-[1-(3,5,5,8,8-pentaméthyl-5,6,7,8-tétrahydro-2-naphthyl)vinyl] phenyl carboxylate de méthyle (3,5 g, 9,7 mmol) dans l'acétate d'éthyle, en présence de palladium sur charbon (900 mg) sous une pression de 6 bars d'hydrogène est agitée à température ambiante 4 h. Le mélange est filtré sur célite puis concentré à l'évaporateur rotatif sous vide à 40°C.
   Solide blanc. Masse: 3,44 g, Rendement: 98%. Fp: 48°C.
   1H/CDCl3: 1.23 à 1.28 (m, 12H); 1.58 (d, 3H); 1.68 (s, 3H); 1.99 (s, 3H); 3.85 (s, 3H): 5.13 (q, 1H); 6.99 (s, 1H); 7.02 (dd, 1H); 7.19 (dt, 1H); 7.25 (s, 1H); 7.33 (dt, 1H); 7.75 (dd, 1H).
   13C/CDCl3: 19.2, CH3/ 22.0, CH/ 31.8, 4^{*}CH3/ 33.7, 2^{*}Cq/ 35.3, 2^{*}CH2/ 37.1, CH/ 51.9, CH3/ 124,7, CH/ 125.5, CH/ 128.2, CH/ 128.3, CH/ 129.8, CH/ 131.8, CH/ 133.6, Cq/ 140.5, Cq/ 141.9, Cq/ 142.3, Cq/ 148.0, Cq/ 168.6, Cq.
(b) 2-[1-(3,5,5,8,8-pentaméthyl-5,6,7,8-tétrahydro-2-naphtyl)éthyl]phényle carbaldéhyde.
   Le 2-[1-(3,5,5,8,8-pentaméthyl-5,6,7,8-tétrahydro-2-naphtyl)éthyl]benzoate de méthyle est soumis à une réduction par l'hydrure de diisobutylaluminium pour donner l'alcool correspondant qui est oxydé en aldéhyde par action de dichromate de pyridinium selon le procédé décrit dans l'exemple 5 (C).
   Solide blanc. Masse 1,6 g. Rendement: 53%. Fp: 135 °C.
   1H/CDCl3: 1.23 à 1.28 (m, 12H); 1.59 (d, 3H); 1.67 (s, 4H); 2.04 (S, 3H); 5.27 (q, 1H); 7.01 (s, 1H); 7.11 (d, 1H); 7.19 (s, 1H); 7.30 (dt, 1H); 7.46 (dt, 1H); 7.81 (dd, 1H); 10.33 (s,1H).
   13C/CDCl3: 19.7, CH3/ 22.6, CH3/ 32.4, 4^{*}CH3/ 34.3, Cq/ 34.5, Cq/ 35.7, 2^{*}CH2/ 36.2, CH/ 125.3, CH/ 126.7, CH/ 128.8, CH/ 128.86, CH/ 132.4, CH/ 133.45, Cq/ 133.52, Cq/ 134.4, CH/ 140.5, Cq/ 142.7, Cq/ 143.1, Cq/ 149.9, Cq/ 193.0, CH.
(c) 3-{2-[1-(3,5,5,8,8-pentaméthyl-5,6,7,8-tétrahydro-2-naphtyl)éthyl] phényle}acrylate d'éthyle.
   Le 3-{2-[1-(3,5,5,8,8-pentaméthyl-5,6,7,8-tétrahydro-2-naphtyl)éthyl]} acrylate d'éthyle est formé par action de triéthylphosphonoacétate sur le 3-[1-3,5,5,8,8,-pentaméthyl-5,6,7,8-tétrahydro-2-naphtyl)éthyl]phényle carbaldéhyde selon le procédé décrit dans l'exemple 5 (e).
   Huile incolore. Masse: 1,9 g, Rendement: 100%.
   1H/CDCl3: 1.23 à 1.33 (m, 15H); 1.51 (d, 3H); 1.66 (s, 4H); 2.09 (s, 3H); 4.25 (q, 2H); 4.52 (q, 1H), 6.3 (d, 1H); 7.00 à 7.013 (m, 2H); 7.18 à 7.30 (m, 3H), 7.48 (dd, 1H); 8.09 (d, 1H).
   13C/CDCl3: 13.9, CH3/ 18.8, CH3/ 21.3, CH3/ 31.4, 4^{*}CH3/ 33.3, Cq/ 33.6, Cq/ 34.8, 2^{*}CH2/ 36.7, CH/ 60.0, CH2/ 119.7, CH/ 124.4, CH/ 125.8, CH/ 126.2, CH/ 126.9, CH/ 127.8, CH/ 129.6, CH/ 132.4, Cq/ 132.6, Cq/ 139.6 Cq/ 141.7, Cq/ 142.0, Cq/ 145.5, 2^{*}Cq/ 166.5, Cq.

### EXEMPLE 18

### Acide 3{2-[1-(3,5,5,8,8-pentaméthyl-5,6,7,8-tétrahydro-2-naphtyl)éthyl] phenyl} acrylique.

Une solution du produit de l'exemple 17 (1,9 g, 4,9 mmol) et d'hydroxyde de sodium (2 g) dans le THF (30ml) est chauffée 4 h à reflux, acidifiée à pH 1 (HCI concentré), extraite à l'acétate d'éthyle et lavée à l'eau. Après séchage la phase organique est concentrée à l'évaporateur rotatif sous vide à 40°C, le produit est lavé à l'heptane.
Solide blanc. Masse: 1,1 g, Rendement: 59%. Fp: 192°C.
1H/CDCl: 1.22 à 1.28 (m, 15H); 1.56 (d, 3H); 1.67 (s, 4H); 2.07 (s, 3H); 4.51 (q, 1H); 6.32 (d, 1H); 7.01 (s, 1H); 7.05 (m, 1H); 7.2 à 7.32 (m, 3H); 7.53 (d, 1H); 8.23 (d, 1H).
13C/CDCl3: 19.4; CH3/ 22.0; CH3/ 32.0; 4^{*}CH3/ 34.0; Cq/ 34,2; Cq/ 35.4; 2^{*}CH2/ 37.4; CH/ 119.3; CH/ 125.0; CH/ 126.5; CH/ 127.0; CH/ 127.7; CH/ 128.5; CH/ 130.7; CH/ 132.8; Cq/ 132.8; Cq/ 140.0; Cq/ 142.4; Cq/ 142.7; Cq/ 145.0; Cq/ 146.4; CH/ 172.2; Cq.

### EXEMPLE 19

### 3{3-[1-(3,5,5,8,8-pentaméthyl-5,6,7,8-tétrahydro-2-naphtyl)éthyl]phenyl} acrylate d'éthyle.

(a) 3-[1-(3,5,5,8,8-pentaméthyl-5,6,7,8-tétrahydro-2-naphfyl)éthyl] benzoate de méthyle.
   Une solution de 3-[1-(3,5,5,8,8-pentaméthyl-5,6,7,8-tétrahydro-2-naphthyl)vinyl] phenyl carboxylate de méthyle (2,5 g, 6,9 mmol) dans l'acétate d'éthyle, en présence de palladium sur charbon (700 mg) sous une pression de 6 bars d'hydroaène est agitée à température ambiante 4 h. Le mélange est filtré sur célite puis concentré à l'évaporateur rotatif sous vide à 40°C.
   Solide blanc. Masse: 2,5 g, Rendement: 99%. Fp: 85°C.
   1H/CDCl3: 1.22 à 1.29 (m, 15H); 1.62 (d, 3H); 1.62 (s, 4H); 2.14 (s, 3H); 3.89 (s, 3H); 4.27 (q, 1H); 7.02 (s, 1H); 7.23 à 7.43 (m, 3H); 7.83 (dt, 1H); 7.92 (s, 1H).
   13C/CDCl3: 19.4, CH3/ 22.4, CH/ 31.9, 4^{*}CH3/ 33.8, 2^{*}Cq/ 35.2, 2*CH2/ 41.0, CH/ 52.0, CH3/ 124.5, CH/ 127.1, CH/ 128.3, CH/ 129.0, -CH/ 130.0, Cq/ 132.3, CH/ 132.9, Cq/ 139.9, Cq/ 142.3, Cq/ 142.6, Cq/ 147.0, Cq/ 167.3, Cq.
(b) 3-[1-(3,5,5,8,8-pentaméthyl-5,6,7,8-tétrahydro-2-naphtyl)éthyl]phényle carbaldéhyde.
   Le 3-[1-(3,5,5,8,8-pentaméthyl-5,6,7,8-tétrahydro-2-naphtyl)éthyl]benzoate de méthyle est soumis à une réduction par l'hydrure de diisobutylaluminium pour donner l'alcool correspondant qui est oxydé en aldéhyde par action de dichromate de pyridinium selon le procédé décrit dans l'exemple 5 (c).
   Huile incolore. Masse 2,1 g. Rendement: 98%.
   1H/ CDCl3: 1.23 à 1.29 (m, 15H); 1.60 (d, 3H); 1.67 (s, 4H); 2.14 (s, 3H); 4.64 (q, 1H); 7.03 (s, 1H); 7.21 (s, 1H); 7.39 à 7.45 (m, 2H); 7.66 à 7.71 (m, 2H); 9.97 (s, 1H).
   13C/CDCl3: 19.6, CH3/ 22.4, CH3/ 32.0, 4^{*}CH3/ 33.9, Cq/ 34.2, Cq/ 35.3, 2^{*}CH2/ 41,1, CH/ 124.7, CH/ 127.6, CH/ 128.6, CH/ 129.0, CH/ 129.1, CH/ 133.0, Cq/ 134.1, CH/ 136.6, Cq/ 139.8, Cq/ 142.6, Cq/ 142.9, Cq/ 148.0, Cq/ 192.8, CH.
(c) 3-{3-[1-(3,5,5,8,8-pentaméthyl-5,6,7,8-tétrahydro-2-naphtyl)éthyl]phényle} acrylate d'éthyle.
   Le 3-{3-[1-(3,5,5,8,8-pentaméthyl-5,6,7,8-tétrahydro-2-naphtyl)éthyl]phényle} acrylate d'éthyle est formé par action de triéthylphosphonoacétate sur le 3-[1-(5,5,8,8-pentaméthyl-5,6,7,8-tétrahydro-2-naphtyl)éthyl]phényle carbaldéhyde selon le procédé décrit dans l'exemple 5 (e).
   Huile incolore. Masse: 2,5 g, Rendement: 96%.
   1H/CDCl3: 1.24 à 1.35 (m, 15H); 1.60 (d, 3H); 1.67 (s, 4H); 2.13 (s, 3H); 4.25 (q, 2H); 6.37 (d, 1H); 7.02 (s, 1H); 7.14 à 7.35 (m, 5H); 7.63 (d, 1H)
   13C/CDCl3: 14.0, CH3/ 19.1, CH3/ 22.0, CH3/ 31.4, 4^{*}CH3/ 33.5, Cq/ 33.7, Cq/ 34.9 CH2/ 40.7, CH/ 60.1, CH2/ 117.6, CH/ 124.1, CH/ 125.0, CH/ 127.4, CH/ 128.0, CH/ 128.5, CH/ 129.5 CH/ 132.7, Cq/ 134.0, Cq/ 139.6, Cq/ 142.0, Cq/ 142.3, Cq/ 144.6, Cq/ 147.1, Cq/ 166.8, Cq.

### EXEMPLE 20

### Acide 3{3-[1-(3,5,5,8,8-pentaméthyl-5,6,7,8-tétrahydro-2-naphtyl) éthyl] phenyl} acrylique.

Une solution du produit de l'exemple 19 (2,5 g, 6,4 mmol) et d'hydroxyde de sodium (2,5 g) dans le THF (50ml) est chauffée 4 h à reflux, acidifiée à pH 1 (HCI concentré), extraite à l'acétate d'éthyle et lavée à l'eau. Après séchage, la phase organique est concentrée à l'évaporateur rotatif sous vide à 40°C, le produit est lavé à 'heptane.
Solide blanc. Masse: 435 mg, Rendement: 18%. Fp: 187°C.
1H/CDCl3: 1.26 à 1.29 (m, 12H); 1.61 (d, 3H); 1.67 (s, 4H); 2.14 (s, 3H); 4.24 (.q, 1H); 6.39 (d, 1H); 7.03 (s, 1H); 7.17 à 7.38 (m, 5H); 7.74 (d, 1H)
13C/CDCl3: 19.9; CH3/ 22.8; CH3/ 32.2; CH3/ 32.3; 2^{*}CH3/ 32.55; CH3/ 34.25; Cq/ 34.5; Cq/ 35.7; 2^{*}CH2/ 41.45; CH/ 117.3; CH/ 124.5; CH/ 126,1; CH/ 128.4; CH / 128.8; CH/ 129.3; CH/ 130.7; CH/ 133.4; Cq/ 134.4; Cq/ 140.3; Cq/ 142.8; Cq/ 143.1; Cq/ 147.9; Cq/ 148.0; CH/ 172.7; Cq.

### EXEMPLE 21

### 3{2-[1-(5,5,8,8-tétraméthyl-5,6,7,8-tétrahydro-2-naphtyl)cyclopropyl]phenyl} acrylate d'éthyle.

(a) 2-[1-(5,5,8,8-tétraméthyl-5,6,7,8-tétrahydro-2-naphtyl)cyclopropyl] benzoate de méthyle.
   Du diiodométhane (230 ul, 2,85 mmol) est additionné goutte à goutte à 60°C à un mélange de 2-[1-(5,5,8,8-tétraméthyl-5,6,7,8-tétrahydro-2-naphthyl)vinyl] phenyl carboxylate de méthyle (500 mg, 1,44 mmol) et d'une solution de diéthylzinc 1M dans l'heptane (2,9 ml, 2,9 mmol) dans le dichlorométhane (10 ml). Le chauffage est poursuivi 4h. La solution est extraite par de l'acétate d'éthyle. Après décantation la phase organique est lavée par une solution d'HCI 1N, puis par de l'eau, séchée sur sulfate de magnésium anhydre et concentrée. Le même procédé est répété une fois.
   Huile incolore. Masse: 500 mg, Rendement: 100%.
(b) 2-[1-(5,5,8,8-tétraméthyl-5,6,7,8-tétrahydro-2-naphtyl)cyclopropyl]phényle carbaldéhyde.
   Le 2-[1-(5,5,8,8-tétraméthyl-5,6,7,8-tétrahydro-2-naphtyl)cyclopropyl] benzoate de méthyle est soumis à une réduction par l'hydrure de diisobutylaluminium pour donner l'alcool correspondant qui est oxydé en aldéhyde par action de Bichromate de pyridinium selon le procédé décrit dans l'exemple 5 (c).
   Huile incolore. Masse 990 mg. Rendement: 61 %
   1H/CDCl3: 1.13 (s, 6H); 1.21 (s, 6H); 1.42 (t, 2H); 1.48 (t, 2H); 1.62 (s, 4H); 6.69 (dd, 1H); 6.83 (d, 1H); 7.13 (d, 1H); 7.26 (s, 1H); 7.41 (tb, 1H); 7.44 à 7.63 (m, 2H); 7.93 (d, 1H).
   13C/CDCl3: 17.8, 2^{*}CH2/ 26.2, Cq/ 31.8, 4^{*}CH3/ 33.8, Cq/ 34.2, Cq/ 35.0, 2^{*}CH2/ 122.8, CH/ 123.5, CH/ 126.5, CH/ 127.4, CH/ 127.6, CH/ 131.6, CH/ 134.1, CH/ 135.3, Cq/ 142.3, Cq/ 142.4, Cq/ 144.7, Cq/ 147.6, Cq/ 192.7, Cq.
(c) 3-{2-[1-(5,5,8,8-tétraméthyl-5,6,7,8-tétrahydro-2-naphtyl)cyclopropyl] phényle}acrylate d'éthyle.
   Le 3-{2-[1-(5,5,8,8-tétraméthyl-5,6,7,8-tétrahydro-2-nàphtyl)cyclopropyl]phényle} acrylate d'éthyle est formé par action de triéthylphosphonoacétate sur 2-[1-(5,5,8,8-tétraméthyl-5,6,7,8-tétrahydro-2-naphtyl)cyclopropyl]phényle carbaldéhyde selon le procédé décrit dans l'exemple 5(e).
   Huile incolore. Masse: 800 mg, Rendement: 66%.
   1H/CDCl3: 1.15 (s, 6H); 1.21 (s, 6H); 1.27 à 1.32 (m, 7H); 1.61 (s, 4H); 4.22 (q, 1H); 6.28 (d, 1H); 6.81 (dd, 1H); 6.98 (d, 1H); 7.11 (d, 1H); 7.26 à 7.39 (m, 2H); 7.55 (t, 2H); 8.30 (d, 1H).
   13C/CDCl3: 13.7, CH3/ 16.3, 2^{*}CH2/ 27.5, Cq/ 31.3, 4^{*}CH3/ 33.4, Cq/ 33.7, Cq/ 34.7, 2^{*}CH2/ 59.8, CH2/ 118,4, CH/ 123.5, CH/ 124.0, CH/ 125.9, 2^{*}CH/ 125,7, CH/ 129.7, CH/ 131.2 CH/ 134.6, Cq/ 141.4, Cq/ 141.5, Cq/ 142.8, CH/ 143.9, Cq/ 144.2, Cq/ 166.2, Cq.

### EXEMPLE 22

### Acide 3{2-[1-(5,5,8,8-tétraméthyl-5,6,7,8-tétrahydro-2-naphtol) cyclopropyl]phenyl} acrylique.

Une solution du produit de l'exemple 21 (800 mg, 2 mmol) et d'hydroxyde de sodium (8.00 mg) dans le THF (20ml) est chauffée 15 h à reflux, acidifiée à pH 1 (HCI concentré), extraite à l'acétate d'éthyle et lavé à l'eau. Après séchage, la phase organique est concentrée à l'évaporateur rotatif sous vide à 40°C, le produit est lavé à l'heptane.
Solide blanc. Masse: 400 mg, Rendement: 54%. Fp: 200°C.
1H/CDCl3: 1.17 à 1.28 (m, 16H); 1.62 (s, 4H); 6.28 (d, 1H); 6.83 (dd, 1H); 7.01 (d, 1H); 7.12 (d, 1H); 7.28 (t, 1H); 7.39 (t, 1H); 7.58 (m, 2H); 8.45 (d, 1H).
13C/CDCl3: 16.4, 2^{*}CH2/ 27.8, Cq/ 31.6, 4^{*}CH3/ 33.7, Cq/ 34,1, Cq/ 35.0, 2^{*}CH2/ 117.6, CH/ 123.9, CH/ 124.3, CH/ 126.3, CH/ 126.5, CH / 127.0, CH/ 130.3, CH/ 131.6, CH/ 134.6, Cq/ 141.5, Cq / 141.9, Cq/ 144.4, Cq/ 144.9, Cq/ 145.6, CH/ 172.0, Cq.

### EXEMPLE 23

### 3{3-[1-(5,5,8,8-tétraméthyl-5,6,7,8-tétrahydro-2-naphtyl)cyclopropyl] phenyl} acrylate d'éthyle.

(a) 3-[1-(5,5,8,8-tétraméthyl-5,6,7,8-tétrahydro-2-naphtyl)cyclopropyl] benzoate de méthyle.
   Du diiodométhane (1,1 ml, 13,7 mmol) est additionné goutte à goutte à 60°C à un mélange de 3-[1-(5,5,8,8-tétraméthyl-5,6,7,8-tétrahydro-2-naphthyl)vinyl] phenyl carboxylate de méthyle (2,2 g, 6,3 mmol) et d'une solution de diéthylzinc 1M dans l'heptane (13 ml, 13 mmol) dans le dichlorométhane (50 ml). Le chauffage est poursuivi 15 h. La solution est extraite par de l'acétate d'éthyle. Après décantation la phase organique est lavée par une solution d'HCI 1N, puis par de l'eau, séchée sur sulfate de magnésium anhydre et concentrée.
   Solide blanc. Masse: 2,3 g, Rendement: 100%.
(b) 3-[1-(5,5,8,8-tétraméthyl-5,6,7,8-tétrahydro-2-naphtyl)cyclopropyl]phényle carbaldéhyde.
   Le 3-[1-(5,5,8,8-tétraméthyl-5,5,7,8-tétrahydro-2-naphtyl)cyclopropyl] benzoate de méthyle est soumis à une réduction par l'hydrure de diisobutylaluminium pour donner l'alcool correspondant qui est oxydé en aldéhyde par action de dichromate de pyridinium selon le procédé décrit dans l'exemple 5 (c).
   Huile jaune. Masse 1,1 g. Rendement: 52%.
   1H/CDCl3: 1.15 à 1.30 (m, 16H); 1.58 (s, 4H); 6.89 (dd, 1H); 7.06 à 7.46 (m, 4H); 7.61 (dt, 1H); 7.68 (s, 1H).
   13C/CDCl3: 16.5, 2^{*}CH2/ 29.5, Cq/ 31.8, 4^{*}CH3/ 34.0, Cq/ 34.3, Cq/ 35.08, CH2/ 35.14, CH2/ 125.7, CH/ 126.3, CH/ 126.5, CH/ 127.4, CH/ 128.9, CH/ 129.3, CH/ 134.9, CH/ 136.5, Cq/ 141.5, Cq/ 142.9, Cq/ 144.8, Cq/ 147.4, Cq/ 192.5, Cq.
(c) 3-{3-[1-(5,5,8,8-tétraméthyl-5,6,7,8-tétrahydro-2-naphtyl)cyclopropyl] phényle}acrylate d'éthyle.
   Le 3-{3-[1-(5,5,8, 8-tétraméthyl-5,6,7,8-tétrahydro-2-naphtyl)cyclopropyl]phényle} acrylate d'éthyle est formé par action de triéthylphosphonoacétate sur 3-[1-(5,5,8,8-tétraméthyl-5,6,7,8-tétrahydro-2-naphtyl)cyclopropyl]phényle carbaldéhyde selon le procédé décrit dans l'exemple 5(e).
   Solide blanc. Masse: 900 mg. Rendement: 100%. Fp: 78°C.
   1H/CDCl3: 1.15 à 1.36 (m, 19H); 1.59 (s, 4H); 4.17 (q, 2H); 6.31 (1H); 6.87 (dd, 1H); 7.04 à 7.32 (m, 6H); 7.55 (d, 1H).
   13C/CDCl3: 14.2, CH3/ 16.3, 2^{*}CH2/ 29.4, 2^{*}CH3/ 31.7, 2^{*}CH3/ 33.8, Cq/ 34.2, Cq/ 34.99, CH2/ 35.05, CH2/ 60.3, CH2/ 118.0, CH/ 125.36, CH/ 125.44, CH/ 126.0, CH/ 126.3, CH/ 128.2, CH/ 128.6, CH/ 130.5, CH/ 134.3, Cq/ 141.8, Cq/ 142.5, Cq/ 144.6, Cq/ 144.7, CH/ 146.7, Cq/ 166.9, Cq.

### EXEMPLE 24

### Acide 3{3-[1-(5,5,8,8-tétraméthyl-5,6,7,8-tétrahydro-2-naphtyl) cyclopropyl]phenyl} acrylique.

Une solution du produit de l'exemple 23 (900 mg, 2,22 mmol) et d'hydroxyde de sodium (900 mg) dans le THF (20ml) est chauffée 4 h à reflux, acidifiée à pH 1 (HCI concentré), extraite à l'acétate d'éthyle et lavée à l'eau. Après séchage, la phase organique est concentrée à l'évaporateur rotatif sous vide à 40°C, le produit est lavé à l'heptane.
Solide blanc. Masse: 450 mg, Rendement: 54%. Fp: 165°C.
1H/CDCl3: 1.27 à 1.35 (m, 16H); 1.66 (s, 4H); 6.41 (d, 1H); 6.95 (dd, 1H); 7.1 (d, 1H), 7.18 (d, 1H), 7.2 à 7.41 (m, 4H); 7.75 (d, 1H).
13C/CDCl3: 16.4 2^{*}CH2/ 29.5, Cq/ 31.8, 4^{*}CH3/ 33.9, Cq/ 34.3, Cq/ 35.1, 2^{*}CH2/ 117.0, CH/ 125.6, CH/ 125.8, CH/ 126.0, CH/ 126.4, CH / 128.6, CH/ 128.8, CH/ 131.0, CH/ 133.9, Cq/ 141.8, Cq/ 142.7, Cq/ 144.7, Cq/ 147.0, Cq/ 147.3, CH/ 171.9, Cq.

### EXEMPLE 25

### 3-{3-[Hydroxyimino-(3,5,5,8,8-pentaméthyl-5,6,7,8-tetrahydro-naphthalen-2-yl)-methyl]-phenyl}-acrylate d'éthyle.

a) (3-lodo-phenyl)-(3,5,5,8,8-pentamethyl-5,6,7,8-tetrahydro-naphthalen-2-yl)-methanone oxime
   Un mélange de 3-iodophenyl-(3,5,5,8,8-pentaméthyl-5,6,7,8-tétrahydro-2-naphthyl)méthanone (6g, 13,9 mmol), de chlorhydrate d'hydroxylamine (4,8g, 69,5 mmol) de tamis moléculaire 4 A dans la pyridine (17 ml) est chauffé 8 h. à reflux. Le milieu réactionnel est extrait à l'acétate d'éthyle et lavé à l'eau. Après séchage, la phase organique est concentrée à l'évaporateur rotatif sous vide à 40°C. Les deux isomères (cis et trans) sont séparés par chromatographie flash sur colonne de silice.
   1er isomère:
   Solide blanc. Masse: 2,98 g, Rendement: 48%. Fp: 58°C.
   1H/CDCl3: 1.23 (s, 6H); 1.32 (s, 6H); 1.69 (s, 4H); 2.12 (s, 3H); 6.99 (s, 1H); 7.01 (d, 1H); 7.20 (s, 1H); 7.33 (d, 1H); 7.66 (d, 1H); 7.91 (t, 1H); 7.98 (s, 1H)
   13C/CDCl3: 19.5; CH3/ 32.0; 4^{*}CH3/ 34.0; Cq/ 34.3; Cq/ 35.1; CH2/ 35.2; CH2/ 94.5; Cq/ 126.3; CH/ 126.6; CH/; 1 28.2; CH/ 129.3; Cq/ 130.1; CH/ 132.9; Cq/ 135.8; CH/ 138.1; Cq/ 138.4; CH/ 142.5; Cq/ 145.8; Cq/ 157.4; Cq.
   2ème isomère
   Solide blanc. Masse: 720 mg, Rendement: 12%. Fp: 145°C.
   1H/CDCl3: 1.23 (s, 6H); 1.32 (s, 6H); 1.69 (s, 4H); 2.12 (s, 3H); 7.07 à 7.08 (m, 1H); 7.13 (d, 1H); 7.18 (s, 1H); 7.42 (d, 1H); 7.70 (d, 1H); 7.9 (s, 1H)
   13C/CDCl3: 20.4; CH3/ 32.0; 4^{*}CH3/ 34.1; Cq/ 34.3; Cq/ 35.2; 2^{*}CH2/ 93.9; Cq/ 128.4; CH/ 129.1; CH/ 129.2; CH/ 129.8; CH/ 133.1; Cq/ 133.8; Cq/ 135.4; Cq/ 138.2; CH/ 138.6; CH/ 142.6; Cq/ 146.2; Cq/ 156.9; Cq.
b) 3-{3-[Hydroxyimino-(3,5,5,8,8-pentamethyl-5,6,7,8-tetrahydro-naphthalen-2-yl)-methyl]-phenyl}-acrylate d'éthyle.
   Une solution de (3-lodo-phenyl)-(3,5,5,8,8-pentamethyl-5,6,7,8-tetrahydro-naphthalen-2-yl)-methanone oxime (320 mg, 0,7 mmol), de diacétate de palladium (20 mg, 89 umol), de tributylamine (392 ul, 1,7 mmol) et d'acrylate d'éthyle (78 ul, 0,7 mmol) dans l'acétonitrile (10 ml) est chauffée 4h à 80°C. Le milieu réactionnel est extrait à l'éther éthylique et lavé à l'eau. Après séchage, la phase organique est concentré à l'évaporateur rotatif sous vide à 40°C. Le produit est purifié par chromatographie flash sur colonne de silice.
   Huile incolore. Masse: 200 mg. Rendement: 66%.
   1H/CDCl3: 1.15 (s 6H); 1.20 à 1.33 (m, 9H); 1.50 (s, 4H); 2.06 (s, 3H); 4.16 (q, 2H); 6.30 (d, 1H); 6.95 (s, 1H); 7.14 (s, 1H); 7.17 à 7.59 (m, 5H); 8.84 (sb, 1H)
   13C/CDCl3: 14.0; CH3/ 19.2; CH3/ 31.6; 4^{*}CH3/ 33.7; Cq/ 33.9; Cq/ 34.8; CH2/ 34.9; CH2/ 60.3; CH2/ 118.5; CH/ 125.9; CH/ 126.8; CH/ 128.1; CH/ 128.6; CH/ 128.7; CH/ 129.2; Cq/ 132.5; Cq/ 134.4; Cq/ 136.5; Cq/ 142.2; Cq/ 144.0; CH/ 145.4; Cq/ 157.6; Cq/ 166.7; Cq

### EXEMPLE 26

### 3-{3-[Hydroxyimino-(5,5,8,8-tetramethyl-5,6,7,8-tetrahydro-naphthalen-2-yl)-methyl]-phenyl}-acrylate d'éthyle.

Le même mode opératoire est utilisé pour l'autre isomère de la (3-lodo-phenyl)-(3,5,5,8,8-pentamethyl-5,6,7,8-tetrahydro-naphthalen-2-yl)-methanone oxime Solide jaune. Masse: 430 mg. Rendement: 46%. Fp: 65°C.
1H/CDCI3: 1.25 à 1.33(m, 15H); 1.68 (s, 4H); 2.05 (s, 3H); 4.25 (q, 2H); 6.41 (d, 1H); 7.07 (s, 1H); 7.20 (s, 1H); 7.46 à 7.72 (m, 5H)

### EXEMPLE 27

### Acide 3-{3-[Hydroxyimino-(3,5,5,8,8-pentamethyl-5,6,7,8-tetrahydro-naphthalen-2-yl)-methyl]-phenyl}-acrylique.

Une solution du produit de l'exemple 25 (820 mg, 1,76 mmol) et d'hydroxyde de sodium (1 g) dans le THF (30ml) est chauffée 4 h à reflux, acidifiée à pH 1 (HCI concentré), extraite à l'acétate d'éthyle et lavée à l'eau. Après séchage, la phase organique est concentrée à l'évaporateur rotatif sous vide à 40°C, le produit est lavé à l'heptane.
Solide blanc. Masse: 643 mg, Rendement: 97%. Fp: 185°C.
1H/CDCl3: 1.23 (s, 6H); 1.32 (s, 6H); 1.70 (s, 4H); 2.14 (s, 3H); 6.50 (d, 1H); 7.03 (s, 1H); 7.22 (s, 1H); 7.26 à 7.90 (m, 6H).

### EXEMPLE 28

### Acide 3-{3-[Hydroxyimino-(5,5,8,8-tétramethyl-5,6,7,8-tetrahydro-naphthalen-2-yl)-methyl]-phenyl}-acrylique.

Une solution du produit de l'exemple 26 (430 mg, 0,92 mmol) et d'hydroxyde de sodium (500 mg) dans le THF (20ml) est chauffée 4 h à reflux, acidifiée à DH 1 (HCI concentré), extraite à l'acétate d'éthyle et lavée à l'eau. Après séchage, la phase organique est concentrée à l'évaporateur rotatif sous vide à 40°C, le produit est lavé à l'heptane.
Solide blanc. Masse: 340 mg, Rendement: 98%. Fp: 132°C.
1H/CDCl3: 1.18 (s, 6H); 1.22 (s, 6H); 1.62 (s, 4H); 1.96 (s, 3H); 6.32 (d, 1H); 6.99 (s, 1H); 7.14 (s, 1H); 7.30 à 7.65 (m, 5H).

### EXEMPLE 29

### 3-{3-[2-(3,5,5,8,8-pentamethyl-5,6,7,8-tetrahydro-naphthalen-2-yl)-[1,3] dithian-2-yl]-phenyl}-acrylate d'éthyle.

a) 2-(3-lodo-phenyl)-2-(3,5,5,8,8-pentamethyl-5,6,7,8-tetrahydro-naphthalen-2-yl)-[1,3]dithiane.
   Un mélange de 3-iodophenyl-(3,5,5,8,8-pentamethyl-5,6,7,8-tétrahydro-2-naphthyl)méthanone (2g, 4,6 mmol), de trifluoréthérate diéthyl éthérate (3,6 mmol) et de propane dithiol (0,5 ml, 5,1 mmol) dans le dichlorométhane (50 ml) est agité à température ambiante 24 h. Le milieu réactionnel est extrait à l'éther éthylique et lavé à l'eau. Après séchage, la phase organique est concentrée à l'évaporateur rotatif sous vide à 40°C. Le produit est purifié par chromatographie flash sur colonne de silice.
   Solide blanc. Masse: 1,54 g, Rendement: 65%.
   1H/CDCl3: 1.26 (s, 12H); 1.68 (s, 4H); 1.90 à 2.04 (m, 2H); 2.08 (s, 3H); 2.75 à 2.97 (m, 4H); 6.99 (s, 1H); 7.03 (d, 1H); 7.42 (d, 1H); 7.58 (d, 1H); 7.73 (s, 1H), 8.09 (t, 1H)
   13C/CDCl3: 22.5; CH3/ 24.2; CH2/ 29.5; 2^{*}CH2/ 31.8; 4^{*}CH3/ 33.8; 2^{*}Cq/ 35.1; 2^{*}CH2/ 94.2; Cq/ 128.0; CH/ 129.0; CH/ 129.9; CH/ 131.2; CH/ 134.0; Cq/ 136.4; CH/ 137.4; CH/ 141.3; Cq/ 144.1; Cq/ 146.1; Cq
b) 3-{3-[2-(3,5,5,8,8-pentamethyl-5,6,7,8-tetrahydro-naphthalen-2-yl)-[1,3] dithian-2-yl]-phenyl}-acrylate d'éthyle.
   Le 3-{3-[2-(3,5,5,8,8-pentamethyl-5,6,7,8-tetrahydro-naphthalen-2-yl)-[1,3]dithian-2-yl]-phenyl}acrylate d'éthyle est formé à partir de 2-(3-iodo-phenyl)-2-(3,5,5,8,8-pentamethyl-5,6,7,8-tetrahydro-naphtalen-2-yl)-[1,3]dithiane selon le procédé décrit dans l'exemple 25b.
   Solide brun. Masse: 770 mg, Rendement: 68%.
   1H/CDCl3: 1.23 à 1.32 (m, 15H); 1.63 (s, 4H); 1.96 à 2.02 (m, 2H); 2.04 (s, 3H); 2.76 à 2.98 (m, 4H); 2.24 (q, 2H); 6.42 (d, 1H); 6.99 (s, 1H); 7.27 (d, 1H); 7.32 (d, 1H); 7.43 (d, 1H); 7.53 (d, 1H); 7.65 (d, 1H); 7.81 (s, 1H); 7.84 (sb, 1H)
   13C/CDCl3: 14.3; CH3/ 22.4; CH3/ 24.3; CH2/ 29.6; 2^{*}CH2/ 31.7; 4^{*}CH3/ 33.7; Cq/ 33.0; Cq/ 35.1; 2^{*}CH2/ 60.4; CH2/ 61.6; Cq/ 118.3; CH/ 126.6; CH/ 128.6; CH/ 128.8; CH/ 129.1; CH/ 130.5; CH/ 131.2; CH/ 134.0; Cq/ 134.4; Cq/ 136.5; Cq/ 141.2; Cq/ 144.0: Cq/ 144.6; CH+Cq/ 167,0; Cq

### EXEMPLE 30

### 3-{3-[2-(3,5,5,8,8-pentamethyl-5,6,7,8-tetrahydro-naphthalen-2-yl)-[1,3] dithian-2-yl]-phenyl}-acrylic acid

De manière analogue à l'exemple 1d) à partir de 960 mg (1,9 mmoles) de 3-{3-[2-(3,5,5,8,8-pentamethyl-5,6,7,8-tetrahydro-naphthalen-2-yl)-[1,3] dithian-2-yl]-phenyl}-acrylate d'éthyle, on obtient 770 mg (68%) d'acide 3-{3-[2-(5,5,8,8-pentamethyl-5,6,7,8-tetrahydro-naphthalen-2-yl)-[1,3]dithian-2-yl]-phenyl} -acrylique.
Solide jaune. Masse: 750 mg, Rendement: 83%.
1H/CDCl3:1.26 (s, 6H); 1.33 (s, 6H); 1.66 (s, 2H); 1.89 (m, 2H); 2.31 (s, 3H); 2.80 (4H); 7.05 à 7.16 (m, 2H); 7.49 (tb, 2H); 7.94 (d, 1H); 8.09 (s, 1H); 8.28 (s, 1H).

### EXEMPLE 31

### Exemples de formulations

### 1) VOIE ORALE

(a) On prépare la composition suivante sous la forme d'un comprimé de 0,8 g

| | |
|---|---|
| Composé de l'exemple 3 | 0,005 g |
| Amidon prégélatinisé | 0,265 g |
| Cellulose microcristalline | 0,300 g |
| Lactose | 0,200 g |
| Stéarate de magnésium | 0,030 g |

(b) On prépare une suspension buvable, destinée à être conditionnée en ampoules de 5 ml

| | |
|---|---|
| Composé de l'exemple 1 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

(c) On prépare la formulation suivante destinée à être conditionnée en gélules:

| | |
|---|---|
| Composé de l'exemple 2 | 0,025 g |
| Amidon de maïs | 0,060 g |
| Lactose q.s.p | 0,300 g |

Les gélules utilisées sont constituées de gélatine, d'oxyde de titane et d'un conservateur.

### 2) VOIE TOPIQUE

(a) On prépare la crème Eau-dans l'Huile non ionique suivante :

| | |
|---|---|
| Composé de l'exemple 4 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p | 100,000 g |

(b) On prépare un gel en réalisant la formulation suivante :

| | |
|---|---|
| Composé de l'exemple 6 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p | 100,000 g |

(c) On prépare une lotion antiséborrhéique en procédant au mélange des ingrédients suivants :

| | |
|---|---|
| Composé de l'exemple 5 | 0,030 g |
| Propylène glycol | 5,000 g |
| Butylhydroxytoluène | 0,100 g |
| Ethanol (à 95°) q.s.p | 100,000 g |

(d) On prépare une composition cosmétique contre les effets néfastes du soleil en procédant au mélange des ingrédients suivants :

| | |
|---|---|
| Composé de l'exemple 8 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

(e) On prépare la crème Huile dans l'Eau non ionique suivante :

| | |
|---|---|
| Composé de l'exemple 7 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

(f) On prépare un gel topique en procédant au mélange des ingrédients suivants :

| | |
|---|---|
| Composé de l'exemple 11 | 0,050 g |
| Ethanol | 43,000 g |
| a -tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

(g) On prépare une lotion capillaire anti-chute et pour la repousse des cheveux en procédant au mélange des ingrédients suivants :

| | |
|---|---|
| Composé de l'exemple 10 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

(h) On prépare une crème anti-acnéique en procédant au mélange des ingrédients suivants :

| | |
|---|---|
| Composé de l'exemple 13 | 0,050 g |
| Acide rétinoïque | 0,010 g |
| Mélange de stéarates de glycérol et de polyéthylène glycol (75 moles) vendu sous le nom de "Gelot 64" par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

(i) On prépare une crème huile dans l'eau en réalisant la formulation suivante:

| | |
|---|---|
| Composé de l'exemple 9 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000 g |

(j) On prépare la crème de type huile dans l'eau suivante :

| | |
|---|---|
| Acide lactique | 5,000 g |
| Composé de l'exemple 10 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Eau q.s.p | 100,000 g |

## Revendications

1. Composés biaromatiques, caractérisés par le fait qu'ils répondent à la formule générale (I) suivante : 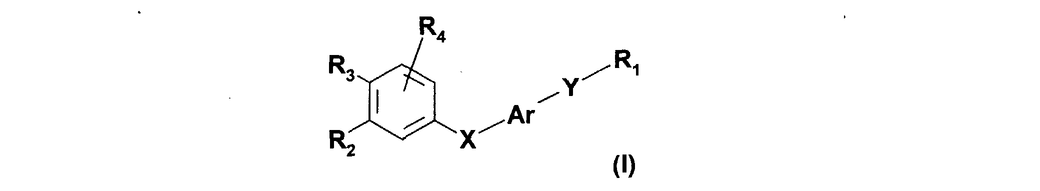 dans laquelle:
- R₁ représente
(i) le radical -CH₃,
(ii) le radical -CH₂-O-R₅,
(iii) le radical -O-R₅,
(iv) le radical -CO-R₆,
R₅ et R₆ ayant les significations données ci-après,
- Y représente un radical choisi parmi les radicaux de formules (a) et (b) suivantes: 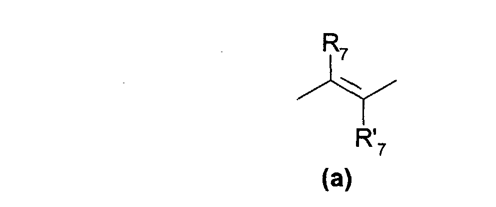 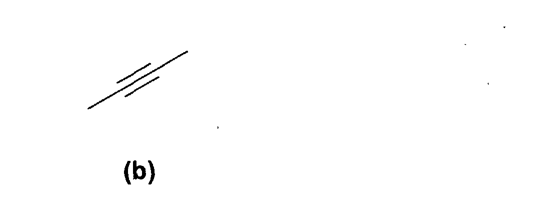 R₇ et R'₇ ayant les significations données ci-après,
- Ar représente un radical choisi parmi les radicaux de formules (c) à (f) suivantes : 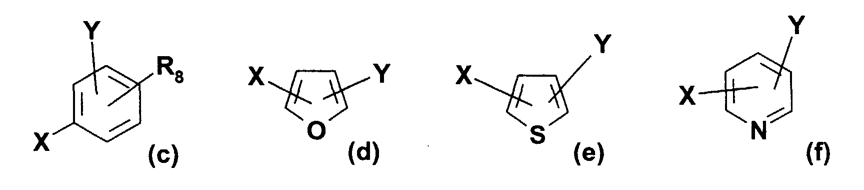 dans lesquelles le radical Y est en position ortho ou méta par rapport au radical X, X et Y de ces formules correspondants à X et Y représentés dans la formule (I),
R₈ ayant la signification donnée ci-après,
- X représente un atome d'oxygène, de soufre, un radical -SO-, -SO₂-, -N(R₉)- ou un radical choisi parmi les radicaux de formules (g) à (r) suivantes: 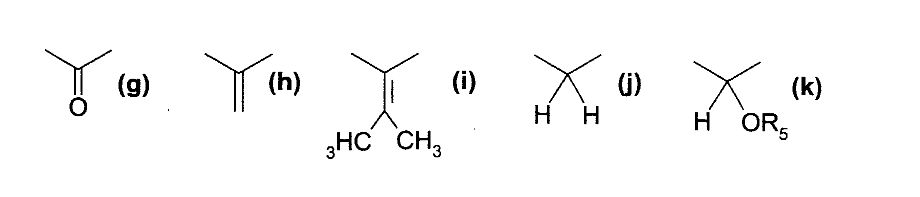 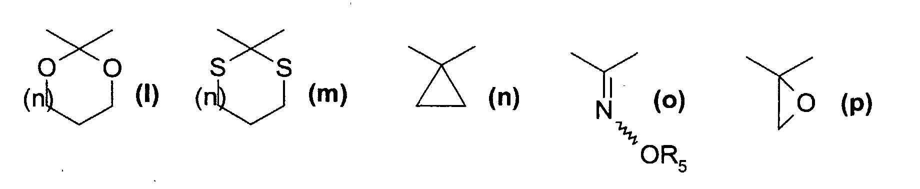 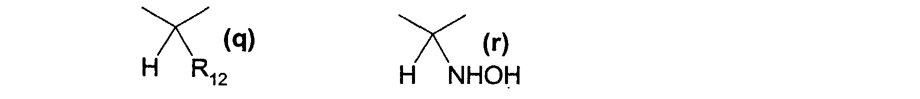 R₅, R₉ et n ayant les significations données ci-après,
- R₂ et R₃, identiques ou différents, sont choisis dans le groupe constitué par :
(i) un atome d'hydrogène,
(ii) un radical alkyle présentant au moins 3 atomes de carbone, parmi lesquels le carbone attaché au radical phényl de la formule (I) est substitué par au moins deux atomes de carbone,
(iii) un radical alkyle linéaire ou ramifié,
(iv) un radical -OR₅,
(v) un radical -SR₅,
(vi) un radical polyéther,
R₅ ayant la signification donnée ci-après,
étant entendu que R₂ et R₃ pris ensemble peuvent former avec le cycle aromatique adjacent un cycle à 5 ou 6 chaînons éventuellement substitué par des groupes méthyle et/ou éventuellement interrompu par un atome d'oxygène ou de soufre,
étant entendu que, lorsque R₂ et R₃ ne forment pas un cycle, au moins un des radicaux R₂ et R₃ a une signification (ii) mentionnée ci-dessus,
- R4 et R₈, identiques ou différents, représentent un atome d'hydrogène, un atome d'halogène, un radical alkyle linéaire ou ramifié, ou un radical -OR₅, un radical polyéther,
- R₅ représente un atome d'hydrogène, un radical alkyle ayant de 1 à 6 atomes de carbone ou un radical -COR₁₀,
R₁₀ ayant la signification donnée ci-après,
- R₆ représente :
(a) un atome d'hydrogène
(b) un radical alkyle ayant de 1 à 6 atomes de carbone
(c) un radical de formule: 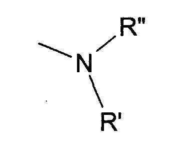 R' et R" ayant les significations données ci-après,
(d) un radical -OR₁₁
R₁₁ ayant la signification donnée ci-après,
- R₇, R'₇ et R₉, identiques ou différents, représentent un atome d'hydrogène ou un radical alkyle ayant de 1 à 6 atomes de carbone,
- n est un nombre entier égal à 0 ou 1,
- R₁₀ représente un radical alkyle ayant de 1 à 6 atomes de carbone,
- R₁₁ représente un atome d'hydrogène, un radical alkyle linéaire ou ramifié, un radical alkényl, un radical mono ou polyhydroxyalkyle, un radical aryle ou aralkyle, éventuellement substitué (s) ou un reste de sucre ou un reste d'amino acide ou de peptide,
- R₁₂ représente un radical alkyle ayant de 1 à 6 atomes de carbone,
- R' et R", identiques ou différents, représentent un atome d'hydrogène, un radical alkyl ayant de 1 à 6 atomes de carbone, un radical mono ou polyhydroxyalkyle, un radical aryle éventuellement substitué ou un reste d'amino acide, de peptide ou de sucre ou encore pris ensemble forment un hétérocycle,
et les isomères optiques et géométriques desdits composés de formule (I) ainsi que leurs sels .

2. Composés selon la revendication 1, caractérisés par le fait qu'ils se présentent sous forme de sels d'un métal alcalin ou alcalino-terreux, de zinc, d'une amine organique, d'un acide minéral ou organique.

3. Composés selon l'une des revendications 1 ou 2, caractérisés par le fait que les radicaux alkyles linéaires ou ramifiés sont choisis parmi le groupe constitué par les radicaux méthyle, éthyle, isopropyle, butyle, tertiobutyle, hexyle, nonyle et dodécyle.

4. Composés selon l'une quelconque des revendications précédentes, caractérisés en ce que les radicaux alkyle présentant au moins 3 atomes de carbone, parmi lesquels le carbone attaché au radical phényl de la formule (I) est substitué par au moins deux atomes de carbone sont choisis parmi les radicaux isopropyle, tertiobutyle, 1,1-diméthylhexyle et 1,1-diméthyldécyle.

5. Composés selon l'une des revendications précédentes, caractérisés en ce que les radicaux polyhydroxyalkyles sont choisis parmi le groupe constitué par les radicaux 2,3-dihydroxypropyle, 2,3,4-trihydroxybutyle, 2,3,4,5-tétrahydroxypentyle ou le reste du pentaérythritol.

6. Composés selon l'une des revendications précédentes, caractérisés en ce que le radical aryle est un radical phényle éventuellement substitué par au moins un atome d'halogène, un radical hydroxyle, un radical alkyle, une fonction nitro, un groupe méthoxy ou une fonction amine éventuellement substituée.

7. Composés selon l'une des revendications précédentes, caractérisés en ce que les radicaux aralkyles sont choisis parmi le groupe constitué par les radicaux benzyle ou phénéthyle, éventuellement substitués par au moins un atome d'halogène, un hydroxyle, une fonction nitro ou un groupe méthoxy.

8. Composés selon l'une des revendications précédentes, caractérisés en ce que les radicaux alkényles sont choisis dans le groupe constitué par les radicaux contenant de 2 à 5 atomes de carbone et présentant une ou plusieurs insaturations éthyléniques, et en particulier le radical allyle.

9. Composés selon l'une des revendications précédentes, caractérisés en ce que les restes de sucre sont choisis dans le groupe constitué par les restes de glucose, de galactose, de mannose et d'acide glucuronique.

10. Composés selon l'une quelconque des revendications précédentes, caractérisés en ce que les restes d'aminoacide sont choisis dans le groupe constitué par les restes dérivant de la lysine, de la glycine ou de l'acide aspartique.

11. Composés selon l'une quelconque des revendications précédentes, caractérisés en ce que les restes de peptides sont choisis dans le groupe constitué par les restes de dipeptides ou de tripeptides.

12. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou polyhydroxyalkyle.

13. Composés selon l'une quelconque des revendications précédentes, caractérisés en ce que les radicaux polyéther sont choisis parmi les radicaux méthoxyméthyl éther, méthoxyéthoxyméthyl éther ou méthylthyométhyl éther.

14. Composés selon la revendication 1, caractérisés par le fait qu'ils sont pris, seuls ou en mélanges, dans le groupe constitué par :
Acide 3-[3-(5,5,8,8-tetramethyl-5,6,7,8-tetrahydro-2-naphthylcarbonyl)phenyl] acrylique.
Acide 3-[3-(3,5,5,8,8-pentamethyl-5,6,7,8-tetrahydro-2-naphthylcarbonyl) phenyl]acrylique.
Acide 3-[3-(3,5,5,8,8-pentamethyl-5,6,7,8-tetrahydro-2-naphthylthio)phenyl] acrylique.
Acide 3-[3-(5,5,8,8-tetramethyl-5,6,7,8-tetrahydro-2-naphthylthio)phenyl] acrylique.
3{2-[1-(3,5,5,8,8-pentaméthyl-5,6,7,8-tétrahydro-2-naphthyl)vinyl] phenyl} acrylate d'éthyle.
Acide 3{2-[1-(3,5,5,8,8-pentaméthyl-5,6,7,8-tétrahydro-2-naphthyl) vinyl]phenyl} acrylique.
3{3-[1-(3,5,5,8,8-pentaméthyl-5,6,7,8-tétrahydro-2-naphthyl)vinyl ]phenyl} acrylate d'éthyle.
Acide 3{3-[1-(3,5,5,8,8-pentaméthyl-5,6,7,8-tétrahydro-2-naphthyl)vinyl] phenyl} acrylique.
3{2-[1-(5,5,8,8-tétraméthyl-5,6,7,8-tétrahydro-2-naphthyl)vinyl]phenyl} acrylate d'éthyle.
Acide 3{2-[1-(5,5,8,8-tétraméthyl-5,6,7,8-tétrahydro-2-naphthyl)vinyl] phenyl} acrylique.
3{3-[1-(5,5,8,8-tétraméthyl-5,6,7,8-tétrahydro-2-naphthyl)vinyl]phenyl} acrylate d'éthyle.
Acide 3{3-[1-(5,5,8,8-tétraméthyl-5,6,7,8-tétrahydro-2-naphthyl) vinyl]phenyl} acrylique.
3{3-[1-(5,5,8,8-tétraméthyl-5,6,7,8-tétrahydro-2-naphtyl)éthyl]phenyl} acrylate d'éthyle.
Acide 3{3-[1-(5,5,8,8-tétraméthyl-5,6,7,8-tétrahydro-2-naphtyl) éthyl] phenyl} acrylique.
3{2-[1-(5,5,8,8-tétraméthyl-5,6,7,8-tétrahydro-2-naphtyl)éthyl]phenyl} acrylate d'éthyle.
Acide 3{2-[1-(5,5,8,8-tétraméthyl-5,6,7,8-tétrahydro-2-naphtyl) éthyl] phenyl} acrylique.
3{2-[1-(3,5,5,8,8-pentaméthyl-5,6,7,8-tétrahydro-2-naphtyl)éthyl]phenyl} acrylate d'éthyle.
Acide 3{2-[1-(3,5,5,8,8-pentaméthyl-5,6,7,8-tétrahydro-2-naphtyl)éthyl] phenyl} acrylique.
3{3-[1-(3,5,5,8,8-pentaméthyl-5,6,7,8-tétrahydro-2-naphtyl)éthyl]phenyl} acrylate d'éthyle.
Acide 3{3-[1-(3,5,5,8,8-pentaméthyl-5,6,7,8-tétrahydro-2-naphtyl) éthyl] phenyl} acrylique.
3{2-[1-(5,5,8,8-tétraméthyl-5,6,7,8-tétrahydro-2-naphtyl)cyclopropyl] phenyl} acrylate d'éthyle.
Acide 3{2-[1-(5,5,8,8-tétraméthyl-5,6,7,8-tétrahydro-2-naphtyl) cyclopropyl]phenyl} acrylique.
3{3-[1-(5,5,8,8-tétraméthyl-5,6,7,8-tétrahydro-2-naphtyl)cyclopropyl] phenyl} acrylate d'éthyle.
Acide 3{3-[1-(5,5,8,8-tétraméthyl-5,6,7,8-tétrahydro-2-naphtyl) cyclopropyl]phenyl} acrylique.
3-{3-[Hydroxyimino-(3,5,5,8,8-pentaméthyl-5,6,7,8-tetrahydro-naphthalen-2-yl)-methyl]-phenyl}-acrylate d'éthyle.
Acide 3-{3-[Hydroxyimino-(3,5,5,8,8-pentaméthyl-5,6,7,8-tetrahydro-naphthalen-2-yl)-methyl]-phenyl}-acrylique.
3-{3-[Hydroxyimino-(5,5,8,8-tetramethyl-5,6,7,8-tetrahydro-naphthalen-2-yl)-methyl]-phenyl}-acrylate d'éthyle.
Acide 3-{3-[Hydroxyimino-(5,5,8,8-tetramethyl-5,6,7,8,-tetrahydro-naphthalen-2-yl)-methyl]-phenyl} acrylique.
3-(3-[2-(3,5,5,8,8-pentamethyl-5,6,7,8-tetrahydro-naphthalen-2-yl)-[1,3] dithian-2-yl]-phenyl}-acrylate d'éthyle.
Acide 3-{3-[2-(3,5,5,8,8-pentamethyl-5,6,7,8-tetrahydro-naphthalen-2-yl)-[1,3] dithian-2-yl]-phenyl}-acrylique
Acide 3-{3-[Hydroxylamine-(3,5,5,8,8-pentamethyl-5,6,7,8-tetrahydro-naphthalen-2-yl)-methyl]-phenyl}-acrylique.
3-{3-[Hydroxylamine-(5,5,8,8-tetramethyl-5,6,7,8-tetrahydro-naphthalen-2-yl)-methyl]-phenyl}-acrylate d'éthyle.
Acide 3-{2-[Hydroxylamine-(3,5,5,8,8-pentamethyl-5,6,7,8-tetrahydro-naphthalen-2-yl)-methyl]-phenyl}-acrylique.
3-{2-[Hydroxylamine-(5,5,8,8-tetramethyl-5,6,7,8-tetrahydro-naphthalen-2-yl)-methyl]-phenyl}-acrylate d'éthyle.
Acide {3-[1-(5,5,8,8-Tetramethyl-5,6,7,8-tetrahydro-naphthalen-2-yl)-cydopropyl]-phenyl}-propynoique.
Acide {3-[1-(3,5,5,8,8-Pentamethyl-5,6,7,8-tetrahydro-naphthalen-2-yl)-cyclopropyl]-phenyl}-propynoique
Acide {2-[1-(5,5,8,8-Tetramethyl-5,6,7,8-tetrahydro-naphthalen-2-yl)-cyclopropyl]-phenyl}-propynoique
Acide {2-[1-(3,5,5,8,8-Pentamethyl-5,6,7,8-tetrahydro-naphthalen-2-yl)-cyclopropyl]-phenyl}-propynoique
Acide 3{2-[1-(3,5,5,8,8-Pentaméthyl-5,6,7,8-tétrahydro-2-naphtyl) cyclopropyl]phenyl} acrylique.
Acide 3{3-[1-(3,5,5,8,8-Pentaméthyl-5,6,7,8-tétrahydro-2-naphtyl) cyclopropyl]phenyl} acrylique.
Acide 3-{4-Hydroxy-3-[1-(5,5,8,8-tetramethyl-5,6,7,8-tetrahydro-naphthalen-2-yl)-cyclopropyl]-phenyl}-acrylique
Acide 3-{3-Hydroxy-2-[1-(5,5,8,8-tetramethyl-5,6,7,8-tetrahydro-naphthalen-2-yl)-cyclopropyl]-phenyl}-acrylique
Acide 3-{3-[1-(5,5,8,8-tetramethyl-5,6,7,8-tetrahydro-naphthalen-2-yl)-cyclopropyl]-4-méthoxyphenyl}-acrylique
Acide 3-{2-[1-(5,5,8,8-tetramethyl-5,6,7,8-tetrahydro-naphthalen-2-yl)-cyclopropyl]-3-méthoxyphenyl}-acrylique
Acide 3-{4-Hydroxy-3-[1-(3,5,5,8,8-pentamethyl-5,6,7,8-tetrahydro-naphthalen-2-yl)-cyclopropyl]-phenyl}-acrylique
Acide 3-{3-Hydroxy-2-[1-(3,5,5,8,8-pentamethyl-5,6,7,8-tetrahydro-naphthalen-2-yl)-cyclopropyl]-phenyl}-acrylique
Acide 3-{3-[1-(3,5,5,8,8-pentamethyl-5,6,7,8-tetrahydro-naphthalen-2-yl)-cyclopropyl]-4-méthoxyphenyl}-acrylique
Acide 3-{2-[1-(3,5,5,8,8-pentamethyl-5,6,7,8-tetrahydro-naphthalen-2-yl)-cyclopropyl]-3-méthoxyphenyl}-acrylique
3-{3-[1-(5,5,8,8-Tetramethyl-5,6,7,8-tetrahydro-naphthalen-2-yl)-cyclopropyl]-phenyl}-acrylamide
3-{2-(1-(5,5,8,8-Tetramethyl-5,6,7,8-tetrahydro-naphthalen-2-yl)-cyclopropyl]-phenyl}-acrylamide
3-{3-[1-(3,5,5,8,8-pentamethyl-5,6,7,8-tetrahydro-naphthalen-2-yl)-cyclopropyl]-phenyl}-acrylamide
3-{2-[1-(5,5,8,8-pentamethyl-5,6,7,8-tetrahydro-naphthalen-2-yl)-cyclopropyl]-phenyl}-acrylamide
*N*-Ethyl-3-{3-[1-(5,5,8,8-tetramethyl-5,6,7,8-tetrahydro-naphthalen-2-yl)-cyclopropyl]-phenyl}-acrylamide
*N*-Ethyl-3-{2-[1-(5,5,8,8-tetramethyl-5,6,7,8-tetrahydro-naphthalen-2-yl)-cyclopropyl]-phenyl}-acrylamide
*N*-Ethyl-3-{3-[1-(3,5,5,8,8-pentamethyl-5,6,7,8-tetrahydro-naphthalen-2-yl)-cyclopropyl]-phenyl}-acrylamide
*N*-Ethyl-3-{2-[1-(3,5,5,8,8-pentamethyl-5,6,7,8-tetrahydro-naphthalen-2-yl)-cyclopropyl]-phenyl}-acrylamide
1-Morpholin-4-yl-3-{3-[1-(5,5,8,8-tetramethyl-5,6,7,8-tetrahydro-naphthalen-2-yl)-cyclopropyl]-phenyl}-propenone
1-Morpholin-4-yl-3-{2-[1-(5,5,8,8-tetramethyl-5,6,7,8-tetrahydro-naphthalen-2-yl)-cyclopropyl]-phenyl}-propenone
1-Morpholin-4-yl-3-{3-[1-(3,5,5,8,8-pentamethyl-5,6,7,8-tetrahydro-naphthalen-2-yl)-cyclopropyl]-phenyl}-propenone
1-Morpholin-4-yl-3-{2-[1-(3,5,5,8,8-pentamethyl-5,6,7,8-tetrahydro-naphthalen-2-yl)-cyclopropyl]-phenyl}-propenone
*N*-(4-Hydroxy-phenyl)-3-{3-[1-(5,5,8,8-tetramethyl-5,6,7,8-tetrahydro-naphthalen-2-yl)-cyclopropyl]-phenyl}-acrylamide
*N*-(4-Hydroxy-phenyl)-3-{2-[1-(5,5,8,8-tetramethyl-5,6,7,8-tetrahydro-naphthalen-2-yl)-cyclopropyl]-phenyl}-acrylamide
*N*-(4-Hydroxy-phenyl)-3-{3-[1-(3,5,5,8,8-pentamethyl-5,6,7,8-tetrahydro-naphthalen-2-yl)-cyclopropyl]-phenyl}-acrylamide
N-(4-Hydroxy-phenyl)-3-{2-[1-(3,5,5,8,8-pentamethyl-5,6,7,8-tetrahydro-naphthalen-2-yl)-cyclopropyl]-phenyl}-acrylamide
3-{3-[1-(5,5,8,8-Tetramethyl-5,6,7,8-tetrahydro-naphthalen-2-yl)-ethyl]-phenyl}-propenal
3-{2-[1-(5,5,8,8-Tetramethyl-5,6,7,8-tetrahydro-naphthalen-2-yl)-ethyl]-phenyl}-propenal
3-{3-[1-(3,5,5,8,8-Pentamethyl-5,6,7,8-tetrahydro-naphthalen-2-yl)-ethyl]-phenyl}-propenal
3-{2-[1-(3,5,5,8,8-Pentamethyl-5,6,7,8-tetrahydro-naphthalen-2-yl)-ethyl]-phenyl}-propenal
3-{3-[1-(5,5,8,8-Tetramethyl-5,6,7,8-tetrahydro-naphthalen-2-yl)-ethyl]-phenyl}-prop-2-en-1-ol
3-{2-[1-(5,5,8,8-Tetramethyl-5,6,7,8-tetrahydro-naphthalen-2-yl)-ethyl]-phenyl}-prop-2-en-1-ol
3-{3-[1-(3,5,5,8,8-Pentamethyl-5,6,7,8-tetrahydro-naphthalen-2-yl)-ethyl]-phenyl}-prop-2-en-1-ol
3-{2-[1-(3,5,5,8,8-Pentamethyl-5,6,7,8-tetrahydro-naphthalen-2-yl)-ethyl]-phenyl}-prop-2-en-1-ol

15. Composés selon la revendication 1, caractérisés par le fait qu'ils présentent l'une au moins des caractéristiques suivantes :
R₁ représente le radical -CO-R₆
Ar représente les radicaux de formule (c) ou (f)
X représente les radicaux de formule (g), (h), (n) ou (m)
R₂ et R₃ pris ensemble forment avec le cycle aromatique adjacent un cycle à 5 ou 6 chaînons éventuellement substitué par des groupes méthyle et/ou éventuellement interrompu par un atome d'oxygène ou de soufre,

16. Composés selon l'une quelconque des revendications précédentes pour une utilisation comme médicament.

17. Composés selon la revendication 16 pour une utilisation comme médicament destiné au traitement des affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle; pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation; pour traiter toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; pour traiter d'autres désordres dermatologiques tels que les dermatoses bulleuses et les maladies du collagène; pour traiter certains troubles ophtalmologiques, notamment les cornéopathies; pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique; pour prévenir ou guérir les stigmates de l'atrophie épidermique et/ou dermique induite par les corticostéroïdes locaux ou systémiques, ou tout autre forme d'atrophie cutanée, pour prévenir ou traiter les troubles de la cicatrisation ou pour prévenir ou réparer les vergetures; pour favoriser la cicatrisation, pour lutter contre les troubles de la fonction sébacée tels que l'hyperséborrhée de l'acné ou la séborrhée simple; pour le traitement ou la prévention des états cancéreux ou précancéreux, plus particulièrement les leucémies promyélocytaires; pour le traitement d'affections inflammatoires telles que l'arthrite, pour le traitement de toute affection d'origine virale au niveau cutané ou général; pour la prévention ou le traitement de l'alopécie; pour le traitement d'affections dermatologiques à composante immunitaire; pour le traitement d'affections du système cardiovasculaire telles que l'artériosclérose ou l'hypertension ainsi que le diabète non-insulino dépendant, pour le traitement de désordres cutanés dus à une exposition aux rayonnements U.V..

18. Composition pharmaceutique, caractérisée par le fait qu'elle comprend, dans un support pharmaceutiquement acceptable, au moins l'un des composés tels que définis à l'une quelconque des revendications 1 à 15.

19. Composition selon la revendication 18, caractérisée en ce que la concentration en composé(s) selon l'une des revendication 1 à 15 est comprise entre 0,001 % et 5 % en poids par rapport à l'ensemble de la composition.

20. Composition cosmétique, caractérisée par le fait qu'elle comprend, dans un support cosmétiquement acceptable, au moins l'un des composés tels que définis à l'une quelconque des revendications 1 à 15.

21. Composition selon la revendication 20, caractérisée en ce que la concentration en composé(s) selon l'une des revendications 1 à 15 est comprise entre 0,001 % et 3 % en poids par rapport à l'ensemble de la composition.

22. Utilisation d'une composition cosmétique telle que définie à l'une des revendications 20 ou 21 pour l'hygiène corporelle ou capillaire.

## Claims

1. Biaromatic compounds, characterized in that they correspond to the general formula (I) below: 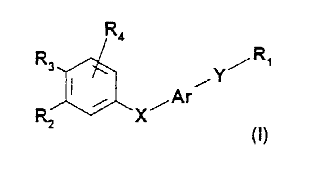 in which:
- R₁ represents
(i) the radical -CH₃,
(ii) the radical -CH₂-O-R₅,
(iii) the radical -O-R₅,
(iv) the radical -CO-R₆,
R₅ and R₆ having the meanings given below,
- Y represents a radical chosen from the radicals of formulae (a) and (b) below: 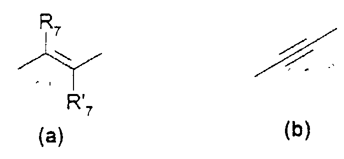 R₇ and R'₇ having the meanings given below,
- Ar represents a radical chosen from the radicals of formulae (c) to (f) below: 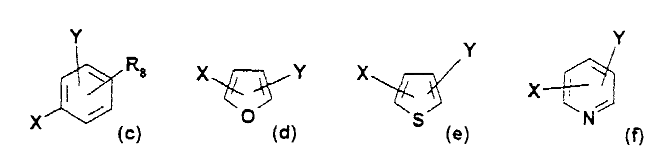 in which the radical Y is in an ortho or meta position relative to the radical X, X and Y of these formulae corresponding to X and Y represented in formula (I),
R₈ having the meaning given below,
- X represents an oxygen or sulphur atom, a radical -SO-, -SO₂-; -N(R₉)- or a radical chosen from the radicals of formulae (g) to (r) below: 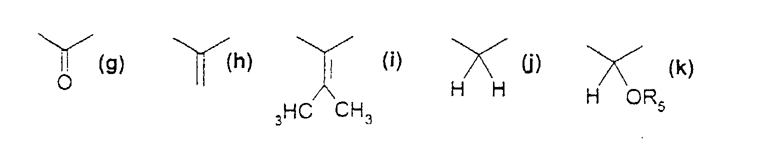 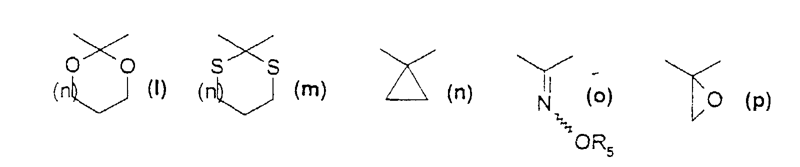 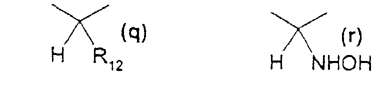 R₅, R₉ and n having the meanings given below,
- R₂ and R₃, which may be identical or different, are chosen from the group consisting of:
(i) a hydrogen atom,
(ii) an alkyl radical having at least 3 carbon atoms, among which the carbon attached to the phenyl radical of formula (I) is substituted with at least two carbon atoms,
(iii) a linear or branched alkyl radical,
(iv) a radical -OR₅,
(v) a radical -SR₅,
(vi) a polyether radical,
R₅ having the meaning given below,
it being understood that R₂ and R₃, taken together, can form, with the adjacent aromatic ring, a 5- or 6-membered ring, optionally substituted with methyl groups and/or optionally interrupted by an oxygen or sulphur atom,
it being understood that, when R₂ and R₃ do not form a ring, at least one of the radicals R₂ and R₃ has a meaning (ii) mentioned above,
- R₄ and R₈, which may be identical or different, represent a hydrogen atom, a halogen atom, a linear or branched alkyl radical, a radical -OR₅, a polyether radical,
- R₅ represents a hydrogen atom, an alkyl radical containing from 1 to 6 carbon atoms or a radical -COR₁₀,
R₁₀ having the meaning given below,
- R₆ represents:
(a) a hydrogen atom
(b) an alkyl radical containing from 1 to 6 carbon atoms
(c) a radical of formula: 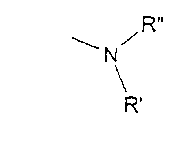 R' and R" having the meanings given below,
(d) a radical -OR₁₁
R₁₁ having the meaning given below,
- R₇, R'₇ and R₉, which may be identical or different, represent a hydrogen atom or an alkyl radical containing from 1 to 6 carbon atoms,
- n is an integer equal to 0 or 1,
- R₁₀ represents an alkyl radical containing from 1 to 6 carbon atoms,
- R₁₁ represents a hydrogen atom, a linear or branched alkyl radical, an alkenyl radical, a mono- or polyhydroxyalkyl radical, an aryl or aralkyl radical, optionally substituted, a sugar residue or an amino acid or peptide residue,
- R₁₂ represents an alkyl radical containing from 1 to 6 carbon atoms,
- R' and R", which may be identical or different, represent a hydrogen atom, an alkyl radical containing from 1 to 6 carbon atoms, a mono- or polyhydroxyalkyl radical, an optionally substituted aryl radical or an amino acid, peptide or sugar residue, or alternatively, taken together, form a heterocycle,
and the optical and geometrical isomers of the said compounds of formula (I), as well as their salts.

2. Compounds according to Claim 1, characterized in that they are in the form of salts of. an alkali metal or alkaline-earth metal, of zinc, of an organic amine or of an inorganic or organic acid.

3. Compounds according to either of Claims 1 and 2, characterized in that the linear or branched alkyl radicals are chosen from the group consisting of the methyl, ethyl, isopropyl, butyl, tert-butyl, hexyl, nonyl and dodecyl radicals.

4. Compounds according to any one of the preceding claims, characterized in that the alkyl radicals having at least 3 carbon atoms, among which the carbon attached to the phenyl radical of formula (I) is substituted with at least two carbon atoms, are chosen from the isopropyl, tert-butyl, 1,1-dimethylhexyl and 1,1-dimethyldecyl radicals.

5. Compounds according to one of the preceding claims, characterized in that the polyhydroxyalkyl radicals are chosen from the group consisting of the 2,3-dihydroxypropyl, 2,3,4-trihydroxybutyl and 2,3,4,5-tetrahydroxypentyl radicals or the pentaerythritol residue.

6. Compounds according to one of the preceding claims, characterized in that the aryl radical is a phenyl radical optionally substituted with at least one halogen atom, a hydroxyl radical, an alkyl radical, a nitro function, a methoxy group or an optionally substituted amine function.

7. Compounds according to one of the preceding claims, characterized in that the aralkyl radicals are chosen from the group consisting of the benzyl or phenethyl radicals, optionally substituted with at least one halogen atom, a hydroxyl, a nitro function or a methoxy group.

8. Compounds according to one of the preceding claims, characterized in that the alkenyl radicals are chosen from the group consisting of radicals containing from 2 to 5 carbon atoms and having one or more ethylenic unsaturations, and in particular. the allyl radical.

9. Compounds according to one of the preceding claims, characterized in that the sugar residues are chosen from the group consisting of glucose, galactose, mannose and glucuronic acid residues.

10. Compounds according to any one of the preceding claims, characterized in that the amino acid. residues are chosen from the group consisting of residues derived from lysine, from glycine or from aspartic acid.

11. Compounds according to any one of the preceding claims, -characterized in that the peptide residues are chosen from the group consisting of dipeptide and tripeptide residues.

12. Compounds according to any one of the preceding claims, characterized in that the heterocyclic radicals are chosen from the group consisting of piperidino, morpholino, pyrrolidino or piperazino radicals, optionally substituted in position 4 with a C₁-C₆ alkyl or polyhydroxyalkyl radical.

13. Compounds according to any one of the preceding claims, characterized in that the polyether radicals are chosen from the methoxymethyl ether, methoxyethoxymethyl ether and methylthiomethyl ether radicals.

14. Compounds according to Claim 1, characterized in that they are taken, alone or as mixtures, from the group consisting of:
3-[3-(5,5,8,8-Tetramethyl-5,6,7,8-tetrahydro-2-naphthylcarbonyl)phenyl]acrylic acid.
3-[3-(3,5,5,8,8-Pentamethyl-5,6,7,8-tetrahydro-2-naphthylcarbonyl)phenyl]acrylic acid.
3-[3-(3,5,5,8,8-Pentamethyl-5,6,7,8-tetrahydro-2-naphthylthio)phenyl]acrylic acid.
3-[3-(5,5,8,8-Tetramethyl-5,6,7,8-tetrahydro-2-naphthylthio)phenyl]acrylic acid.
Ethyl 3-{2-[1-(3,5,5,8,8-pentamethyl-5,6,7,8-tetrahydro-2-naphthyl)vinyl]phenyl}acrylate.
3-{2-[1-(3,5,5,8,8-Pentamethyl-5,6,7,8-tetrahydro-2-naphthyl)vinyl]phenyl}acrylic acid.
Ethyl 3-{3-[1-(3,5,5,8,8-pentamethyl-5,6,7,8-tetrahydro-2-naphthyl)vinyl]phenyl}acrylate.
3-{3-[1-(3,5,5,8,8-Pentamethyl-5,6,7,8-tetrahydro-2-naphthyl)vinyl]phenyl}acrylic acid.
Ethyl 3-{2-[1-(5,5,8,8-tetramethyl-5,6,7,8-tetrahydro-2-naphthyl)vinyl]phenyl}acrylate.
3-{2-[1-(5,5,8,8-Tetramethyl-5,6,7,8-tetrahydro-2-naphthyl)vinyl]phenyl}acrylic acid.
Ethyl 3-{3-[1-(5,5,8,8-tetramethyl-5,6,7,8-tetrahydro-2-naphthyl)vinyl]phenyl}acrylate.
3-{3-[1-(5,5,8,8-Tetramethyl-5,6,7,8-tetrahydro-2-naphthyl)vinyl]phenyl}acrylic acid.
Ethyl 3-{3-[1-(5,5,8,8-tetramethyl-5,6,7,8-tetrahydro-2-naphthyl)ethyl]phenyl}acrylate.
3-{3-[1-(5,5,8,8-Tetramethyl-5,6,7,8-tetrahydro-2-naphthyl)ethyl]phenyl}acrylic acid.
Ethyl 3-{2-[1-(5,5,8,8-tetramethyl-5,6,7,8-tetrahydro-2-naphthyl)ethyl]phenyl}acrylate.
3-{2-[1-(5,5,8,8-Tetramethyl-5,6,7,8-tetrahydro-2-naphthyl)ethyl]phenyl}acrylic acid.
Ethyl 3-{2-[1-(3,5,5,8,8-pentamethyl-5,6,7,8-tetrahydro-2-naphthyl)ethyl]phenyl}acrylate.
3-{2- [1-(3,5,5,8,8-Pentamethyl-5,6,7,8-tetrahydro-2-naphthyl)ethyl]phenyl)acrylic acid.
Ethyl 3-{3-[1-(3,5,5,8,8-Pentamethyl-5,6,7,8-tetrahydro-2-naphthyl)ethyl]phenyl}acrylate.
3-{3-[1-(3,5,5,8,8-Pentamethyl-5,6,7,8-tetrahydro-2-naphthyl)ethyl]phenyl}acrylic acid.
Ethyl 3-{2-[1-(5,5,8,8-tetramethyl-5,6,7,8-tetrahydro-2-naphthyl)cyclopropyl]phenyl}acrylate.
3-{2-[1-(5,5,8,8-Tetramethyl-5,6,7,8-tetrahydro-2-naphthyl) cyclopropyl]phenyl}acrylic acid.
Ethyl 3-{3-[1-(5,5,8,8-tetramethyl-5,6,7,8-tetrahydro-2-naphthyl)cyclopropyl]phenyl}acrylate.
3-{3-[1-(5,5,8,8-Tetramethyl-5,6,7,8-tetrahydro-2-naphthyl)cyclopropyl]phenyl}acrylic acid.
Ethyl 3-{3-[hydroxyimino-(3,5,5,8,8-pentamethyl-5,6,7,8-tetrahydro-2-naphthyl)methyl]phenyl}-acrylate.
3-{3-[Hydroxyimino-(3,5,5,8,8-pentamethyl-5,6,7,8-tetrahydro-2-naphthyl)methyl]phenyl}acrylic acid.
Ethyl 3-{3-[hydroxyimino-(5,5,8,8-tetramethyl-5,6,7,8-tetrahydro-2-naphthyl)methyl]phenyl}-acrylate.
3-{3-[Hydroxyimino-(5,5,8,8-tetramethyl-5,6,7,8-tetrahydro-2-naphtnyl)methyl]phenyl}acrylic acid.
Ethyl 3-{3-[2-(3,5,5,8,8-pentamethyl-5,6,7,8-tetrahydro-2-naphthyl)-[1,3] -dithian-2-yl]phenyl}-acrylate.
3-{3-[2-(3,5,5,8,8-Pentamethyl-5,6,7,8-tetrahydro-2-naphthyl)-[1,3]-dithian-2-yl]phenyl}acrylic acid.
3-{3-[Hydroxylamine-(3,5,5,8,8-pentamethyl-5,6,7,8-tetrahydro-2-naphthyl)methyl]phenyl}acrylic acid.
Ethyl 3-{3-[hydroxylamine-(5,5,8,8-tetramethyl-5,6,7,8-tetrahydro-2-naphthyl)methyl]phenyl}-acrylate.
3-{2-[Hydroxylamine-(3,5,5,8,8-pentamethyl-5,6,7,8-tetrahydro-2-naphthyl)methyl]phenyl}acrylic acid.
Ethyl 3-{2-[Hydroxylamine-(5,5,8,8-tetramethyl-5,6,7,8-tetrahydro-2-naphthyl)methyl]phenyl}-acrylate.
{3-[1-(5,5,8,8-Tetramethyl-5,6,7,8-tetrahydro-2-naphthyl)cyclopropyl]phenyl}propynoic acid.
{3-[1-(3,5,5,8,8-Pentamethyl-5,6,7,8-tetrahydro-2-naphthyl)cyclopropyl]phenyl}propynoic acid.
{2-[1-(5,5,8,8-Tetramethyl-5,6,7,8-tetrahydro-2-naphthyl)cyclopropyl]phenyl}propynoic acid.
{2-[1-(3,5,5,8,8-Pentamethyl-5,6,7,8-tetrahydro-2-naphthyl)cyclopropyl]phenyl}propynoic acid.
3-{2-[1-(3,5,5,8,8-Pentamethyl-5,6,7,8-tetrahydro-2-naphthyl) cyclopropyl]phenyl}acrylic acid.
3-{3-[1-(3,5,5,8,8-Pentamethyl-5,6,7,8-tetrahydro-2-naphthyl)cyclopropyl]phenyl}acrylic acid.
3-{4-Hydroxy-3-[1-(5,5,8,8-tetramethyl-5,6,7,8-tetrahydro-2-naphthyl)cyclopropyl]phenyl}-acrylic acid.
3-{3-Hydroxy-2-[1-(5,5,8,8-tetramethyl-5,6,7,8-tetrahydro-2-naphthyl)cyclopropyl]phenyl}-acrylic acid.
3-{3-[1-(5,5,8,8-Tetramethyl-5,6,7,8-tetrahydro-2-naphthyl)cyclopropyl]-4-methoxyphenyl}acrylic acid.
3-{2-[1-(5,5,8, 8-Tetramethyl-5,6,7,8-tetrahydro-2-naphthyl)cyclopropyl]-3-methoxyphenyl}acrylic acid.
3-{4-Hydroxy-3-[1-(3,5,5,8,8-pentamethyl-5,6,7,8-tetrahydro-2-naphthyl)cyclopropyl]phenyl}-acrylic acid.
3-{3-Hydroxy-2-[1-(3,5,5,8,8-pentamethyl-5,6,7,8-tetrahydro-2-naphthyl)cyclopropyl]phenyl}-acrylic acid.
3-{3-[1-(3,5,5,8,8-Pentamethyl-5,6,7,8-tetrahydro-2-naphthyl)cyclopropyl]-4-methoxyphenyl}acrylic acid.
3-{2-[1-(3,5,5,8,8-Pentamethyl-5,6,7,8-tetrahydro-2-naphthyl)cyclopropyl]-3-methoxyphenyl}acrylic acid.
3-{3-[1-(5,5,8,8-Tetramethyl-5,6,7,8-tetrahydro-2-naphthyl)cyclopropyl]phenyl)acrylamide.
3-{2-[1-(5,5,8,8-Tetramethyl-5,6,7,8-tetrahydro-2-naphthyl)cyclopropyl]phenyl}acrylamide.
3-{3-[1-(3,5,5,8,8-Pentamethyl-5,6,7,8-tetrahydro-2-naphthyl)cyclopropyl]phenyl}acrylamide.
3-{2-[1-(5,5,8,8-Pentamethyl-5,6,7,8-tetrahydro-2-naphthyl)cyclopropyl]phenyl}acrylamide.
*N*-Ethyl-3-{3-[1-(5,5,8,8-tetramethyl-5,6,7,8-tetrahydro-2-naphthyl)cyclopropyl]phenyl}acrylamide.
*N*-Ethyl-3-{2-[1-(5,5,8,8-tetramethyl-5,6,7,8-tetrahydro-2-naphthyl)cyclopropyl]phenyl}acrylamide.
*N*-Ethyl-3-{3-[1-(3,5,5,8,8-pentamethyl-5,6,7,8-tetrahydro-2-naphthyl)cyclopropyl]phenyl}-acrylamide.
*N*-Ethyl-3-{2-[1-(3,5,5,8,8-pentamethyl-5,6,7,8-tetrahydro-2-naphthyl)cyclopropyl]phenyl}-acrylamide.
1-Morpholin-4-yl-3-{3-[1-(5,5,8,8-tetramethyl-5,6,7,8-tetrahydro-2-naphthyl)cyclopropyl]-phenyl}propenone.
1-Morpholin-4-yl-3-{2-[1-(5,5,8,8-tetramethyl-5,6,7,8-tetrahydro-2-naphthyl)cyclopropyl]-phenyl}propenone.
1-Morpholin-4-yl-3-{3-[1-(3,5,5,8,8-pentamethyl-5,6,7,8-tetrahydro-2-naphthyl)cyclopropyl]-phenyl}propenone.
1-Morpholin-4-yl-3-{2-[1-(3,5,5,8,8-pentamethyl-5,6,7,8-tetrahydro-2-naphthyl)cyclopropyl]-phenyl}propenone.
*N*-(4-Hydroxyphenyl)-3-(3-[1-(5,5,8,8-tetramethyl-5,6,7,8-tetrahydro-2-naphthyl)cyclopropyl]-phenyl}acrylamide.
*N*-(4-Hydroxyphenyl)-3-{2-[1-(5,5,8,8-tetramethyl-5,6,7,8-tetrahydro-2-naphthyl)cyclopropyl]-phenyl}acrylamide.
*N*-(4-Hydroxyphenyl)-3-{3-[1-(3,5,5,8,8-pentamethyl-5,6,7,8-tetrahydro-2-naphthyl)cyclopropyl]-phenyl}acrylamide.
*N*-(4-Hydroxyphenyl)-3-{2-[1-(3,5,5,8,8-pentamethyl-5,6,7,8-tetrahydro-2-naphthyl)cyclopropyl]-phenyl}acrylamide.
3-{3-[1-(5,5,8,8-Tetramethyl-5,6,7,8-tetrahydro-2-naphthyl) ethyl]phenyl}propenal.
3-{2-[1-(5,5,8,8-Tetramethyl-5,6,7,8-tetrahydro-2-naphthyl) ethyl]phenyl}propenal.
3-{3-[1-(3,5,5,8,8-Pentamethyl-5,6,7,8-tetrahydro-2-naphthyl)ethyl]phenyl}propenal.
3-{2-[1-(3,5,5,8,8-Pentamethyl-5,6,7,8-tetrahydro-2-naphthyl)ethyl]phenyl}propenal.
3-{3-[1-(5,5,8,8-Tetramethyl-5,6,7,8-tetrahydro-2-naphthyl)ethyl]phenyl}prop-2-en-1-ol.
3-{2-[1-(5,5,8,8-Tetramethyl-5,6,7,8-tetrahydro-2-naphthyl)ethyl]phenyl}prop-2-en-1-ol.
3-{3-[1-(3,5,5,8,8-Pentamethyl-5,6,7,8-tetrahydro-2-naphthyl) ethyl]phenyl}prop-2-en-1-ol.
3-{2-[1-(3,5,5,8,8-Pentamethyl-5,6,7,8-tetrahydro-2-naphthyl)ethyl]phenyl}prop-2-en-1-ol.

15. Compounds according to Claim 1, characterized in that they have at least one of the following characteristics:
R₁ represents the radical -CO-R₆
Ar represents the radicals of formula (c) or (f)
X represents the radicals of formula (g), (h), (n) or (m)
R₂ and R3, taken together, form, with the adjacent aromatic ring, a 5- or 6-membered ring optionally substituted with methyl groups and/or optionally interrupted by an oxygen or sulphur atom.

16. Compounds according to any one of the preceding claims, for use as medicaments.

17. Compounds according to Claim 16, for use as medicaments intended for the treatment of dermatological complaints associated . with a keratinization disorder which has a bearing on differentiation and on proliferation, in particular for treating common acne, comedones, polymorphonuclear leukocytes, acne rosacea, nodulocystic acne, acne conglobata, senile acne and secondary acne such as solar acne, medication-induced acne or occupational acne; for treating other types of keratinization. disorders, in particular ichthyosis, ichthyosiform states, Darier's disease, palmoplantar keratoderma, leukoplasias and leukoplasiform states, and cutaneous or mucous (buccal) lichen; for treating other dermatological complaints associated with a keratinization disorder having an inflammatory and/or immunoallergic component and, in particular, all forms of psoriasis, whether it is cutaneous, mucous or ungual psoriasis, and even psoriatic rheumatism, or alternatively cutaneous atopy, such as eczema, or respiratory atopy or alternatively gingival hypertrophy; the compounds may also be used in certain inflammatory complaints which do not exhibit a keratinization disorder; for treating all dermal or epidermal proliferations, whether benign or malignant and whether or not they are of viral origin, such as common warts, flat warts and verruciform epidermodysplasia, it being possible for the oral or florid papillomatoses and the proliferations to be induced by ultraviolet radiation, in particular in the case of basocellular and spinocellular epitheliomas; for treating other dermatological disorders such as bullosis and collagen diseases; for treating certain ophthalmological disorders, in particular corneopathies; for repairing or combating both light-induced and chronological ageing of the skin or for reducing actinic keratoses and pigmentations, or any pathology associated with chronological or actinic ageing; for preventing or curing the stigmata of epidermal and/or dermal atrophy induced by local or systemic corticosteroids, or any other form of skin atrophy; for preventing or treating cicatrization disorders or for preventing or repairing stretchmarks; for encouraging cicatrization, for combating disorders of sebaceous functioning such as the hyperseborrhoea of acne or simple seborrhoea; for the treatment or prevention of cancerous or precancerous states, more-particularly promyelocytic leukaemias; for the treatment of inflammatory complaints such as arthritis; for the treatment of any complaint of viral origin on the skin or generally; for the prevention or treatment of alopecia; for the treatment of dermatological complaints having an immunological component; for the treatment of complaints of the cardiovascular system such as arteriosclerosis, hypertension and non-insulin-dependent diabetes for the treatment of skin disorders due to exposure to UV radiation.

18. Pharmaceutical composition, characterized in that it comprises, in a pharmaceutically acceptable support, at least one of the compounds as defined in any one of Claims 1 to 15.

19. Composition according to Claim 18, characterized in that the concentration of compound(s) according to one of Claims 1 to 15 is between 0.001% and 5% by weight relative to the composition as a whole.

20. Cosmetic composition, characterized in that it comprises, in a cosmetically acceptable support, at least one of the compounds as defined in any one of Claims 1 to 15.

21. Composition according to Claim 20, characterized in that the concentration of compound(s) according to one of Claims 1 to 15 is between 0.001% and 3% by weight relative to the composition as a whole.

22. Use of a cosmetic composition as defined in either of Claims 20 and 21, for hair or body hygiene.

## Patentansprüche

1. Diaromatische Verbindungen, dadurch gekennzeichnet, daß sie der folgenden allgemeinen Formel (I) 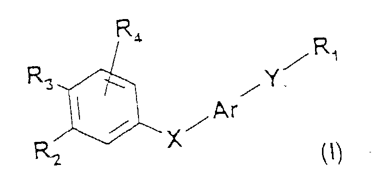 entsprechen, in der bedeuten:
- R₁
(i) die Gruppe -CH₃,
(ii) die Gruppe -CH₂-O-R₅,
(iii) die Gruppe -O-R₅,
(iv) die Gruppe -CO-R₆,
worin R₅ und R₆ die weiter unten angegebene Bedeutung haben,
- Y eine Gruppe, die unter den Gruppen der folgenden Formeln (a) und (b) 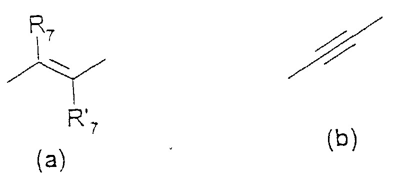 ausgewählt ist, in denen R₇ und R'₇ die weiter unten angegebene Bedeutung haben,
- Ar eine Gruppe, die unter den Gruppen der folgenden Formeln (c) bis (f) 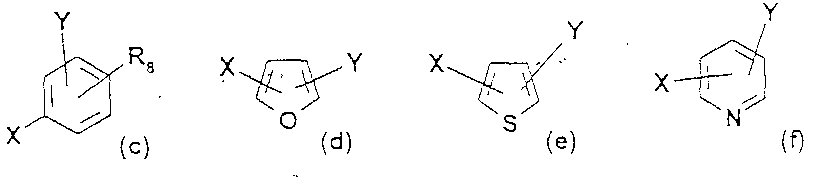 ausgewählt ist, in denen sich die Gruppe Y in o- oder m-Stellung, bezogen auf die Gruppe X befindet, wobei X und Y in diesen Formel den Gruppen X und Y in der Formel (I) entsprechen, worin R₈ die weiter unten angegebene Bedeutung hat,
- X ein Sauerstoffatom, ein Schwefelatom, eine Gruppe -SO-, -SO₂-, -N(R₉)- oder eine Gruppe, die unter den Gruppen der folgenden Formeln (g) bis (r) 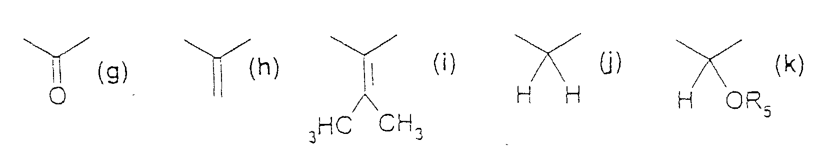 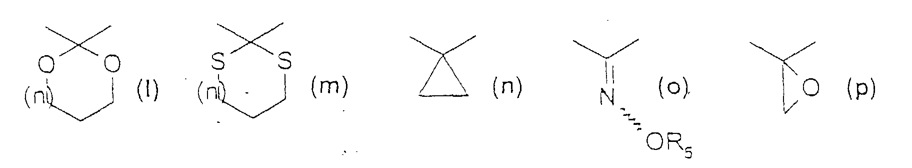 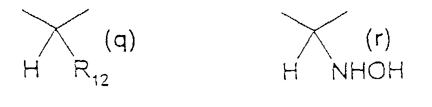 ausgewählt ist, worin R₅, R₁₂ und n die weiter unten angegebene Bedeutung haben,
- R₂ und R₃ Reste, die gleich oder verschieden sind und ausgewählt sind unter
(i) Wasserstoff,
(ii) Alkylgruppen, die mindestens 3 Kohlenstoffatome aufweisen, von denen das Kohlenstoffatom, das mit dem Phenylrest der Formel (I) verbunden ist, mit mindestens zwei Kohlenstoffatomen substituiert ist,
(iii) geradkettigen oder verzweigten Alkylresten,
(iv) den Gruppen -OR₅,
(v) den Gruppen -SR₅,
(vi) Polyether-Gruppen,
worin R₅ die weiter unten angegebene Bedeutung hat,
mit der Maßgabe, daß R₂ und R₃ zusammen mit dem benachbarten aromatischen Ring einen Ring mit 5 oder 6 Ringgliedern bilden können, der gegebenenfalls mit Methylgruppen substituiert ist und/oder gegebenenfalls durch ein Sauerstoffatom oder ein Schwefelatom unterbrochen ist, und
mit der Maßgabe, daß mindestens einer der Reste R₂ und R₃ eine der oben angegebenen Bedeutungen (ii) hat, wenn R₂ und R₃ keinen Ring bilden,
- R₄ und R₈, die gleich oder verschieden sind, ein Wasserstoffatom, ein Halogenatom, einen geradkettigen oder verzweigten Alkylrest oder eine Gruppe -OR₅, eine Polyether-Gruppe,
- R₅ ein Wasserstoffatom, einen Alkylrest mit 1 bis 6 Kohlenstoffatomen oder eine Gruppe -COR₁₀, worin R₁₀ die weiter unten angegebene Bedeutung hat,
- R₆
(a) ein Wasserstoffatom,
(b) einen Alkylrest mit 1 bis 6 Kohlenstoffatomen,
(c) eine Gruppe der Formel 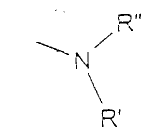 worin R' und R" die weiter unten angegebene Bedeutung haben,
(d) eine Gruppe -OR₁₁, worin R₁₁ die weiter unten angegebene Bedeutung hat,
- R₇, R'₇ und R₉, die gleich oder verschieden sind, ein Wasserstoffatom oder einen Alkylrest mit 1 bis 6 Kohlenstoffatomen,
- n die ganze Zahl 0 oder 1,
- R₁₀ einen Alkylrest mit 1 bis 6 Kohlenstoffatomen,
- R₁₁ ein Wasserstoffatom, geradkettiges oder verzweigtes Alkyl, Alkenyl, Mono-oder Polyhydroxyalkyl, Aryl oder Aralkyl, das/die gegebenenfalls substituiert ist/sind, einen Zuckerrest, eine Aminosäuregruppe oder einen Peptidrest,
- R₁₂ einen Alkylrest mit 1 bis 6 Kohlenstoffatomen,
- R' und R", die gleich oder verschieden sind, ein Wasserstoffatom, niederes Alkyl, Mono- oder Polyhydroxyalkyl, Aryl, das gegebenenfalls substituiert ist, oder eine Aminosäuregruppe, einen Peptidrest oder einen Zuckerrest, oder zusammen Gruppen, die einen Heterocyclus bilden,
und die optischen und geometrischen Isomere dieser Verbindungen der Formel (I) sowie ihre Salze.

2. Verbindungen nach Anspruch 1, dadurchgekenrtzeichnet, daß sie in Form eines Alkali- oder Erdalkalisalzes, des Zinksalze, eines Salzes eines organischen Amins, einer anorganischen Säure oder einer organischen Säure vorliegen.

3. Verbindungen nach einem der Ansprüche 1 und 2, dadurch gekennzeichnet, daß die geradkettigen oder verzweigten Alkylreste unter Methyl, Ethyl, Isopropyl, Butyl, tert.-Butyl, Hexyl, Nonyl und Dodecyl ausgewählt sind.

4. Verbindungen nach einem der vorhergehenden Ansprüche, dadurch gekennzeichnet, daß die Alkylreste, die mindestens 3 Kohlenstoffatome aufweisen, von denen das Kohlenstoffatom, das mit dem Phenylrest der Formel (I) verbunden ist, mit mindestens zwei Kohlenstoffatomen substituiert ist, unter Isopropyl, *tert.*-Butyl, 1,1-Di-methylhexyl und 1,1-Dimethyldecyl ausgewählt sind.

5. Verbindungen nach einem der vorhergehenden Ansprüche, dadurch gekennzeichnet, daß die Polyhydroxyalkylreste unter 2-3-Dihydroxypropyl, 2,3,4-Trihydroxybutyl, 2,3,4,5-Tetrahydroxypentyl und dem Pentaerythrit-Rest ausgewählt sind.

6. Verbindungen nach einem der vorhergehenden Ansprüche, dadurch gekennzeichnet, daß es sich bei dem Arylrest um einen Phenylrest handelt, der gegebenenfalls mit mindestens einem Halogenatom, einer Hydroxygruppe, einem Alkylrest, einer Nitrogruppe, Methoxygruppe oder Amingruppe, die gegebenenfalls substituiert ist, substituiert ist.

7. Verbindungen nach einem der vorhergehenden Ansprüche, dadurch gekennzeichnet, daß die Aralkylreste unter Benzyl und Phenethyl, die gegebenenfalls mit mindestens einem Halogenatom, einer Hydroxygruppe, einer Nitrogruppe oder einer Methoxygruppe substituiert sind, ausgewählt sind.

8. Verbindungen nach einem der vorhergehenden Ansprüche, dadurch gekennzeichnet, daß die Alkenylreste unter den Resten, die 2 bis 5-Kohlenstoffatome enthalten und einfach oder mehrfach ethylenisch ungesättigt sind, und insbesondere unter dem Allylrest ausgewählt sind.

9. Verbindungen nach einem der vorhergehenden Ansprüche, dadurch gekennzeichnet, daß die Zuckerreste unter den Glucose-, Galactose-, Mannose- und Glucuronsäurere- sten ausgewählt sind.

10. Verbindungen nach einem der vorhergehenden Ansprüche, dadurch gekennzeichnet, daß die Aminosäuregruppen unter den Gruppen ausgewählt sind, die von Lysin, Glycin und Asparaginsäure abgeleitet sind.

11. Verbindungen nach einem der vorhergehenden Ansprüche, dadurch gekennzeichnet, daß die Peptidreste unter den Dipeptid- und Tripeptidresten ausgewählt sind.

12. Verbindungen nach einem der vorhergehenden Ansprüche, dadurch gekennzeichnet, daß die heterocyclischen Reste unter dem Piperidino-, Morpholino-, Pyrrolidino- und Piperazino-Rest, die gegebenenfalls in 4-Stellung mit C₁-C₆-Alkyl oder Polyhydroxyalkyl substituiert sind, ausgewählt sind.

13. Verbindungen nach einem der vorhergehenden Ansprüche, dadurch gekennzeichnet, daß die Polyether-Gruppen unter den Methoxymethylether-, Methoxyethoxymethylether- und Methylthiomethylether-Gruppen ausgewählt sind.

14. Verbindungen nach Anspruch 1, dadurch gekennzeichnet, daß sie einzeln oder im Gemisch aus der Gruppe entnommen werden, die aus den folgenden Verbindungen besteht:
3-[3-(5,5,8,8-Tetramethyl-5,6,7,8-tetrahydro-2-naphthylcarbonyl)-phenyl]-acrylsäure.
3-[3-(3,5,5,8,8-Pentamethyl-5,6,7,8-tetrahydro-2-naphthylcarbonyl)-phenyl]-acrylsäure.
3-[3-(3,5,5,8,8-Pentamethyl-5,6,7,8-tetrahydro-2-naphthylthio)-phenyl]-acrylsäure.
3-[3-(5,5,8,8-Tetramethyl-5,6,7,8-tetrahydro-2-naphthylthio)-phenyl]-acrylsäure.
3-{2-[1-(3,5,5,8,8-Pentamethyl-5,6,7,8-tetrahydro-2-naphthyl)-vinyl]-phenyl}-acrylsäureethylester.
3-(2-[1-(3,5,5,8,8-Pentamethyl-5,6,7,8-tetrahydro-2-naphthyl)-vinyl]-phenyl}-acrylsäure.
3-{3-[1-(3,5,5,8,8-Pentamethyl-5,6,7,8-tetrahydro-2-naphthyl)-vinyl]-phenyl}-acrylsäureethylester.
3-(3-[1-(3,5,5,8,8-Pentamethyl-5,6,7,8-tetrahydro-2-naphthyl)-vinyl]-phenyl }-acrylsäure.
3-{2-[1-(5,5,8,8-Tetramethyl-5,6,7,8-tetrahydro-2-naphthyl)-vinyl]-phenyl}-acrylsäureethylester.
3-{2-[1-(5,5,8,8-Tetramethyl-5,6,7,8-tetrahydro-2-naphthyl)-vinyl]-phenyl}-acrylsäure.
3-{3-[1-(5,5,8,8-Tetramethyl-5,6,7,8-tetrahydro-2-naphthyl)-vinyl]-phenyl}-acrylsäureethylester.
3-{3-[1-(5,5,8,8-Tetramethyl-5,6,7,8-tetrahydro-2-naphthyl)-vinyl]-phenyl}-acrylsäure.
3-{3-[1-(5,5,8,8-Tetramethyl-5,6,7,8-tetrahydro-2-naphthyl)-ethyl]-phenyl}-acrylsäureethylester.
3-{3-[1-(5,5,8,8-Tetramethyl-5,6,7,8-tetrahydro-2-naphthyl)-ethyl]-phenyl}-acrylsäure.
3-{2-[1-(5,5,8,8-Tetramethyl-5,6,7,8-tetrahydro-2-naphthyl)-ethyl]-phenyl}-acrylsäureethylester.
3-{2-[1-(5,5,8,8-Tetramethyl-5,6,7,8-tetrahydro-2-naphthyl)-ethyl]-phenyl}-acrylsäure.
3-{2-[1-(3,5,5,8,8-Pentamethyl-5,6,7,8-tetrahydro-2-naphthyl)-ethyl]-phenyl)-acrylsäureethylester.
3-{2-[1-(3,5,5,8,8-Pentamethyl-5,6,7,8-tetrahydro-2-naphthyl)-ethyl]-phenyl}-acrylsäure.
3-{3-[1-(3,5,5,8,8-Pentamethyl-5,6,7,8-tetrahydro-2-naphthyl)-ethyl]-phenyl}-acrylsäureethylester.
3-(3-[1-(3,5,5,8,8-Pentamethyl-5,6,7,8-tetrahydro-2-naphthyl)-ethyl]-phenyl}-acrylsäure.
3-{2-[1-(5,5,8,8-Tetramethyl-5,6,7,8-tetrahydro-2-naphthyl)-cyclopropyl]-phenyl}-acrylsäureethylester.
3-{2-[1-(5,5,8,8-Tetramethyl-5,6,7,8-tetrahydro-2-naphthyl)-cyclopropyl]-phenyl}-acrylsäure.
3-{3-[1-(5,5,8,8-Tetramethyl-5,6,7,8-tetrahydro-2-naphthyl)-cyclopropyl]-phenyl}-acrylsäureethylester.
3-{3-[1-(5,5,8,8-Tetramethyl-5,6,7,8-tetrahydro-2-naphthyl)-cyclopropyl]-phenyl}-acrylsäure.
3-{3-[Hydroxyimino-(3,5,5,8,8-pentamethyl-5,6,7,8-tetrahydro-2-naphthalinyl)-methyl]-phenyl}-acrylsäureethylester.
3-{3-[Hydroxyimino-(3,5,5,8,8-pentamethyl-5,6,7,8-tetrahydro-2-naphthalinyl)-methyl]-phenyl}-acrylsäure.
3-{3-[Hydroxyimino-(5,5,8,8-tetramethyl-5,6,7,8-tetrahydro-2-naphthalinyl)-methyl]-phenyl}-acrylsäureethylester.
3-{3-[Hydroxyimino-(5,5,8,8-tetramethyl-5,6,7,8-tetrahydro-2-naphthalinyl)-methyl]-phenyl}-acrylsäure.
3-{3-[2-(3,5,5,8,8-Pentamethyl-5,6,7,8-tetrahydro-2-naphthalinyl)-[1,3]-dithian-2-yl]-phenyl}-acrylsäureethylester.
3-{3-[2-(3,5,5,8,8-Pentamethyl-5,6,7,8-tetrahydro-2-naphthalinyl)-[1,3]-dithian-2-yl]-phenyl}-acrylsäure.
3-{3-[Hydroxylamin-(3,5,5,8,8-pentamethyl-5,6,7,8-tetrahydronaphthalin-2-yl)-methyl]-phenyl}-acrylsäure.
3-{3-[Hydroxylamin-(5,5,8,8-tetramethyl-5,6,7,8-tetrahydronaphthalin-2-yl)-methyl]-phenyl}-acrylsäureethylester.
3-{2-[Hydroxylamin-(5,5,8,8 tetramethyl-5,6,7,8-tetrahydro-2-naphthalinyl)-methyl]-phenyl}-acrylsäure.
3-{2-[Hydroxylamin-(5,5,8,8-tetramethyl-5,6,7,8-tetrahydronaphthalin-2-yl)-methyl]-phenyl}-acrylsäureethylester.
{3-[1-(5,5,8,8-Tetramethyl-5,6,7,8-tetrahydro-2-naphthalinyl)-cyclopropyl]-phenyl}-propinsäure.
{3-[1-(3,5,5,8,8-Pentamethyl-5,6,7,8-tetrahydro-2-naphthalinyl)-cyclopropyl]-phenyl}-propinsäure.
{2-[1-(5,5,8,8-Tetramethyl-5,6,7,8-tetrahydro-2-naphthalinyl)-cyclopropyl]-phenyl}-propinsäure.
{2-[1-(3,5,5,8,8-Pentamethyl-5,6,7,8-tetrahydro-2-naphthalinyl)-cyclopropyl]-phenyl}-propinsäure.
3-{2-[1-(3,5,5,8,8-Pentamethyl-5,6,7,8-tetrahydro-2-naphthyl)-cyclopropyl]-phenyl}-acrylsäure.
3-{3-[1-(3,5,5,8,8-Pentamethyl-5,6,7,8-tetrahydro-2-naphthyl)-cyclopropyl]-phenyl}-acrylsäure.
3-{4-Hydroxy-3-1-(5,5,8,8-tetramethyl-5,6,7,8-tetrahydro-2-naphthalinyl)-cyclopropyl]-phenyl}-acrylsäure.
3-{3-Hydroxy-2-[1-(5,5,8,8-tetramethyl-5,6,7,8-tetrahydro-2-naphthalinyl)-cyclopropyl]-phenyl}-acrylsäure.
3-{3-1-(5,5,8,8-Tetramethyl-5,6,7,8-tetrahydro-2-naphthalinyl)-cyclopropyl]-4-methoxyphenyl}-acrylsäure.
3- {2-[1-(5,5,8,8-Tetramethyl-5,6,7,8-tetrahydro-2-naphthalinyl)-cyclopropyl]-3-methoxyphenyl}-acrylsäure.
3- {4-Hydroxy-3-[1-(3,5,5,8,8-pentamethyl-5,6,7,8-tetrahydro-2-naphthalinyl)-cyclopropyl]-phenyl}-acrylsäure.
3-{3-Hydroxy-2-[1-(3,5,5,8,8-pentamethyl-5,6,7,8-tetrahydro-2-naphthalinyl)-cyclopropyl]-phenyl}-acrylsäure.
3-{3-[1-(3,5,5,8,8-Pentamethyl-5,6,7,8-tetrahydro-2-naphthalinyl)-cyclopropyl]-4-methoxyphenyl}-acrylsäure.
3-{2-[1-(3,5,5,8,8-Pentamethyl-5,6,7,8-tetrahydro-2-naphthalinyl)-cyclopropyl]-3-methoxyphenyl}-acrylsäure
3-{3-[1-(5,5,8,8-Tetramethyl-5,6,7,8-tetrahydro-2-naphthalinyl)-cyclopropyl]-phenyl}-acrylamid.
3-{2-[1-(5,5,8,8-Tetramethyl-5,6,7,8-tetrahydro-2-naphthalinyl)-cyclopropyl]-phenyl}-acrylamid.
3-{3-[1-(3,5,5,8,8-Pentamethyl-5,6,7,8-tetrahydro-2-naphthalinyl)-cyclopropyl]-phenyl}-acrylamid.
3-{2-[1-(3,5,5,8,8-Pentamethyl-5,6,7,8-tetrahydro-2-naphthalinyl)-cyclopropyl]-phenyl}-acrylamid.
*N*-Ethyl-3-{3-[1-(5,5,8,8-Tetramethyl-5,6,7,8-tetrahydro-2-naphthalinyl)-cyclopropyl]-phenyl}-acrylamid.
*N*-Ethyl-3-{2-[1-(5,5,8,8-Tetramethyl-5,6,7,8-tetrahydro-2-naphthalinyl)-cyclopropyl]-phenyl}-acrylamid.
*N*-Ethyl-3-{3-[1-(3,5,5,8,8-Pentamethyl-5,6,7,8-tetrahydro-2-naphthalinyl)-cyclopropyl]-phenyl}-acrylamid.
*N*-Ethyl-3-{2-[1-(3,5,5,8,8-Pentamethyl-5,6,7,8-tetrahydro-2-naphthalinyl)-cyclopropyl]-phenyl}-acrylamid.
1-Morpholin-4-yl-3-{3-[1-(5,5,8,8-tetramethyl-5,6,7,8-tetrahydro-2-naphthalinyl)-cyclopropyl]-phenyl}-propenon.
1-Morpholin-4-yl-3-{2-[1-(5,5,8,8-tetramethyl-5,6,7,8-tetrahydro-2-naphthalinyl)-cyclopropyl]-phenyl}-propenon.
1-Morpholin-4-yl-3-{3-[1-(3,5,5,8,8-pentamethyl-5,6,7,8-tetrahydro-2-naphthalinyl)-cyclopropyl]-phenyl}-propenon.
1-Morpholin-4-yl-3-{2-[1-(3,5,5,8,8-pentamethyl-5,6,7,8-tetrahydro-2-naphthalinyl)-cyclopropyl]-phenyl}-propenon.
*N*-(4-Hydroxyphenyl)-3-{3-[1-(5,5,8,8-tetramethyl-5,6,7,8-tetrahydro-2-naphthalinyl)-cyclopropyl]-phenyl}-acrylamid.
*N*-(4-Hydroxyphenyl)-3-{2-[1-(5,5,8,8-tetramethyl-5,6,7,8-tetrahydro-2-naphthalinyl)-cyclopropyl]-phenyl}-acrylamid.
*N*-(4-Hydroxyphenyl)-3-{3-[1-(3,5,5,8,8-pentamethyl-5,6,7,8-tetrahydro-2-naphthalinyl)-cyclopropyl]-phenyl}-acrylamid.
*N*-(4-Hydroxyphenyl)-3-{2-[1-(3,5,5,8,8-pentamethyl-5,6,7,8-tetrahydro-2-naphthalinyl)-cyclopropyl]-phenyl}-acrylamid.
3-{3-[1-(5,5,8,8-Tetramethyl-5,6,7,8-tetrahydro-2-naphthalinyl)-ethyl]-phenyl}-propenal.
3- {2-[1-(5,5,8,8-Tetramethyl-5,6,7,8-tetrahydro-2-naphthalinyl)-ethyl]-phenyl}-propenal.
3-{3-[1-(3,5,5,8,8-Pentamethyl-5,6,7,8-tetrahydro-2-naphthalinyl)-ethyl]-phenyl}-propenal.
3-{2-[1-(3,5,5,8,8-Pentamethyl-5,6,7,8-tetrahydro-2-naphthalinyl)-ethyl]-phenyl}-propenal.
3-{3-[1-(5,5,8,8-Tetramethyl-5,6,7,8-tetrahydro-2-naphthalinyl)-ethyl]-phenyl}-prop-2-en-1-ol.
3- {2-[1-(5,5,8,8-Tetramethyl-5,6,7,8-tetrahydro-2-naphthalinyl)-ethyl]-phenyl}-prop-2-en-1-ol.
3- {3-[1-(3,5,5,8,8-Pentamethyl-5,6,7,8-tetrahydro-2-naphthalinyl)-ethyl]-phenyl}-prop-2-en-1-ol.
3-{2-[1-(3,5,5,8,8-Pentamethyl-5,6,7,8-tetrahydro-2-naphthalinyl)-ethyl]-phenyl}-prop-2-en-1-ol.

15. Verbindungen nach Anspruch 1, dadurch gekennzeichnet, daß sie mindestens eine der folgenden Bedingungen erfüllen:
R₁ stellt eine Gruppe -CO-R₆ dar,
Ar stellt eine der Gruppen der Formeln (c) und (f) dar,
X stellt eine der Gruppen der Formeln (g), (h), (n) und (m) dar,
R₂ und R₃ bilden zusammen genommen mit dem benachbarten aromatischen Ring einen Ring mit 5 oder 6 Ringgliedern, der gegebenenfalls mit Methylgruppen substituiert ist und/oder gegebenenfalls durch ein Sauerstoff- oder Schwefelatom unterbrochen ist.

16. Verbindungen nach einem der vorhergehenden Ansprüche zur Verwendung als Arzneimittel.

17. Verbindungen nach Anspruch 16 zur Verwendung als Arzneimittel, das vorgesehen ist zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonicá, der polymorphen Akne, der Akne rosaceae, der nodulären Akne, der Akne conglobata, der altersbedingten Aknen, der als Nebenwirkung auftretenden Aknen, wie der Akne solaris, der medikamentenbedingten Akne oder der Akne professionalis, zur Behandlung anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psonasis, die die Haut, die Schleimhäute und die Fingerund Zehennägel betreffen, und des psoriatischen Rheumas und der Hautatopien, wie Ekzemen, oder der respiratorischen Atopie oder auch der Hypertrophie des Zahnfleischs, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris, Verruca plana, Epidermodysplasia verruciformis, orale Papillomatose, Papillomatosis florida, und der Wucherungen, die durch UV-Strahlung hervorgerufen werden können, insbesondere des Epithelioma basocellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hyperseborrhö bei Akne oder der einfachen Seborrhö, zur Bekämpfung von oder Vorbeugung vor krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Herz-Kreislauf-Erkrankungen, wie Arteriosklerose oder Bluthochdruck, sowie des Insulin-unabhängigen Diabetes, zur Behandlung von Hautproblemen, die durch UV-Strahlung hervorgerufen werden.

18. Pharmazeutische Zusammensetzung, dadurch gekennzeichnet, daß sie in einem pharmazeutisch akzeptablen Träger mindestens eine der Verbindungen, die wie in den Ansprüchen 1 bis 15 definiert sind, enthält.

19. Zusammensetzung nach Anspruch 18, dadurch gekennzeichnet, daß die Konzentration an Verbindung(en) nach einem der Ansprüche 1 bis 15 im Bereich von 0,001 bis 5 Gew.-%, bezogen auf das Gesamtgewicht der Zusammensetzung, liegt.

20. Kosmetische Zusammensetzung, dadurch gekennzeichnet, daß sie in einem kosmetisch akzeptablen Träger mindestens eine der wie in einem der Ansprüche 1 bis 15 definierten Verbindungen enthält.

21. Zusammensetzung nach Anspruch 20, dadurch gekennzeichnet, daß die Konzentration an Verbindung(en) nach einem der Ansprüche 1 bis 15 im Bereich von 0,001 bis 3 Gew.-%, bezogen auf das Gesamtgewicht der Zusammensetzung, liegt.

22. Verwendung einer wie in einem der Ansprüche 20 und 21 definierten kosmetischen Zusammensetzung für die Körperreinigung oder die Reinigung der Haare.
